# EUROPEAN PATENT APPLICATION

(11) **EP 4 803 525 A1**
(43) Date of publication of application: **09.09.2026**
(21) Application number: 24884982.0
(22) Date of filing: 01.11.2024
(51) Int. Cl.: C07D 495/04, C07D 519/00, A61K 31/519, A61P 1/00, A61P 11/00, A61P 25/00

(54) **PHOSPHODIESTERASE 4B INHIBITOR, AND PREPARATION METHOD THEREFOR AND USE THEREOF**

(30) Priority: 03.11.2023 CN 202311452896
(71) Applicant: Hefei Institute of Pharmaceutical Industry Co., Ltd., Hefai, Anhui 230088 (CN); Hefei Amvite Pharmaceutical Co., Ltd., Hefei, Anhui 230601 (CN)
(72) Inventor: XU, Qinlong, Hefei, Anhui 230601 (CN); CHU, Zhaoxing, Hefei, Anhui 230601 (CN); WANG, Yan, Hefei, Anhui 230601 (CN); LIN, Gaofeng, Hefei, Anhui 230601 (CN); MO, Jiajia, Hefei, Anhui 230601 (CN); SHAO, Li, Hefei, Anhui 230601 (CN); ZHAO, Yan, Hefei, Anhui 230601 (CN); YE, Wenfeng, Hefei, Anhui 230601 (CN); HE, Guangwei, Hefei, Anhui 230601 (CN)
(74) Representative: Schulz Junghans Patentanwälte PartGmbB
(86) International application number: PCT/CN2024/129408
(87) International publication number: WO 2025/092985

(57) **Abstract**

Provided in the present invention are a phosphodiesterase 4B selective inhibitor, and a preparation method therefor and the use thereof. Specifically, provided in the present invention are a compound as represented by formula (I); or a stereoisomer, a tautomer, a solvate, a prodrug, an isotopic marker and a pharmaceutically acceptable salt thereof; and a pharmaceutical composition containing same. Further provided in the present invention is the use of the compound and/or pharmaceutical composition of the present invention in the preparation of a drug for preventing and/or treating inflammatory diseases and fibrotic diseases. The compound and/or pharmaceutical composition of the present invention exhibits relatively high phosphodiesterase 4B inhibitory activity.

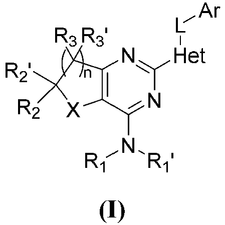

## Description

### TECHNICAL FIELD

The present invention relates to the field of medicinal chemistry, specifically to a class of selective phosphodiesterase 4B inhibitors and preparation method and use thereof.

### BACKGROUND ART

Inflammation is a very common and important basic pathological process. The defensive response of living tissues with vascular systems to damage factors is called inflammation. Inflammatory responses are closely related to a variety of diseases, such as dermatitis, psoriasis, and systemic lupus erythematosus, etc. For example, systemic lupus erythematosus is caused by gene alterations that lead to epidermal hyperplasia, which in turn triggers a series of inflammatory responses. Therefore, treatment of inflammation plays an important role in treating the above-mentioned diseases. Studies have shown that various inflammatory responses in the body are related to the molar concentration of intracellular cAMP. cAMP and cGMP play crucial regulatory roles in cellular activities; the regulation of their concentrations is primarily determined by the balance between synthesis of nucleotide cyclase and hydrolysis mediated by phosphodiesterases (PDEs).

Phosphodiesterases (PDEs) have the function of hydrolyzing intracellular second messengers cyclic adenosine monophosphate (cAMP) and cyclic guanosine monophosphate (cGMP). A decrease in intracellular cAMP or cGMP level can disrupt the biochemical processes mediated by these second messengers. PDEs are widely distributed in the human body, and their physiological effects involve multiple research areas. Excessive levels of PDEs in the body can lead to a decrease in cAMP concentration, thereby inducing diseases such as asthma, depression, psoriasis, and inflammation. There are 11 subtypes of PDEs, among which PDE4 plays a significant role in inflammatory cells. In recent years, PDEs have attracted widespread attention from many scholars as a new therapeutic target, and are becoming a new research hotspot.

There are four subtypes of PDE4, namely PDE4A to PDE4D. Existing PDE4 inhibitors such as rolipram, roflumilast, cilomilast, and apremilast are all non-selective PDE4 inhibitors. Due to poor selectivity, existing PDE4 inhibitors cause a variety of drug side effects, especially the inhibition of CNS PDE4D, which can lead to severe vomiting. International patent application WO2013026797 discloses a novel PDE4B inhibitor-compound II. However, further research has found that compound II has low selectivity for PDE4D and has caused vomiting as a side effect in clinical trials. Therefore, it is of great clinical significance to find and discover PDE4B inhibitors of better selectivity.

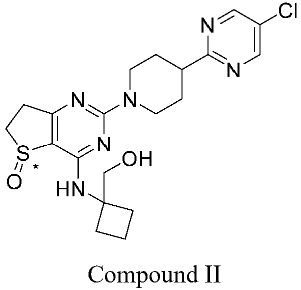

### SUMMARY OF THE INVENTION

To overcome the shortcomings of the prior art, on one hand, the present invention provides a compound having the following formula (I), 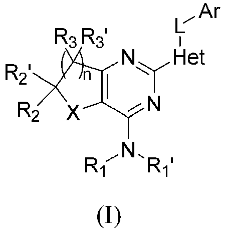
or a stereoisomer, tautomer, solvate, prodrug, isotopologue and/or pharmaceutically acceptable salt thereof, wherein
X is selected from S=O or S(=O)₂;
R₁ is selected from H or C₁ - C₆ alkyl;
R₁' is selected from C₃-C₈ carbocyclyl, C₆-C₁₀ aryl, 5-10 membered heteroaryl, or 5-10 membered heterocyclyl; wherein the carbocyclyl, aryl, heteroaryl, and heterocyclyl are substituted with R₄-(CRₐR_{b})ₘ- and optionally substituted with halogen, hydroxyl, amino, mercapto, C₁-C₆ alkyl, and/or C₁-C₆ alkoxyl;
R₂, R₂', R₃, and R₃' are each independently selected from hydrogen, C₁-C₆ alkyl, C₂-C₆ alkenyl, or C₂-C₆ alkynyl;
R₄ is selected from hydroxyl, amino, mercapto, carboxyl, -SO₃H, -PO₄H, tetrazolyl, triazolyl, -C(=O)OC₁-C₆ alkyl, or -C(=O)NR_{c}R_{d};
Rₐ and R_{b} are each independently selected from hydrogen, C₁-C₆ alkyl, halogen, hydroxyl, or Rₐ and R_{b}, together with the carbon atom to which they are attached, form a C₃-C₆ carbocyclyl; R_{c} and R_{d} are each independently selected from hydrogen, C₁-C₆ alkyl, or R_{c} and R_{d}, together with the nitrogen atom to which they are attached, form a 3- to 6-membered heterocyclic group;
Het is selected from 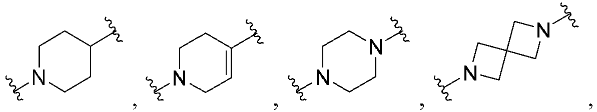 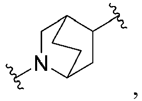 5- or 6-membered nitrogen-containing heteroaryl;
L is selected from a bond, C₁-C₆ alkylene, -C(=O)-, -NRₐC(=O)-, -C(=O)-N(Rₐ)-, -OC(=O)-, or -C(=O)O-;
Ar is selected from 5-10 membered heterocyclyl, 5-10 membered heteroaryl, C₆-C₁₀ aryl, or C₃-C₈ carbocyclyl; said heterocyclyl, heteroaryl, aryl, and carbocyclyl are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 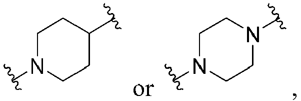 and R₁' is C₃-C₈ carbocyclyl, L is not a bond.

On the other hand, the present invention provides a pharmaceutical composition comprising the compound of formula (I) or a stereoisomer, tautomer, solvate, prodrug, isotopologue, and/or pharmaceutically acceptable salt thereof, and a pharmaceutically acceptable carrier.

In another aspect, the present invention provides the use of the compound of formula (I) or a stereoisomer, tautomer, solvate, prodrug, isotopologue and pharmaceutically acceptable salt thereof, or a pharmaceutical composition comprising the same, in the manufacture of a medicament for the prevention and/or treatment of a disease mediated by phosphodiesterase 4B.

Beneficial effects: compared with the prior art, the compounds of the present invention not only have significantly better inhibitory activity against phosphodiesterase 4B and stronger target selectivity, but also have better therapeutic effects on idiopathic pulmonary fibrosis.

### BRIEF DESCRIPTION OF THE DRAWINGS

Fig. 1 illustrates the results of lung tissue pathological examination of the effects of the compounds of the present invention on chronic obstructive pulmonary disease in rats.
Fig. 2 illustrates the results of lung tissue pathological examination of the effects of the compounds of the present invention on bleomycin-induced pulmonary fibrosis in rats.

### DETAILED DESCRIPTION OF THE INVENTION

### Definition

As used in the present specification, the following words and phrases are generally intended to have the meanings set forth below, unless the context in which they are used indicates otherwise.

As used herein, the term "alkyl" refers to a monovalent group of a straight or branched saturated hydrocarbon chain (typically having 1 to 6 carbon atoms, 1 to 4 carbon atoms, or 1 to 3 carbon atoms). This term is exemplified by groups such as methyl, ethyl, 1-propyl (n-propyl), 2-propyl (isopropyl), 1-butyl (n-butyl), 2-methyl-1-propyl (isobutyl), 2-butyl (sec-butyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, etc.

As used herein, the term "alkylene" refers to a divalent group of a straight or branched saturated hydrocarbon chain (typically having 1 to 6 carbon atoms, 1 to 4 carbon atoms, or 1 to 3 carbon atoms). The term is exemplified by groups such as methylene, ethylene, propylene, butylene, pentylene, hexylene, etc.

As used herein, the term "alkenyl" refers to a monovalent group of a straight or branched unsaturated hydrocarbon chain having 2 to 6 carbon atoms (more typically 2 to 4 carbon atoms, or 2 to 3 carbon atoms) and carbon-carbon double bond(s) (e.g., 1 or 2 carbon-carbon double bonds). The term is exemplified by groups such as vinyl (i.e., -CH=CH₂), propen-1-yl (i.e., -CH=CHCH₃), propen-3-yl (or allyl, i.e., -CH₂CH=CH₂), propen-2-yl (i.e., -C(CH₃)=CH₂), butadienyl (including 1,2-butadienyl and 1,3-butadienyl), etc.

As used herein, the term "alkynyl" refers to a monovalent group of a straight or branched unsaturated hydrocarbon chain (typically having 2 to 6 carbon atoms, 2 to 4 carbon atoms, or 2 to 3 carbon atoms) and having carbon-carbon triple bond(s) (e.g., 1 or 2 carbon-carbon triple bonds). The term is exemplified by groups such as ethynyl (i.e., -C=CH), propargyl (i.e., -CH₂C≡CH), propynyl (i.e., -C≡CCH₃), etc.

As used herein, the term "aryl" refers to an aromatic carbocyclic group having 6 to 14 carbon atoms (more typically having 6 to 10 carbon atoms, or 6 carbon atoms) that possesses a single ring (e.g., a phenyl group), multiple rings (e.g., biphenyl), or multiple fused rings (e.g., naphthyl, fluorenyl, and anthryl). The term is exemplified by groups such as phenyl, fluorenyl, naphthyl, anthryl, 1,2,3,4-tetrahydronaphthyl (if the point of attachment is through the aryl group), and the like.

As used herein, the term "halogen" refers to fluorine, chlorine, bromine, and iodine.

As used herein, the term "alkoxyl" refers to an "alkyl-O-" group, wherein the alkyl group is as defined herein. The term is exemplified by groups such as methoxyl, ethoxyl, n-propoxyl, isopropoxyl, n-butoxyl, isobutoxyl, tert-butoxyl, etc.

As used herein, the term "carbocyclyl" refers to a monovalent saturated or partially unsaturated group of 3 to 8 membered monocyclic ring, multiple fused rings, bridged rings or spiro rings having 3 to 8 carbon atoms as ring atoms. The carbocyclic ring or carbocyclyl can be saturated or partially unsaturated, and can be fused with another saturated, partially unsaturated, or aromatic ring, provided that the ring atom connected to the target molecule is not an aromatic carbon. Examples of carbocyclic ring or carbocyclyl include, but are not limited to, cyclopropane, cyclobutane, cyclopentane, cyclohexane, cyclobutene, cyclopentene, cyclohexene, cycloheptene, cyclopentadiene, etc.

As used herein, the term "heteroaryl" refers to an aromatic cyclic group comprising a monocyclic or multiple fused rings (e.g., comprising two rings) containing 5 to 10 ring atoms, wherein the ring atoms, in addition to carbon atoms, also contain at least one heteroatom selected from oxygen, nitrogen, and/or sulfur. The sulfur and nitrogen atoms may also be present in an oxidized form if the ring is aromatic. Multiple fused cyclic heteroaryl groups are formed by fusing a monocyclic heteroaryl group as defined above with one or more rings selected from the following to form a multiple fused ring system: heteroaryl (to form for example naphthyridinyl such as 1,8-naphthyridinyl), heterocycle (to form for example 1, 2, 3, 4-tetrahydronaphthyridinyl such as 1,2,3,4-tetrahydro-1,8-naphthyridinyl), carbocycle (to form for example 5,6,7,8-tetrahydroquinolyl) and aryl (to form for example indazolyl). It should be understood that the point of attachment ofheteroaryl can be on any suitable atom of the heteroaryl group, including carbon atoms and heteroatoms (e.g., nitrogen). Exemplary heteroaryls include, but not limited to, pyridyl, pyrrolyl, pyrazinyl, pyrimidinyl, pyridazinyl, pyrazolyl, thienyl, indolyl, imidazolyl, oxazolyl, isoxazolyl, thiazolyl, furyl, oxadiazolyl, thiadiazolyl, quinolyl, isoquinolyl, benzothiazolyl, benzoxazolyl, indazolyl, quinoxalyl, quinazolyl, 5,6,7,8-tetrahydroisoquinolinyl benzofuranyl, benzimidazolyl, thianaphthenyl, pyrrolo[2,3-b]pyridinyl, quinazolinyl-4(3H)-one, triazolyl, 4,5,6,7-tetrahydro-1H-indazolyl, and 3b,4,4a,5-tetrahydro-1H-cyclopropa[3,4]cyclopenta[1,2-c]pyrazole.

As used herein, the term "heterocyclyl" refers to a monovalent or divalent saturated or partially unsaturated group having 3 to 8-membered monocyclic or multiple fused rings, bridged rings or spiro rings having 3 to 14 ring atoms within the ring, wherein the ring atoms contain at least one nitrogen atom in addition to carbon atom. Examples of heterocyclyl include, but not limited to, azirridine ring, azetidine ring, tetrahydropyrrole ring, piperidine ring, azepane rin, azocane ring, tetrahydroimidazole ring, tetrahydropyrazole ring, tetrahydrooxazole ring, tetrahydroisooxazole ring, tetrahydrothiazole ring, tetrahydroisothiazole rin, piperazine ring, morpholine ring, dihydropyridyl , 4,5,6,7 -tetrahydro-1H-benzo[d]imidazole, 4,5,6,7-tetrahydro-1H-imidazo[4,5-c]pyridine, etc.

As used herein, the term "therapeutically effective amount" refers to an amount sufficient to effect treatment as defined below when administered to a mammal in need of such treatment. The therapeutically effective amount will vary with the subject and disease condition being treated, the weight and age of the subject, the severity of the disease condition, the manner of administration, etc., and can be readily determined by one of ordinary skill in the art.

As used herein, the term "stereoisomer" refers to a compound having the same chemical composition and connectivity, but the atoms of which have different orientations in space that cannot be interchanged by single bond rotation. "Stereoisomer" includes both "diastereoisomers" and "enantiomers". "Diastereoisomers" are stereoisomers that have two or more chiral centers and molecules of which are not mirror images of each other. Diastereoisomers have different physical properties, such as melting point, boiling point, spectral characteristics, and reactivity. Mixtures of diastereoisomers can be separated using high-resolution analytical procedures such as crystallization, electrophoresis, and chromatography. "Enantiomers" refer to two stereoisomers of a compound that are nonoverlapping mirror images of each other.

As used herein, the term "tautomer" refers to the coexistence of two (or more) compounds that differ only in the position and electron distribution of one (or more) active atoms, such as keto-enol tautomers.

As used herein, the term "pharmaceutically acceptable salts" refers to salts that retain the biological effectiveness and properties of a given compound, and which are not biologically or otherwise undesirable. Pharmaceutically acceptable salts may be acid addition salts and/or base addition salts. Acid addition salts can be prepared from inorganic and organic acids. Salts derived from inorganic acids include hydrochlorides, hydrobromides, sulfates, nitrates, phosphates, carbonates, bisulfates, hydrophosphates, dihydrophosphates, bicarbonates, and the like; salts derived from organic acids include formates, acetates, propionates, glycolates, pyruvates, oxalates, malates, malonates, succinates, maleates, fumarates, tartrates, citrates, benzoates, cinnamates, mandelates, methanesulfonates, ethanesulfonates, p-toluenesulfonates, salicylates, lactates, nicotinates, lauryl sulfates, naphthalenesulfonates, camphorsulfonates, gluconates, glucuronates, oleates, palmitates, stearates, pamoates, trifluoroacetates, etc. Base addition salts can be formed with inorganic or organic bases. Salts derived from inorganic bases include sodium, potassium, ammonium, calcium, magnesium, iron, zinc, copper, lithium, barium, aluminum salts and the like; salts derived from organic bases include salts formed with various primary, secondary and tertiary amines, such as ethylamine, diethylamine, n-propylamine, isopropylamine, diethanolamine, meglumine, lysine, piperazine, piperidine, morpholine, tromethamine, choline and the like.

As used herein, the term "pharmaceutically acceptable" means that the substance or composition must be chemically and/or toxicologically compatible with the other ingredients comprising the formulation and/or the mammal to be treated therewith.

As used herein, the term "solvate" refers to an association complex or complex of one or more solvent molecules and a compound of the present invention. Examples of solvents that form solvates include, but not limited to, water, isopropanol, ethanol, methanol, DMSO, ethyl acetate, acetic acid, and ethanolamine. The term "hydrate" refers to a complex in which the solvent molecule is water.

As used herein, the term "prodrug" refers to those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Any general formula or structure given herein, including general formula I or any general formula disclosed herein, is also intended to represent the unlabeled form and isotopically labeled form of the compound. These forms of compounds can also be called "isotopologue". Isotopologue has the structure described herein, except that one or more atoms are replaced by atoms with a selected atomic mass or mass number. Examples of isotopes that can be incorporated into the compounds of the present invention include isotopes of hydrogen, carbon, nitrogen, oxygen, phosphorus, fluorine, chlorine, and iodine, such as, but not limited to, ²H (deuterium, D), ³H (tritium), ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I. Various isotopologues of the present invention, such as those doped with radioactive isotopes (e.g., ³H, ¹³C, and ¹⁴C). These compounds are synthesized using methods well-known in the field, such as by using starting materials in which one or more hydrogens have been replaced by deuterium.

### Compounds

In one embodiment, the compound of the present invention is the compound represented by formula (I): 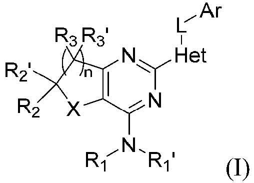 or a stereoisomer, tautomer, solvate, prodrug, isotopologue and/or pharmaceutically acceptable salt thereof, wherein X, R₁, R₁', R₂, R₂', R₃, R₃', Het, L, Ar, and n are as described above.

In one embodiment, the compound of the present invention is a compound of formula (I) or a stereoisomer, tautomer, solvate, prodrug, isotopologue, or pharmaceutically acceptable salt thereof, wherein,
X is selected from S=O or S(=O)₂;
R₁ is selected from H or C₁ - C₆ alkyl;
R₁' is selected from C₃-C₈ carbocyclyl, C₆-C₁₀ aryl, or 5-10 heteroaryl; wherein the carbocyclyl, aryl, and heteroaryl group are substituted with R₄-(CRₐR_{b})ₘ- and optionally substituted with halogen, hydroxyl, amino, mercapto, C₁-C₆ alkyl, and/or C₁-C₆ alkoxyl;
R₂, R₂', R₃, and R₃' are each independently selected from hydrogen or C₁-C₆ alkyl;
R₄ is selected from hydroxyl, amino, mercapto, carboxyl, -SO₃H, -PO₄H, tetrazolyl, triazolyl, -C(=O)OC₁-C₆ alkyl, or -C(=O)NR_{c}R_{d};
Rₐ and R_{b} are each independently selected from hydrogen, C₁-C₆ alkyl, halogen, hydroxyl, or Rₐ and R_{b}, together with the carbon atom to which they are attached, form a C₃-C₆ carbocyclyl; R_{c} and R_{d} are each independently selected from hydrogen, C₁-C₆ alkyl, or R_{c} and R_{d}, together with the nitrogen atom to which they are attached, form a 3- to 6-membered heterocyclyl;
Het is selected from 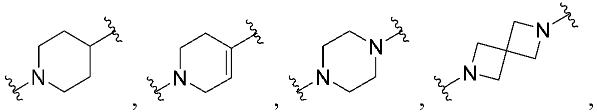 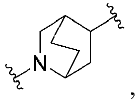 5- or 6-membered nitrogen-containing heteroaryl;
L is selected from a bond, C₁-C₆ alkylene, -C(=O)-, -NRₑC(=O)-, -C(=O)-N(Rₑ)-, - OC(=O)-, or -C(=O)O-; Rₑ is selected from hydrogen or C₁-C₆ alkyl;
Ar is selected from 5-10-membered heteroaryl or C₆-C₁₀ aryl; said heteroaryl and aryl are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 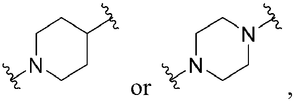 and R₁' is C₃-C₈ carbocyclyl, L is not a bond.

In one embodiment, the compound of the present invention is a compound of formula (I) or a stereoisomer, tautomer, solvate, prodrug, isotopologue, and/or pharmaceutically acceptable salt thereof, wherein,
R₁' is selected from cyclopropyl, cyclobutyl, cyclopentyl, cyclohexyl, phenyl, pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl; all of which are substituted by R₄-(CRₐR_{b})ₘ- and optionally substituted by halogen, hydroxyl, amino, mercapto, C₁-C₆ alkyl, and/or C₁-C₆ alkoxyl;
R₂, R₂', R₃, and R₃' are each independently selected from hydrogen or C₁-C₆ alkyl;
R₄ is selected from hydroxyl, amino, mercapto, carboxyl, -SO₃H, -PO₄H, tetrazolyl, triazolyl, -C(=O)OC₁-C₂₀ alkyl, or -C(=O)NR_{c}R_{d};
Rₐ and R_{b} are each independently selected from hydrogen, C₁-C₆ alkyl, halogen, hydroxyl, or Rₐ and R_{b}, together with the carbon atom to which they are attached, form a C₃-C₆ carbocyclyl; R_{c} and R_{d} are each independently selected from hydrogen, C₁-C₆ alkyl, or R_{c} and R_{d}, together with the nitrogen atom to which they are attached, form a 3- to 6-membered heterocyclyl;
Het is selected from 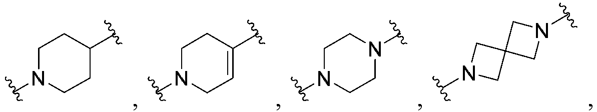 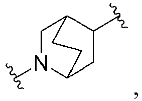 5- or 6-membered nitrogen-containing heteroaryl;
L is selected from a bond, C₁-C₆ alkylene, -C(=O)-, -NRₐC(=O)-, -C(=O)-N(Rₐ)-, - OC(=O)-, or -C(=O)O-;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl; all of which are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 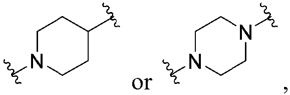 and R₁' is cyclopropyl, cyclobutyl, cyclopentyl, or cyclohexyl, L is not a bond.

In one embodiment, the compound of the present invention is a compound of formula (I) or a stereoisomer, tautomer, solvate, prodrug, isotopologue, and/or pharmaceutically acceptable salt thereof, wherein,
R₁' is selected from cyclobutyl, phenyl, or pyridyl; all of which are substituted by R₄-(CRₐR_{b})ₘ- and optionally substituted by halogen;
R₂, R₂', R₃, and R₃' are each independently selected from hydrogen or C₁-C₆ alkyl;
R₄ is selected from hydroxyl, amino, mercapto, carboxyl, -SO₃H, -PO₄H, tetrazolyl, triazolyl, -C(=O)OC₁-C₂₀ alkyl, or -C(=O)NR_{c}R_{d};
Rₐ and R_{b} are each independently selected from hydrogen, C₁-C₃ alkyl, or halogen, or Rₐ and R_{b}, together with the carbon atom to which they are attached, form cyclopropyl or cyclobutyl; R_{c} and R_{d} are each independently selected from hydrogen, C₁-C₃ alkyl, or R_{c} and R_{d}, together with the nitrogen atom to which they are attached, form a 3 to 6-membered heterocyclyl;
Het is selected from 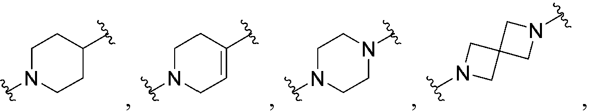 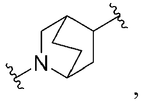 5- or 6-membered nitrogen-containing heteroaryl;
L is selected from a bond, C₁-C₆ alkylene, -C(=O)-, -NRₐC(=O)-, -C(=O)-N(Rₐ)-, - OC(=O)-, or -C(=O)O-;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl; all of which are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 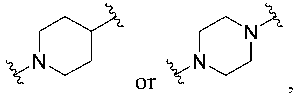 and R₁' is cyclobutyl, L is not a bond.

In one embodiment, the compound of the present invention is the compound represented by formula (II): 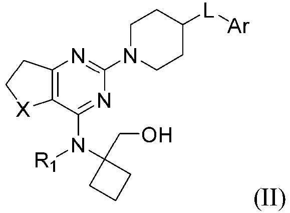
or a stereoisomer, tautomer, solvate, prodrug, isotopologue, and/or pharmaceutically acceptable salt thereof, wherein,
X is selected from S=O or S(=O)₂;
R₁ is selected from H or C₁ - C₆ alkyl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl; all of which are optionally substituted by one or more groups selected from the group consisting of: halogen, hydroxyl, C₁-C₆ alkyl, and C₁-C₆ alkoxyl;
with the proviso that: when X is S=O, L is not a bond.

In one embodiment, the compound of the present invention is the compound represented by formula (III): 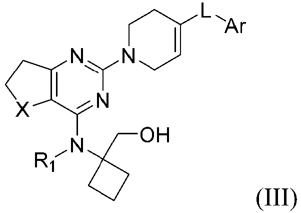
or a stereoisomer, tautomer, solvate, prodrug, isotopologue, and/or pharmaceutically acceptable salt thereof, wherein,
X is selected from S=O or S(=O)₂;
R₁ is selected from H or C₁ - C₆ alkyl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl; all of which are optionally substituted by one or more groups selected from the group consisting of: halogen, hydroxyl, C₁-C₆ alkyl, and C₁-C₆ alkoxyl.

In one embodiment, the compound of the present invention is the compound represented by formula (IV): 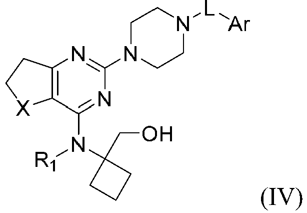
or a stereoisomer, tautomer, solvate, prodrug, isotopologue and/or pharmaceutically acceptable salt thereof, wherein,
X is selected from S=O or S(=O)₂;
R₁ is selected from H or C₁ - C₆ alkyl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl; all of which are optionally substituted by one or more groups selected from the group consisting of: halogen, hydroxyl, C₁-C₆ alkyl, and C₁-C₆ alkoxyl;
with the proviso that: when X is S=O, L is not a bond.

In one embodiment, the compound of the present invention is the compound represented by formula (V): 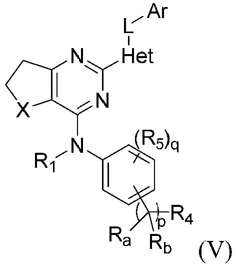
or a stereoisomer, tautomer, solvate, prodrug, isotopologue, and/or pharmaceutically acceptable salt thereof, wherein,
X is selected from S=O or S(=O)₂;
R₁ is selected from H or C₁ - C₆ alkyl;
R₄ is selected from carboxyl, -SO₃H, -PO₄H, tetrazolyl, triazolyl, -C(=O)OC₁-C₆ alkyl, or -C(=O)NR_{c}R_{d};
Rₐ and R_{b} are each independently selected from hydrogen, C₁-C₃ alkyl, or halogen, or Rₐ and R_{b}, together with the carbon atom to which they are attached, form cyclopropyl or cyclobutyl; R_{c} and R_{d} are each independently selected from hydrogen, C₁-C₃ alkyl, or R_{c} and R_{d}, together with the nitrogen atom to which they are attached, form a 3 to 6-membered heterocyclyl;
R₅ is selected from halogen, hydroxyl, amino, mercapto, C₁-C₆ alkyl, or C₁-C₆ alkoxyl;
Het is selected from 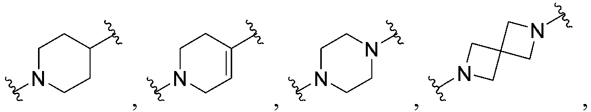 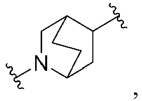 5- or 6-membered nitrogen-containing heteroaryl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl; all of which are optionally substituted by one or more groups selected from the group consisting of: halogen, hydroxyl, C₁-C₆ alkyl, and C₁-C₆ alkoxyl;
p is selected from 1, 2, or 3; q is selected from 1, 2, or 3.

In one embodiment, the compound of the present invention is a compound of formula (V) or a stereoisomer, tautomer, solvate, prodrug, isotopologue, or pharmaceutically acceptable salt thereof, wherein,
X is selected from S=O or S(=O)₂;
R₁ is selected from H or C₁ - C₆ alkyl;
R₄ is selected from carboxyl, -SO₃H, -PO₄H, tetrazolyl, triazolyl, or -C(=O)OC₁-C₆ alkyl;
Rₐ and R_{b} are each independently selected from hydrogen or halogen, or Rₐ and R_{b}, together with the carbon atom to which they are attached, form cyclopropyl or cyclobutyl;
R₅ is selected from halogen, hydroxyl, amino, mercapto, C₁-C₆ alkyl, or C₁-C₆ alkoxyl;
Het is selected from 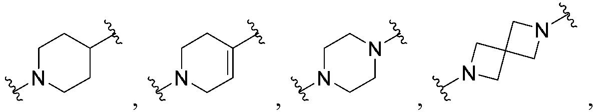 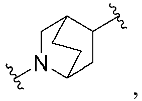 or triazolyl;
L is selected from a bond, C₁-C₆ alkylene, -C(=O)-, -NRₑC(=O)-, -C(=O)-N(Rₑ)-, - OC(=O)-, or -C(=O)O-; Rₑ is selected from hydrogen or C₁-C₆ alkyl;
Ar is selected from pyridinyl, pyrimidinyl, or phenyl; all of which are optionally substituted by one or more groups selected from the group consisting of: halogen, hydroxyl, C₁-C₆ alkyl, and C₁-C₆ alkoxyl;
p is selected from 1; q is selected from 1.

In one embodiment, the compounds of the present invention include, but are not limited to, the following compounds: 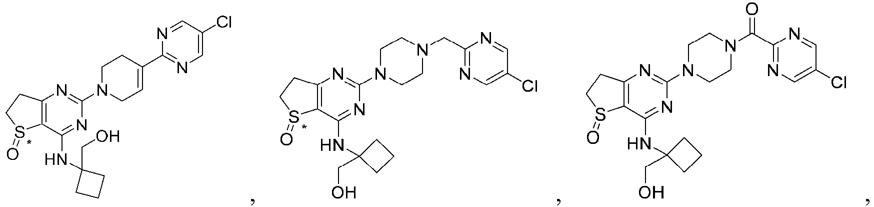 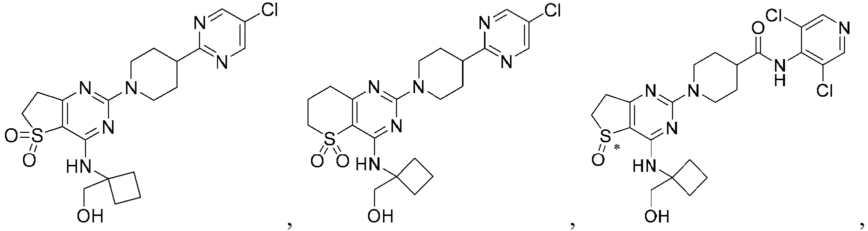 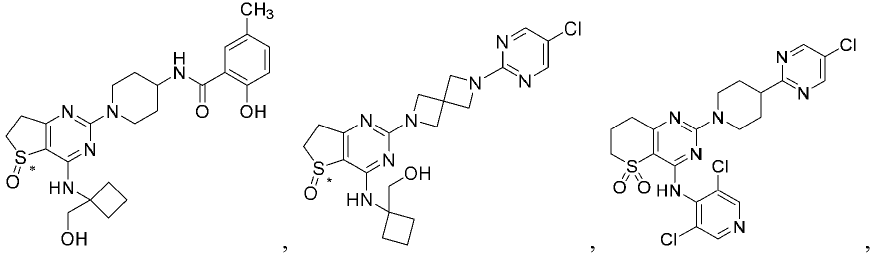 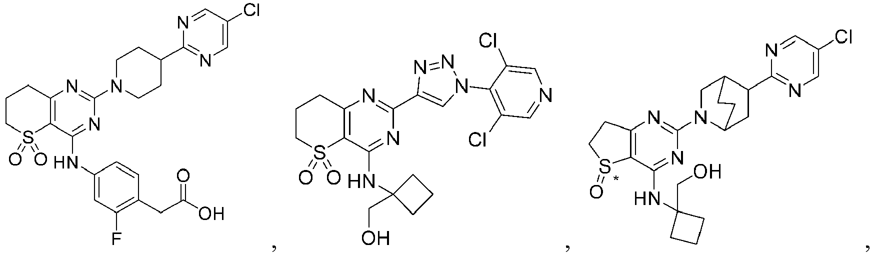 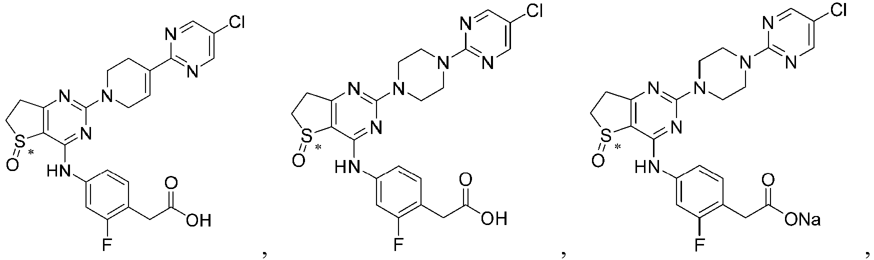 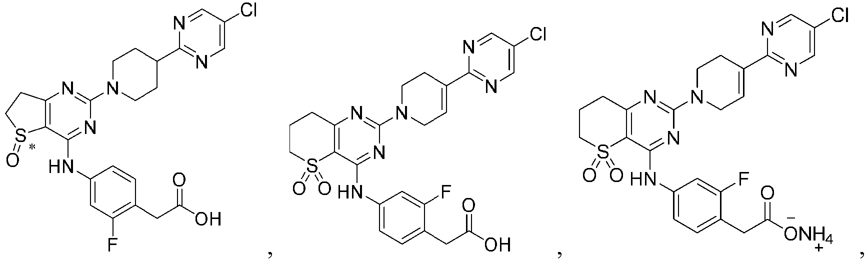 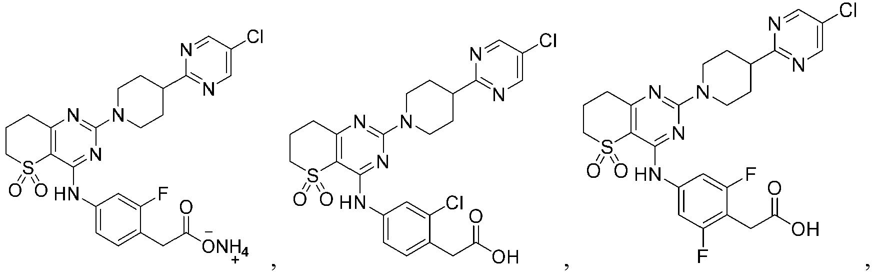 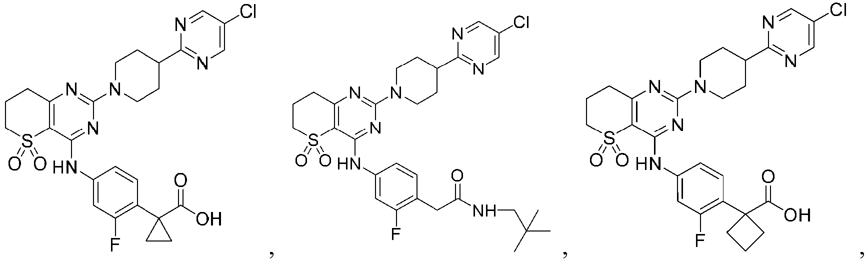 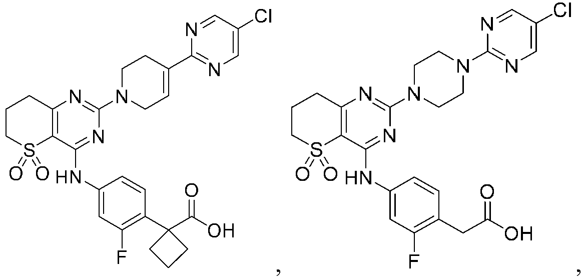 or a stereoisomer, tautomer, solvate, prodrug, and/or pharmaceutically acceptable salt thereof.

### Pharmaceutical composition and administration

The pharmaceutical composition provided by the present invention comprises the compound of the present invention or a stereoisomer, tautomer, solvate, prodrug, isotopologue and/or pharmaceutically acceptable salt thereof, and at least one pharmaceutically acceptable carrier. The pharmaceutically acceptable carriers are known to those skilled in the art, including diluents, lubricants, disintegrants, binders, buffers, preservatives, stabilizers, wetting agents, flow aids, emulsifiers, colorants, flavoring agents, sweeteners, etc. Depending on the route of administration, such as oral administration, parenteral administration, and rectal administration, the pharmaceutical composition of the present invention can be prepared in solid form (including but not limited to tablets, capsules, pills, granules, powders, pulvis, suppositories) or liquid form (including but not limited to solutions, suspensions, emulsions, tinctures, and syrups). When the pharmaceutical composition of the present invention is in solid form, the pharmaceutically acceptable carrier typically includes one or more of the following: a) a diluent, such as lactose, glucose, sucrose, mannitol, sorbitol, cellulose, etc.; b) a lubricant, such as silica, talc, stearic acid, polyethylene glycol, etc.; c) a binder, such as magnesium aluminosilicate, gelatinized starch, gelatin, astragalus gum, methylcellulose, sodium carboxymethyl cellulose, microcrystalline cellulose, polyvinylpyrrolidone, etc.; d) a disintegrant, such as starch, alginic acid, agar, corn starch; e) a stabilizer, such as an antioxidant like ascorbic acid; f) a flow aid, such as silica; g) a flavoring agent, such as peppermint, methyl salicylate; and a sweetener, such as sucrose, saccharin. When the pharmaceutical composition of the present invention is in liquid form, the pharmaceutically acceptable carrier typically includes one or more of the following: a) a diluent, such as water for injection, physiological saline, Ringer's solution, polyethylene glycol, glycerin, propylene glycol, etc.; b) an antioxidant, such as ascorbic acid or sodium bisulfite; c) a buffer, such as acetate, phosphate, etc.

The effective dosage of the compounds of the present invention depends at least on the nature and extent of the disease being treated, the method of delivery, and the drug formulation, and will be ultimately determined by the clinician. It can be expected to be about 0.0001 to about 100 mg per kilogram of body weight per day; usually about 0.01 to about 10 mg per kilogram of body weight per day; more typically, about 0.01 to about 5 mg per kilogram of body weight per day; most typically about 0.05 to about 0.5 mg per kilogram of body weight per day. For example, the daily candidate dose for an adult weighing approximately 70 kg will be in the range of 1 mg to 1000 mg, preferably in the range of 5 mg to 500 mg, and can be administered in single or multiple doses.

### Indications

The compounds of the present invention exhibit extremely strong inhibitory activity against PDE4B and can be used to prevent and/or treat inflammatory diseases associated with PDE4B, including but not limited to atopic dermatitis, asthma, chronic obstructive pulmonary disease (COPD), idiopathic pulmonary fibrosis, interstitial lung disease, chronic sinusitis, allergic rhinitis, allergic dermatitis, contact dermatitis, psoriasis, systemic lupus erythematosus, ulcerative colitis, segmental ileitis, depression, bipolar disorder, mania, anxiety, schizophrenia, Alzheimer's disease, stroke, chronic pain, liver fibrosis, kidney fibrosis, and nephritis.

### Examples

### Example 1

### Step 1: Synthesis of tert-butyl 4-(5-chloropyrimidin-2-yl)-3,6-dihydropyridine-1(2H)-carboxylate (2)

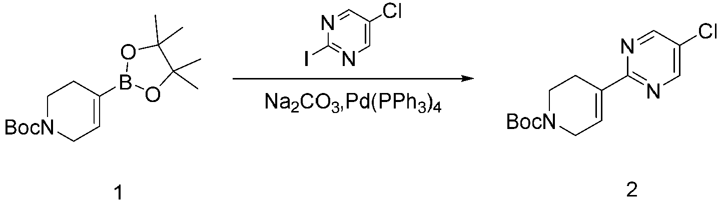

To a 100 mL single-necked flask, were added tert-butyl 4-(4,4,5,5-tetramethyl-1,3,2-dioxaborolan-2-yl)-3,6-dihydropyridin-1(2*H*)-yl-carboxylate (1, 1.00 g, 3.23 mmol), 5-chloro-2-iodopyrimidine (932 mg, 3.88 mmol), dioxane (10 mL), H₂O (2 mL), and Na₂CO₃ (1.02 g, 9.70 mmol) in sequence. The mixture was purged with nitrogen three times. Finally, Pd(PPh₃)₄ (186 mg, 0.16 mmol) was added, and the mixture was reacted at 90°C for 16 h. TLC (*V*_{petroleum ether} : *V*_{ethyl acetate} = 10:1) monitored that the starting materials have completely reacted. Water (50 mL) was added to the reaction solution, and the mixture was extracted with ethyl acetate (50 mL × 3). The organic phases were combined, dried with anhydrous sodium sulfate, filtered, concentrated, and purified by column chromatography (SiO₂, *V*_{petroleum ether} : *V*_{ethyl acetate} = 20:1) to obtain 510 mg of white solid **2,** with a yield of 53.4%. ¹H NMR (400 MHz, CDCl₃) *δ* (ppm): 8.62 (s, 2H, ArH), 7.19 (s, 1H, CH), 4.17 (m, 2H, CHCH₂), 3.63 (m, 2H, NCH₂), 2.68 (m, 2H, CCH₂), 1.49 (s, 9H, C(CH₃)₃).

### Step 2 Synthesis of 5-chloro-2-(1,2,3,6-tetrahydropyridin-4-yl)pyrimidine hydrochloride (3)

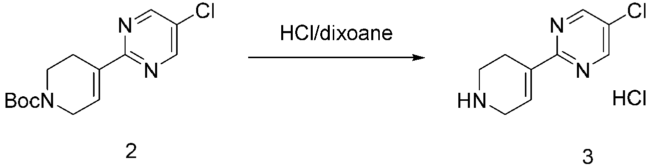

**2** (510 mg, 1.72 mmol), anhydrous dichloromethane (2 mL), and HCl (4M dioxane solution, 6 mL) were added to a 50 mL single-necked flask in sequence. Under nitrogen protection, the reaction was carried out at room temperature for 2 hours. TLC (*V*_{petroleum ether} : *V*_{ethyl acetate} = 10:1) monitored that the starting materials have completely reacted. The reaction solution was directly concentrated to obtain 390 mg of white solid crude product **3,** which was used directly in the next step without purification.

¹H NMR (400 MHz, DMSO- *d*₆) *δ* (ppm): 9.03 (s, 2H, NH, HCl), 8.95 (s, 2H, ArH), 7.16 (s, 1H, C=CH), 3.86 (d, *J =*4.4 Hz, 2H, CHCH₂), 3.38 (m, 2H, NHCH₂), 2.77 (d, *J*=2.1 Hz, 2H, CCH₂).

### Step 3: Synthesis of (1-aminocyclobutyl)methanol hydrochloride (5)

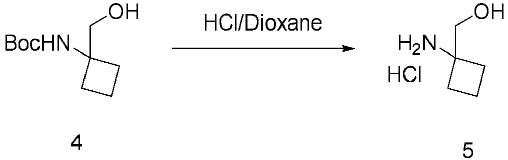

Tert-butyl (1-(hydroxymethyl)cyclobutyl)carbamate (4, 3.00 g, 14.90 mmol) and dichloromethane (6 mL) were added sequentially to a 50 mL Schlenk reaction tube. After stirring to dissolve, 1,4-dioxane solution of hydrogen chloride (4.0 M, 6 mL) was added dropwise to the reaction system at 0°C. After the addition is completed, the reaction was carried out at room temperature for 3 h under nitrogen protection. TLC (*V*_{dichloromethane} : *V*ₘₑₜₕₐₙₒₗ = 50:1) monitored that the starting materials have completely reacted. The reaction solution was concentrated under reduced pressure, and the crude product was slurried with ethyl acetate (5 mL), filtered, and dried to obtain 1.98 g of white solid **5,** with a yield of 96.6%.

¹H NMR (600 MHz, DMSO- *d*₆) *δ* (ppm): 8.20 (br, 3H, NH ₂, HCl), 5.47 (t, *J* =5.3 Hz, 1H, OH), 3.55 (d, *J*=5.3 Hz, 2H, CH₂OH), 2.23-2.11 (m, 2H, CH₂CH₂CH₂), 2.05-1.96 (m, 2H, CH₂CH₂CH₂), 1.90-1.81 (m, 1H, H of CH₂CH₂CH₂), 1.79-1.71 (m, 1H, H of CH₂CH₂CH₂); HRMS (ESI): m/z [M-HCl+H]⁺ Calcd for C₅H₁₁NO: 102.0919; Found: 102.0915.

### Step 4 Synthesis of (1-((2-chloro-6,7-dihydrothiopheno[3,2-d]pyrimidin-4-yl)amino)cyclobutyl)methanol (7)

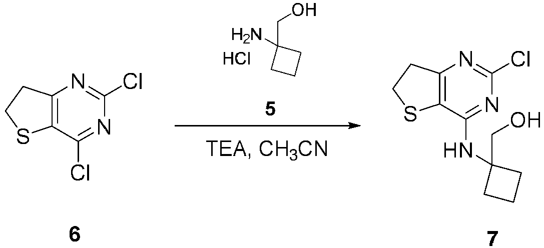

2,4-dichloro-6,7-dihydrothiopheno[3,2-d]pyrimidine **(6,** 1.37 g, 6.60 mmol), **5** (1.00 g, 7.27 mmol), acetonitrile (8 mL) and triethylamine (3.34 g, 33.00 mmol) were added in sequence to 50 mL of Schlenk, and reacted overnight at 76°C under nitrogen protection. TLC (*V*_{petroleum ether} : *V*_{ethyl acetate} = 1:1) monitored that the reaction was completed, with approximately 30% of the reactants being remained. After the reaction solution was concentrated under reduced pressure, it was purified by flash preparative chromatography (20 g, *V*_{petroleum ether} : *V*_{ethyl acetate} = 1:1) to obtain 1.08 g of white solid **7,** with a yield of 60.0%.

¹H NMR (600 MHz, CDCl₃) *δ* (ppm): 4.67 (s, 1H, NH), 3.90 (s, 3H, CH₂OH), 3.44-3.39 (m, 2H, SCH₂), 3.31-3.23 (m, 2H, SCH₂CH₂), 2.41-2.32 (m, 2H, CH₂CH₂CH₂), 2.19-2.11 (m, 2H, CH₂CH₂CH₂), 2.03-1.94 (m, 1H, H of CH₂CH₂CH₂), 1.94-1.85 (m, 1H, H of CH₂CH₂CH₂); HRMS (ESI): m/z [M+H]⁺ Calcd for C₁₁H₁₄ClN₃OS: 272.0624; Found: 272.0604.

### Step 5 Synthesis of 2-chloro-4-((1-(hydroxymethyl)cyclobutyl)amino)-6,7-dihydrothiopheno[3,2-d]pyrimidine-5-oxide (8)

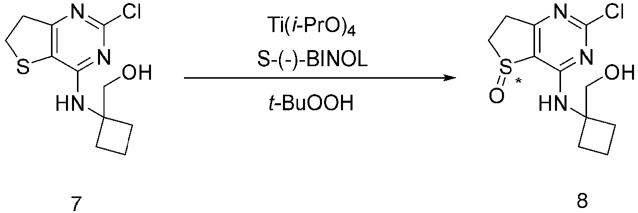

**7** (500 mg, 1.84 mmol), S-(-)-BINOL (53 mg, 0.184 mmol), dichloromethane (3 mL), tetraisopropyl titanate (26 mg, 0.092 mmol), and water (33 mg, 1.84 mmol) were added in sequence to a 50 mL Schlenk reaction tube. After the addition was complete, stirring was performed at room temperature for 1 h under nitrogen protection. Then tert-butanol peroxide (262 mg, 2.03 mmol) was added. After the addition was completed, the reaction was carried out at room temperature for 2 h under nitrogen protection. TLC (*V*_{dichloromethane} : *V*ₘₑₜₕₐₙₒₗ = 15:1) monitored that the starting materials have completely reacted. The system was directly purified by column chromatography without any working up (SiO₂, *V*_{dichloromethane} : *V*ₘₑₜₕₐₙₒₗ = 15:1) to obtain 340 mg of pale yellow solid **8,** with a yield of 64.2%.

¹H NMR (600 MHz, DMSO-*d*₆) *δ* (ppm): 8.61 (s, 1H, NH), 4.90 (t, *J=5.6* Hz, 1H, OH), 3.75-3.65 (m, 2H, CH₂OH), 3.60-3.52 (m, 1H, H of SCH₂), 3.39-3.34 (m, 1H, H of SCH₂), 3.18-3.10 (m, 1H, H of SCH₂CH₂), 3.06-2.99 (m, 1H, H of SCH₂CH₂), 2.31-2.15 (m, 4H, CH₂CH₂CH₂), 1.83-1.69 (m, 2H, CH₂CH₂CH₂); HRMS (ESI): m/z [M+H]⁺ Calcd for C₁₁H₁₄ClN₃O₂S: 288.0573; Found: 288.0554.

### Step 6 Preparation of 2-(4-(5-chloropyrimidin-2-yl)-3,6-dihydropyridin-1(2H)-yl)-4-((1-(hydroxymethyl)cyclobutyl)amino)-6,7-dihydrothiopheno[3,2-d]pyrimidine-5-oxide (Example 1)

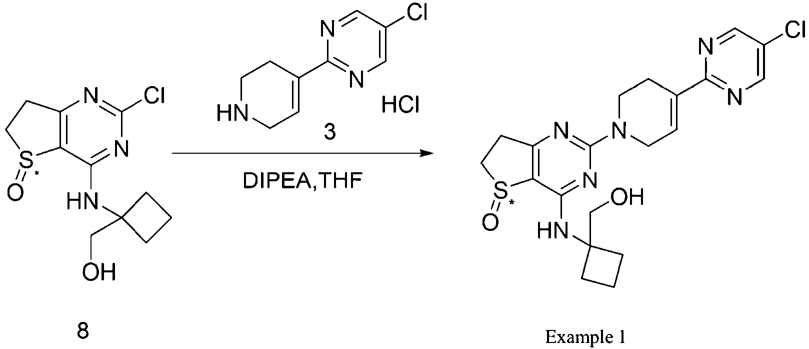

**3** (168 mg, 0.72 mmol), **8** (200 mg, 0.69 mmol), *N,N*-diisopropylethylamine (224 mg, 1.73 mmol), tetrahydrofuran (8 mL) and water (2 mL) were added in sequence to a 50 mL single-necked flask. After the addition was complete, purging was performed with nitrogen for three times and the reaction was carried out at 65°C overnight. TLC (*V*_{dichloromethane} : *V*ₘₑₜₕₐₙₒₗ = 10:1)monitored that the starting materials have completely reacted. Water (20 mL) was added to the reaction solution, and the mixture was extracted with ethyl acetate (20 mL × 3). The organic phases were combined, dried with anhydrous sodium sulfate, filtered, concentrated, and purified by thick preparative plate (*V*_{dichloromethane} : *V*ₘₑₜₕₐₙₒₗ = 10:1) to obtain 104 mg of white solid of the **compound of Example 1,** with a yield of 44.6%.

¹H NMR (400 MHz, DMSO-*d*₆) *δ* (ppm): 8.88 (s, 2H, ArH), 7.37 (s, 1H, C=CH), 7.28 (s, 1H, NH), 4.84 (t, *J*=5.7 Hz, 1H, OH), 4.45 (s, 2H, CH₂OH), 3.96 (t, *J=*5.6 Hz, 2H, NCH₂CH₂), 3.76-3.69 (m, 2H, NCH₂CH), 3.41 (d*, J=*8.5 Hz, 1H, H of SCH₂), 3.22-3.14 (m, 1H, H of SCH₂), 2.98-2.90 (m, 1H, H of SCH₂CH₂), 2.89-2.82 (m, 1H, H of SCH₂CH₂), 2.64 (s, 2H, NCH₂CH₂), 2.35 (m, 2H, H of CH₂CH₂CH₂), 2.25-2.16 (m, 2H, H of CH₂CH₂CH₂), 1.78 (d, *J=*9.5 Hz, 2H, H of CH₂CH₂CH₂); ¹³C NMR (100 MHz, DMSO-*d*₆) *δ* (ppm): 175.32, 162.09, 158.12, 156.22, 128.79, 58.97, 48.94, 29.47, 14.41.; HRMS (ESI): m/z [M+H]⁺ Calcd for C₂₀H₂₃ClN₆O₂S: 447.1370; Found: 447.1363

### Example 2

### Step 1: Synthesis of 2-bromomethyl-5-chloropyrimidine (2)

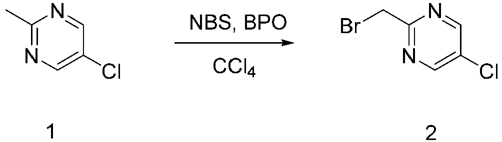

**Compound 1** (1.0 g, 7.78 mmol) and carbon tetrachloride (20 mL) were added in sequence to a 50 mL single-necked flask. NBS (1.52 g, 8.56 mmol) and BPO (0.19 g, 0.78 mmol) were added by stirring. After the addition was complete, the mixture was refluxed under nitrogen protection for 36 h. TLC (*V*_{petroleum ether} : *V*_{ethyl} acetate = 10:1) monitored approximately 30% of the reactants being remained. The reaction solution was cooled to room temperature, filtered, and the filter cake was washed with dichloromethane (10 mL). The filtrate was concentrated and purified by flash preparative chromatography (12 g, *V*_{petroleum ether} : *V*_{ethyl acetate} = 10:1) to obtain 320 mg of white solid **intermediate 2,** yield 17.8%.

¹ H NMR (600 MHz, CDCl₃) *δ* (ppm): 8.68 (s, 2H, ArH), 4.58 (s, 2H, CH₂).

### Step 2 Synthesis of tert-butyl 4-((S-chloropyrimidin-2-yl)methyl)piperazine-1-carboxylate (3)

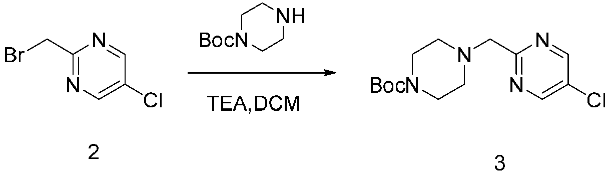

**Intermediate** 2 (0.35 g, 1.69 mmol), N-Boc-piperazine (0.38 g, 2.03 mmol), and dichloromethane (20 mL) were added in sequence to a 50 mL single-necked flask. The mixture was cooled to 0°C, and then triethylamine (0.21 g, 2.03 mmol) was added. After the addition was complete, the mixture was reacted at room temperature for 3 h under nitrogen protection. TLC (ethyl acetate) monitored that the reaction was completed. The reaction was quenched with water (15 mL), and the reaction solution was extracted with dichloromethane (30 mL × 3). The organic phases were combined, washed with saturated sodium chloride solution (50 mL × 3), dried with anhydrous sodium sulfate, concentrated, and purified by flash preparative chromatography (12 g, *V*_{petroleum ether} : *V*_{ethyl acetate} = 1 : 1) to obtain 281 mg of white solid **intermediate 3,** with a yield of 53.3%.

¹H NMR (600 MHz, DMSO -d₆) *δ* (ppm): 8.86 (s, 2H, ArH), 3.70 (s, 2H, CH₂), 3.27-3.23 (m, 4H, piperazine-H), 2.41-2.39 (m, 4H, piperazine-H), 1.34 (s, 9H, C(CH₃)₃).

### Step 3 Synthesis of 1-((5-chloropyrimidin-2-yl)methyl)piperazine trifluoroacetate (4)

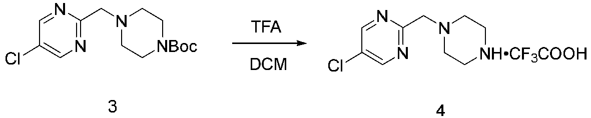

**Intermediate 3** (0.28 g, 0.9 mmol) and dichloromethane (6 mL) were added in sequence to a 25 mL single-necked flask. The mixture was cooled to 0°C, and TFA (2 mL) was added. After the addition was complete, the mixture was reacted at room temperature for 1 h under nitrogen protection. TLC (ethyl acetate) monitored that the reaction was completed. The solution was concentrated to obtain 190 mg of white solid **intermediate 4,** with a yield of 99%.

### Step 4 Synthesis of 2-(4-((S-chloropyrimidin-2-yl)methyl)piperazin-1-yl)-4-((1-(hydroxymethyl)cyclobutyl)amino)-6,7-dihydrothiopheno[3,2-d]pyrimidine 5-oxide (Example 2)

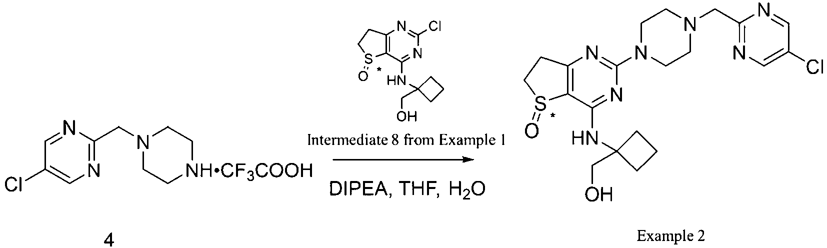

**Intermediate 4** (0.095 g, 0.45 mmol), **Intermediate 8** (0.129 g, 0.45 mmol) **of Example 1,** DIPEA (0.174 g, 1.35 mmol), THF (4 mL), and H₂O (0.5 mL) were added in sequence to a 25 mL single-necked flask. After the addition was complete, the mixture was reacted overnight at 65°C under nitrogen protection. TLC (*V*_{dichloromethane} : *V*ₘₑₜₕₐₙₒₗ = 20:1) monitored that the reaction was completed. Concentration was performed under reduced pressure, THF (4 mL) was added, then slurried for 1 h, filtered, the filter cake was dried to obtain 100 mg of white solid **Example 2,** with a yield of 47.9%.

¹H NMR (400 MHz, CDCl₃) *δ* (ppm): 8.68 (s, 2H, ArH), 5.90 (s, 1H, NH), 4.13 (s, 1H, OH), 3.91-3.84 (m, 4H, Piperazine-H), 3.84-3.82 (m, 4H, Piperazine-H), 3.61-3.50 (m, 1H, H of CH₂), 3.41-3.32 (m, 1H, H of CH₂), 3.05-2.91 (m, 2H, CH₂), 2.58 (t, J=4.9 Hz, 4H, CH₂, CH₂), 2.29-2.21 (m, 4H, CH₂CH₂), 1.97-1.89 (m, 1H, H of CH₂), 1.87-1.82 (m, 1H, H of CH₂).

¹³C NMR (151 MHz, DMSO-*d*₆) *δ* (ppm): 175.50, 165.42, 162.37, 158.18, 156.13, 129.73, 109.85, 64.11, 63.86, 59.01, 52.83, 48.97, 44.21, 32.96, 30.19, 30.03, 14.90.

MS (ESI): m/z [M+H]⁺ Calcd for C₂₀H₂₆ClN₇O₂S: 464.1635; Found: 464.1625.

### Example 3

### Step 1 Synthesis of tert-butyl 4-(5-chloropyrimidin-2-ylformyl)piperazine-1-carboxylate (2)

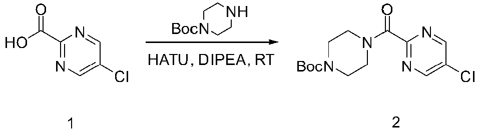

**Compound 1** (500 mg, 3.15 mmol), DMF (10 mL), HATU (1.37 g, 3.6 mmol), and DIPEA (0.84 mL, 4.8 mmol) were added in sequence to a 100 mL single-necked flask under nitrogen protection and stirred at room temperature for 0.5 h. Then, tert-butyl piperazin-1-ylcarboxylate (447 mg, 2.4 mmol) was added, and the mixture was reacted overnight at room temperature. TLC (*V*_{dichloromethane} : *V*ₘₑₜₕₐₙₒₗ = 20:1) monitored that the starting materials have completely reacted. After it was quenched with water (30 mL), extracted with ethyl acetate (30 mL × 3), the organic phases were combined, dried with anhydrous sodium sulfate, filtered, concentrated, and purified by column chromatography (*V*_{dichloromethane} : *V*ₘₑₜₕₐₙₒₗ = 15:1), and 370 mg of yellow solid **intermediate 2** was obtained, with a yield of 47.0%.

¹H NMR (600MHz, DMSO-*d*₆) *δ* (ppm): 9.04 (s, 2H, ArH), 3.65-3.61 (m, 2H, piperazine-CH₂), 3.44-3.41 (m, 2H, piperazine-CH₂), 3.29-3.25 (m, 2H, piperazine-CH₂), 3.20-3.17 (m, 2H, piperazine-CH₂), 1.40 (s, 9H, C(CH₃)₃).

### Step 2 Synthesis of (5-chloropyrimidin-2-yl)(piperazin-1-yl)methanone hydrochloride (3)

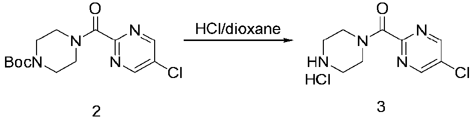

**Compound 2** (320 mg, 0.98 mmol) and 1 M HCl/dioxane (6 mL) solution were added to a 50 mL single-necked flask, and the mixture was reacted at room temperature for 1 h under nitrogen protection. TLC (*V*_{dichloromethane} : *V*ₘₑₜₕₐₙₒₗ = 20:1) monitored that the starting materials have completely reacted. The reaction solution was concentrated to dryness under reduced pressure, and ethyl acetate (30 mL) was added, followed by slurry and filtration, to obtain 150 mg of yellow solid 3, with a yield of 58.2%.

¹H NMR (400MHz, D₂O) *δ* (ppm): 8.88 (s, 2H, ArH), 3.95 (t, *J* = 5.6 Hz, 2H, piperazine-CH₂), 3.66 (t, *J* = 5.2 Hz, 2H, piperazine-CH₂), 3.35 (t, *J* = 5.6 Hz, 2H, piperazine-CH₂), 3.23 (t, *J =* 5.2 Hz, 2H, piperazine-CH₂).

### Step 3: Synthesis of (5-chloropyrimidin-2-yl)(4-(4-((1-(hydroxymethyl)cyclobutyl)amino)-5-oxo-6,7-dihydrothieno[3,2-d]pyrimidin-2-yl)piperazin-1-yl)methanone (Example 3)

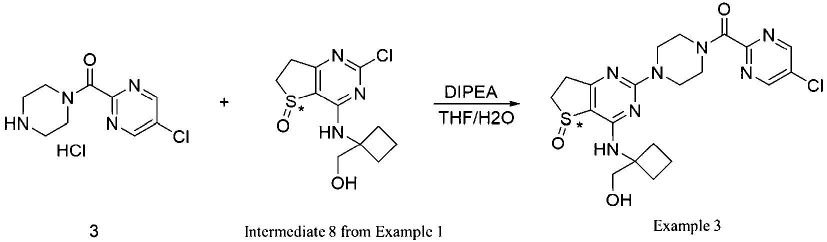

**Intermediate 3** (150 mg, 0.57 mmol), **Intermediate 8** (156 mg, 0.54 mmol) of **Example 1,** tetrahydrofuran (8 mL), DIPEA (0.24 mL, 1.35 mmol), and water (2 mL) were added in sequence to a 50 mL single-necked flask. The mixture was then reacted overnight at 65°C under nitrogen protection. TLC (*V*_{dichloromethane} : *V*ₘₑₜₕₐₙₒₗ = 20:1) monitored that the starting materials have completely reacted. Water (10 mL) was added to the reaction solution, the mixture was extracted with ethyl acetate (20 mL × 3), and the organic phases were combined, washed with saturated brine (20 mL), dried with anhydrous sodium sulfate, filtered, concentrated and purified by flash preparative chromatography (4 g, *V*_{dichloromethane} : *V*ₘₑₜₕₐₙₒₗ = 20:1), and 200 mg of crude product was obtained. The crude product was slurried with tetrahydrofuran (10 mL) to obtain 170 mg of white solid **Example 3,** with a yield of 65.9%.

¹H NMR (600MHz, CDCl₃) *δ* (ppm): 8.79 (s, 2H, ArH), 6.29 (s, 1H, NH), 4.07-3.93 (m, 3H, piperazine-H), 3.89-3.81 (m, 5H, piperazine-H), 3.78-3.72 (m, 1H, OH), 3.62-3.53 (m, 1H, H of SOCH₂), 3.43-3.33 (m, 3H, 1H of SOCH₂, 2H of SOCH₂CH₂), 3.08-2.94 (m, 2H, CH₂OH), 2.37-2.24 (m, 2H, NHCCH₂),2.23-2.14 (m, 2H, NHCCH₂),1.93-1.81 (m, 2H, NHCCH₂CH₂).

¹³C NMR (150MHz, DMSO-*d*₆) *δ* (ppm): 175.50, 164.52, 162.37, 159.99, 158.19, 156.84, 131.29, 110.54, 64.08, 59.06, 49.05, 46.32, 44.60, 43.83, 41.56, 33.03, 30.21, 30.05, 14.92.

HRMS (ESI): m/z [M+H]⁺ Calcd for C₂₀H₂₄ClN₇O₃S: 478.1428; Found: 478.1399.

### Example 4

### Step 1 Synthesis of 2-chloro-4-((1-(hydroxymethyl)cyclobutyl)amino)-6,7-dihydrothiopheno[3,2-d]pyrimidine 5,5-dioxide (2)

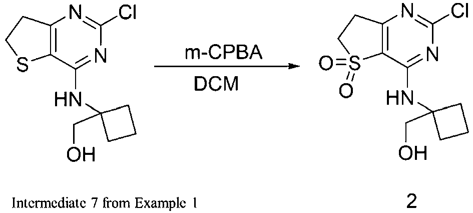

**Intermediate 7 from Example 1** (100 mg, 0.37 mmol), m-chloroperoxybenzoic acid (85%, 189 mg, 0.93 mmol) and dichloromethane (4 mL) were added in sequence to a 25 mL single-necked flask. After the addition was complete, the mixture was reacted at room temperature for 7 h. TLC (*V*_{petroleum ether} : *V*_{ethyl acetate} = 1:1) monitored that the starting materials have completely reacted. Dichloromethane (10 mL) was added to the reaction solution, which was then washed successively with saturated sodium thiosulfate solution (10 mL), saturated sodium bicarbonate solution (10 mL), and saturated saline solution (10 mL) after dilution. The solution was dried over anhydrous sodium sulfate, filtered, concentrated, and purified by flash preparative chromatography (4 g, *V*_{petroleum ether} : *V*_{ethyl acetate} = 1:1) to obtain 79 mg of white solid **intermediate 2,** with a yield of 70.3%.

¹H NMR (600 MHz, DMSO-*d*₆) *δ* (ppm): 7.53 (s, 1H, NH), 4.99 (t, *J=5.6* Hz, 1H, OH), 3.67 (d, *J*=5.7 Hz, 2H, CH₂OH), 3.63 (t, *J*=6.9 Hz, 2H, SCH₂), 3.26-3.20 (m, 2H, SCH₂CH₂), 2.40-2.30 (m, 2H, CH₂CH₂CH₂), 2.20-2.12 (m, 2H, CH₂CH₂CH₂), 1.86-1.69 (m, 2H, CH₂CH₂CH₂).

HRMS (ESI): m/z [M+H]⁺ Calcd for C₁₁H₁₄ClN₃O₃S: 304.0522; Found: 304.0503.

### Step 2 Synthesis of 2-(4-(5-chloropyrimidin-2-yl)piperidin-1-yl)-4-((6-(hydroxymethyl)bicyclo[3.2.0]heptane-6-yl)amino)-6,7-dihydrothiopheno[3,2-d]pyrimidine 5,5-dioxide (Example 4)

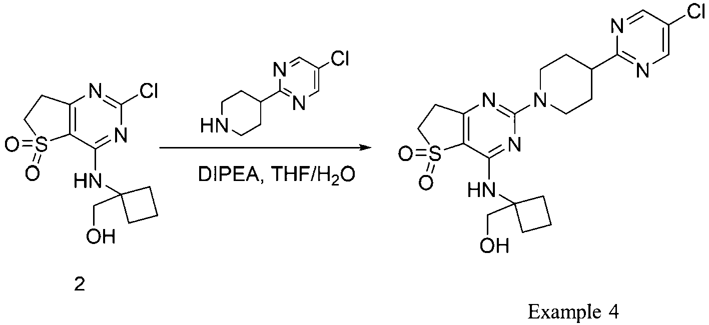

**Intermediate 2** (150 mg, 0.494 mmol), 5-chloro-2-(piperidin-4-yl)pyrimidine (103 mg, 0.519 mmol), tetrahydrofuran (2 mL), water (0.5 mL), and N,N-diisopropylethylamine (160 mg, 1.24 mmol) were added in sequence to a 10 mL Schlenk reaction tube. After the addition was complete, the reaction was carried out overnight at 65°C under nitrogen protection. TLC (*V*_{petroleum ether} : *V*_{ethyl acetate} = 1:2) monitored that the starting materials have completely reacted. The reaction system was filtered, the filter cake was washed with tetrahydrofuran (5 mL), and dried to obtain 167 mg of white solid **Example 4,** with a yield of 72.7%.

¹H NMR (600 MHz, CDCl₃) *δ* (ppm): 8.62 (s, 2H, ArH), 5.61 (s, 1H, NH), 4.48-5.10 (m, 2H, OH and 1H of SCH₂), 3.90 (d, *J=*5.2 Hz, 2H, CH₂OH), 3.85 (t, *J=*5.4 Hz, 1H, H of SCH₂), 3.42 (t, *J*=7.0 Hz, 2H, SCH₂CH₂), 3.22-3.14 (m, 1H, CH), 3.14-3.04 (m, 4H, N(CH₂)₂), 2.35-2.28 (m, 2H, CHCH₂), 2.28-2.20 (m, 2H, CHCH₂), 2.11-2.03 (m, 2H, CH₂CH₂CH₂), 2.02-1.94 (m, 1H, H of CH₂CH₂CH₂), 1.93-1.78 (m, 3H, CH₂CH₂CH₂ and ¹H of CH₂CH₂CH₂).

¹³C NMR (151 MHz, DMSO-*d*₆) *δ* (ppm): 170.71, 168.62, 162.49, 156.20, 155.63, 129.06, 104.65, 64.68, 58.78, 50.53, 44.42, 30.75, 29.94, 28.84, 14.46.

HRMS (ESI): m/z [M+H]⁺ Calcd for C₂₀H₂₅ClN₆O₃S: 465.1475; Found: 465.1457.

### Example 5

### Step 1 Synthesis of methyl 4-((2-methoxy-2-oxoethyl)thio)butyrate (3)

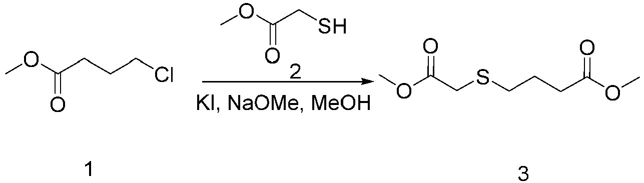

**Compound 2** (13.36 g, 0.126 mol) and methanol (90 mL) were added in sequence to a 500 mL single-necked flask. After stirring at room temperature for 0.5 h, potassium iodide (150 mg, 0.88 mmol) and **compound 1** (20.00 g, 0.146 mol) were added, and the mixture was stirred at 65°C for 20 h. TLC (*V*_{petroleum ether} : *V*_{ethyl acetate} = 10:1) monitored that the starting materials have completely reacted. The reaction solution was cooled to room temperature, filtered, and the filtrate was collected and concentrated, DCM (200 mL) was added to dissolve the same, and the solution was washed with saturated brine (100 mL × 3), dried, filtered and concentrated to obtain 26.00 g of pale yellow oily **intermediate 3,** with a yield of 93.1%.

¹H NMR (600 MHz, CDCl₃) *δ* (ppm): δ 3.72 (s, 3H, OCH₃), 3.66 (s, 3H, OCH₃), 3.21 (s, 2H, COCH₂S), 2.67 (t, *J*=7.2 Hz, 2H, SCH₂), 2.43 (t, *J*=7.2 Hz, 2H, COCH₂), 1.95-1.90 (m, 2H, COCH₂SCH₂CH₂).

### Step 2 Synthesis of methyl 3-oxotetrahydro-2H-thiapyran-2-carboxylate (4)

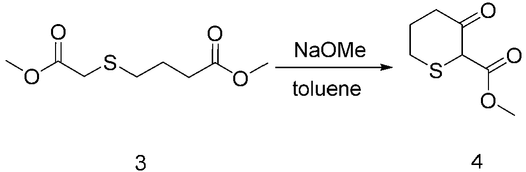

Sodium methoxide (7.50 g, 0.139 mol), toluene (260 mL), and **intermediate 3** (26.00 g, 0.126 mol) were added in sequence to a 500 mL single-necked flask and stirred at 105°C for 3 h under a nitrogen atmosphere. TLC (*V*_{petroleum ether} : *V*_{ethyl acetate} = 10:1) monitored that the starting materials have completely reacted. After the reaction solution was cooled to an ice bath, concentrated hydrochloric acid (35 mL) was added, followed by extraction with EA (150 mL × 3). The organic phases were combined, washed with saturated brine (100 mL × 3), dried, filtered, concentrated, and purified by flash preparative chromatography (120 g × 2, *V*_{petroleum ether} : *V*_{ethyl acetate} = 100: 3) to obtain 15.10 g of pale yellow oily **intermediate 4,** with a yield of 86.7%.

### Step 3 Synthesis of 2-(methylthio)-7,8-dihydro-6H-thiapyrano[3,2-d]pyrimidine-4-ol (6)

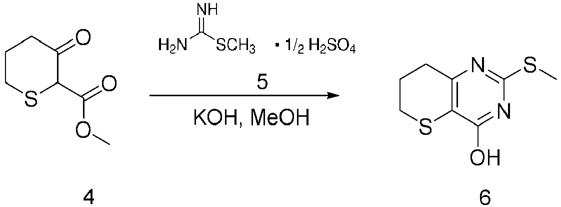

Potassium hydroxide (6.30 g, 0.113 mol), methanol (85 mL), S-methylisothiourea sulfate **(5)** (12.80 g, 0.046 mol) and **intermediate 4** (12.00 g, 0.069 mol) were added in sequence to a 250 mL single-necked flask and stirred at room temperature for 16 h under a nitrogen atmosphere. TLC (*V*_{dichloromethane} : *V*ₘₑₜₕₐₙₒₗ = 15:1) monitored that the starting materials have completely reacted. The reaction solution was poured into ice water (150 mL), acetic acid (20 mL) was added, and the mixture was stirred in an ice bath for 0.5 h. The mixture was then filtered, and the filter cake was collected. The filter cake was then stirred in pure water (150 mL) at room temperature for 1 h and filtered again. The filter cake was washed with water (30 mL × 2), and collected and dried to obtain 11.20 g of white solid **intermediate 6,** with a yield of 76.2%.

¹H NMR (600 MHz, DMSO-*d*₆) *δ* (ppm): 2.91-2.87 (m, 2H, SCH₂), 2.61 (t, *J=*6.3 Hz, 2H, ArCH₂), 2.45 (s, 3H, SCH₃), 2.04-1.99 (m, 2H, SCH₂CH₂).

### Step 4 Synthesis of 7,8-dihydro-6H-thiapyro[3,2-d]pyrimidine-2,4-diol (7)

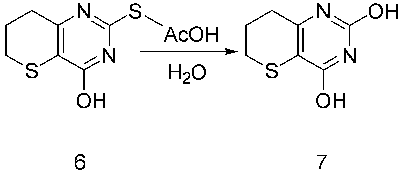

Intermediate 6 (8.00 g, 37.41 mmol), water (30 mL), and acetic acid (60 mL) were added in sequence to a 250 mL single-necked flask and stirred at 110°C for 3 days under a nitrogen atmosphere. TLC (*V*_{dichloromethane} : *V*ₘₑₜₕₐₙₒₗ = 10:1) monitored that the starting materials have completely reacted. The reaction solution was cooled in an ice bath until solid precipitated. The mixture was filtered, and the filter cake was washed with water (30 mL × 2). The filter cake was collected and dried to obtain 5.60 g of white solid intermediate 7, with a yield of 82.3%.

¹H NMR (600 MHz, DMSO-*d*₆) *δ* (ppm): 11.16 (s, 1H, OH), 10.82 (s, 1H, OH), 2.93-2.74 (m, 2H, SCH₂), 2.46-2.35 (m, 2H, ArCH₂), 1.99-1.96 (m, 2H, SCH₂CH₂).

### Step 5 Synthesis of 2,4-dichloro-7,8-dihydro-6H-thiapyro[3,2-d]pyrimidine (8)

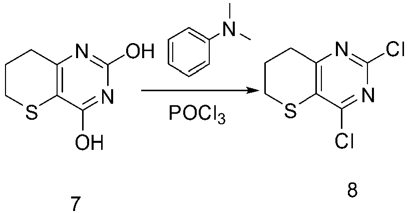

**Intermediate 7** (2.60 g, 14.20 mmol), phosphorus oxychloride (6 mL), and *N,N -* dimethylaniline (172 mg, 1.42 mmol) were added in sequence to a 250 mL single-necked flask and stirred at 110°C for 3 h under a nitrogen atmosphere. TLC (*V*_{dichloromethane} : *V*ₘₑₜₕₐₙₒₗ = 15:1) monitored that the starting materials have completely reacted. The reaction solution was cooled to room temperature, and quenched by adding the reaction solution dropwise to ice water (100 mL). DCM (100 mL × 3) was added for extraction, followed by washing with saturated brine (50 mL × 3), drying, filtration, concentration, and purification by flash preparative chromatography (40 g, *V*_{petroleum ether}: *V*_{ethyl acetate} = 10:1) to obtain 2.50 g of white solid **intermediate 8,** with a yield of 80.6%.

¹H NMR (600 MHz, DMSO-*d*₆) *δ* (ppm): 3.15-3.11 (m, 2H, SCH₂), 2.90 (t, *J*=6.2 Hz, 2H, ArCH₂), 2.12-2.05 (m, 2H, SCH₂CH₂).

### Step 6 Synthesis of (1-((2-chloro-7,8-dihydro-6H-thiapyro[3,2-d]pyrimidin-4-yl)amino)cyclobutyl)methanol (9)

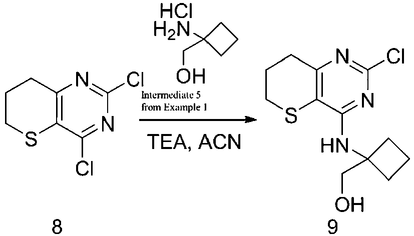

**Intermediate 8** (584 mg, 2.64 mmol), acetonitrile (8 mL), **intermediate 5** (400 mg, 2.91 mmol) of Example 1, and triethylamine (1.34 g, 16.52 mmol) were added in sequence to a 50 mL single-necked flask and stirred at 75°C for 16 h under a nitrogen atmosphere. TLC (*V*_{petroleum ether} : *V*_{ethyl acetate} = 3:1) monitored that the starting materials have completely reacted. The mixture was concentrated under reduced pressure and purified by flash preparative chromatography (24 g, *V*_{petroleum ether} : *V*_{ethyl} acetate = 3:2) to obtain 420 mg of white solid **intermediate 9,** with a yield of 55.9%.

¹H NMR (600 MHz, DMSO-*d*₆) *δ* (ppm): 6.16 (s, 1H, NH), 4.86 (t, *J=5.6* Hz, 1H, OH), 3.65 (d, J=5.7 Hz, 2H, HOCH₂), 3.09-3.04 (m, 2H, SCH₂), 2.67 (t, J=6.3 Hz, 2H, ArCH₂), 2.31-2.26 (m, 2H, SCH₂CH₂), 2.15-2.05 (m, 4H, CH₂CH₂), 1.84-1.68 (m, 2H, CH₂).

### Step 7 Synthesis of 2-chloro-4-((1-(hydroxymethyl)cyclobutyl)amino)-7,8-dihydro-6H-thiapyro[3,2-d]pyrimidine 5,5-dioxide (10)

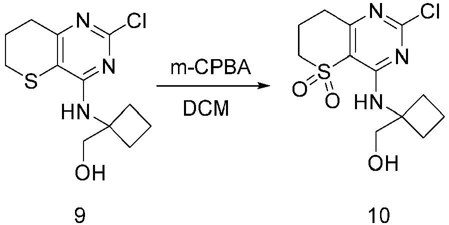

**Intermediate 9** (300 mg, 1.05 mmol), DCM (15 mL), and 85% m-CPBA (532 mg, 2.62 mmol) were added in sequence to a 50 mL single-necked flask and stirred at room temperature for 4 h under a nitrogen atmosphere. TLC (*V*_{petroleum ether} : *V*_{ethyl acetate} = 1:1) monitored that the starting materials have completely reacted. The reaction was quenched by adding sodium thiosulfate aqueous solution (30 mL). After extraction with DCM (40 mL × 3), and the organic phases were combined. The mixture was washed successively with saturated NaHCO₃ solution (20 mL × 2) and saturated brine (30 mL), dried, filtered, concentrated, and purified by flash preparative chromatography (12 g, *V*_{dichloromethane} : *V*ₘₑₜₕₐₙₒₗ = 50: 1) to obtain 320 mg of white solid **intermediate 10,** with a yield of 90.9%.

HRMS (ESI): m/z [M+H]⁺ Calcd for C₁₂H₁₆ClN₃O₃S: 3187.0684; Found: 318.0663.

### Step 8 Synthesis of 2-(4-(5-chloropyrimidin-2-yl)piperidin-1-yl)-4-((1-(hydroxymethyl)cyclobutyl)amino)-7,8-dihydro-6H-thiapyro[3,2-d]pyrimidine 5,5-dioxide (Example 5)

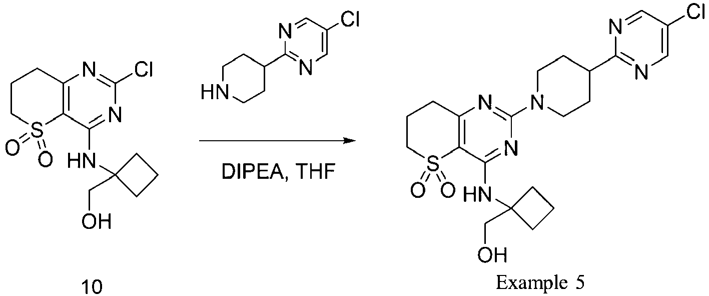

**Compound 10** (315 mg, 0.99 mmol), THF (4 mL), water (0.5 mL), DIPEA (320 mg, 2.48 mmol), and 5-chloro-2-(piperidin-4-yl)pyrimidine (236 mg, 1.19 mmol) were added in sequence to a 25 mL single-necked flask. The reaction was carried out overnight at 65°C under a nitrogen atmosphere. TLC (*V*_{dichloromethane} : *V*_{ethyl} acetate = 1:1) monitored that the starting materials have completely reacted. The reaction was quenched with water (20 mL), extracted with ethyl acetate (50 mL × 3), washed with saturated brine (20 mL × 2), dried, filtered, concentrated, and purified by flash preparative chromatography (12 g, *V*_{dichloromethane} : *V*ₘₑₜₕₐₙₒₗ = 50: 1) to obtain a crude product. The crude product was slurried overnight with a mixed solvent (DCM: MeOH = 10: 1, 15 mL), filtered, and the filter cake was washed with water (10 mL), collected and dried to obtain 410 mg of white solid, **Example 5,** with a yield of 90.5%.

¹H NMR (600 MHz, CDCl₃) *δ* (ppm): 8.61 (s, 2H, ArH), 6.81 (s, 1H, NH), 4.74 (br, 2H, SO₂CH₂), 4.12 (t, *J=*5.2 Hz, 1H, OH), 3.88 (d, *J=*5.1 Hz, 2H, HOCH₂), 3.34-3.26 (m, 2H, ArCH₂), 3.19-3.11 (m, 1H, CH), 3.07-2.96 (m, 2H, NCH₂), 2.76 (t, *J*=6.5 Hz, 2H, NCH₂), 2.43-2.40 (m, 2H, SCH₂CH₂), 2.34-2.31 (m, 2H, CHCH₂), 2.25-2.17 (m, 2H, CHCH₂), 2.09-2.01 (m, 2H, CCH₂), 1.98-1.74 (m, 4H, CCH₂CH₂).

¹³C NMR (151 MHz, CDCl₃) *δ* (ppm): 170.77, 166.01, 159.45, 157.34, 156.20, 129.05, 105.62, 64.28, 58.80, 51.61, 44.47, 43.88, 31.92, 30.74, 30.08, 18.95, 14.60.

### Example 6

### Step 1 Synthesis of 1-(tert-butyl)-4-(4-nitrophenyl)piperidine-1,4-dicarboxylate (2)

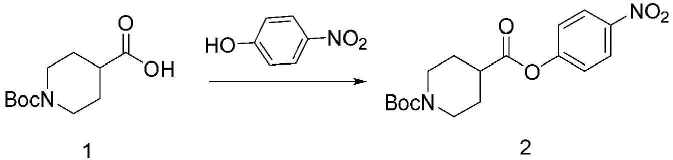

**Compound 1** (2.00 g, 8.72 mmol), p-nitrophenol (1.21 g, 8.72 mmol), and acetonitrile (90 mL) were added in sequence to a 250 mL single-necked flask, and dissolved by stirring. DCC (1.80 g, 8.72 mmol) was added. After the addition was complete, the mixture was allowed to react overnight at room temperature under nitrogen protection. TLC (*V*_{petroleum ether} : *V*_{ethyl acetate} = 3:1) monitored that the starting materials have completely reacted. The reaction system was filtered, and the filtrate was concentrated under reduced pressure and then purified by flash preparative chromatography (40 g, *V*_{petroleum ether} : *V*_{ethyl acetate} = 3:1) to obtain 1.65 g of white solid intermediate 2, with a yield of 54.0%.

¹H NMR (600 MHz, CDCl₃) *δ* (ppm): 8.37-8.22 (m, 2H, ArH), 7.32-7.26 (m, 2H, ArH), 4.12 (s, 2H, NCH₂), 3.03-2.86 (m, 2H, NCH₂), 2.83-2.71 (m, 1H, CH), 2.06 (d, *J=*13.0 Hz, 2H, CH**CH₂),** 1.85-1.71 (m, 2H, CH**CH₂**), 1.48 (s, 9H, (CH₃)₃).

### Step 2 Synthesis of tert-butyl 4-((3,5-dichloropyridin-4-yl)carbamoyl)piperidine-1-carboxylate (3)

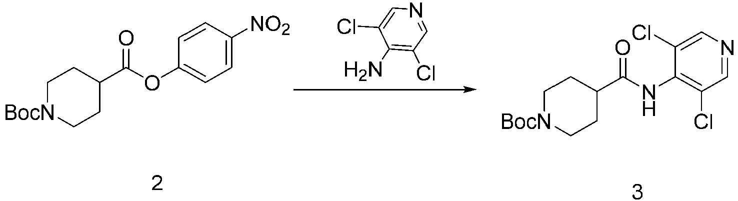

**Intermediate 2** (1.00 g, 2.85 mmol), 4-amino-3,5-dichloropyridine (930 mg, 5.70 mmol), and N,N-dimethylformamide (15 mL) were added in sequence to a 50 mL single-necked flask. Then, sodium hydride (60%, dispersed in mineral oil, 205 mg, 5.13 mmol) was added to the reaction system in two batches at 0°C. After the addition was complete, the reaction was carried out at room temperature for 4 h under nitrogen protection. TLC (*V*_{petroleum ether} : *V*_{ethyl acetate} = 3:1) monitored that the starting materials have completely reacted. Water (30 mL) was added to the reaction solution to quench the reaction, and the mixture was extracted with ethyl acetate (20 mL × 3). The organic phases were combined, washed with saturated brine (30 mL), dried over anhydrous sodium sulfate, filtered, concentrated and purified by flash preparative chromatography (20 g, *V*_{petroleum ether} : *V*_{ethyl acetate} = 1:1) to obtain 950 mg of white solid intermediate 3, with a yield of 89.0%.

¹H NMR (600 MHz, CDCl₃) *δ* (ppm): 8.53 (d, *J=*1.4 Hz, 2H, ArH), 4.17 (s, 2H, NCH₂), 2.93 -2.79 (m, 2H, NCH₂), 2.62-2.51 (m, 1H, CH), 1.98 (d, *J*=13.2 Hz, 2H, CHCH₂), 1.87-1.72 (m, 2H, CHCH₂), 1.47 (s, 9H, (CH₃)₃).

HRMS (ESI): m/z [M+H]⁺ Calcd for C₁₆H₂₁Cl₂N₃O₃: 374.1038; Found: 373.0960.

### Step 3 Synthesis of N- (3,5-dichloropyridin-4-yl)piperidine-4-carboxamide hydrochloride (4)

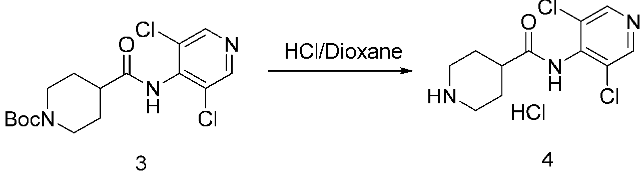

**Intermediate 3** (950 mg, 2.54 mmol) and dichloromethane (3 mL) were added in sequence to a 50 mL Schlenk reaction tube. After dissolved by stirring, 1,4-dioxane solution of hydrogen chloride (4.0 M, 3 mL) was added dropwise to the reaction system at 0°C. The reaction was carried out under nitrogen protection at room temperature for 2 h. TLC (*V*_{petroleum ether} : *V*_{ethyl acetate} = 2:1) monitored that the starting materials have completely reacted. The reaction system was filtered, and the filter cake was washed with ethyl acetate (5 mL), and dried to obtain 766 mg of white solid **intermediate 4,** with a yield of 97.0%.

¹H NMR (400 MHz, DMSO-*d*₆) *δ* (ppm): 10.48 (s, 1H, HCl), 9.24 (s, 1H, NH), 8.85 (s, 1H, NH), 8.69 (s, 2H, ArH), 3.29 (d, *J=*13.5 Hz, 2H, NCH₂), 3.00-2.89 (m, 2H, NCH₂), 2.87-2.77 (m, 1H, CH), 2.09-1.97 (m, 2H, CH**CH₂**), 1.95-1.79 (m, 2H, CH**CH₂**).

### Step 4: Synthesis of N- (3,5-dichloropyridin-4-yl)-1-(4-((1-(hydroxymethyl)cyclobutyl)amino)-5-oxo-6,7-dihydrothiopheno[3,2-d]pyrimidin-2-yl)piperidine-4-carboxamide (Example 6)

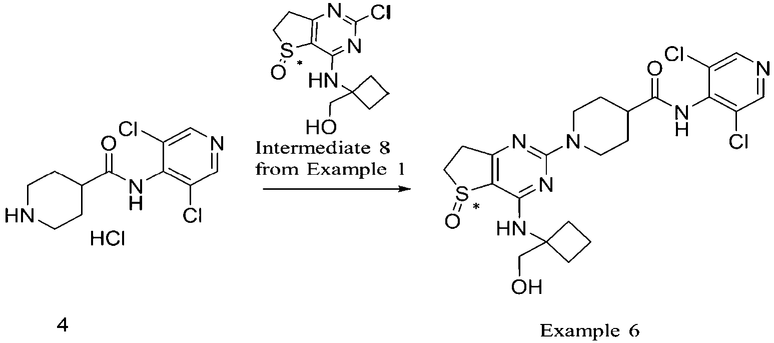

**Intermediate 4** (240 mg, 0.77 mmol), **Intermediate 8 from Example** 1 (202 mg, 0.70 mmol), tetrahydrofuran (3 mL), water (0.75 mL), and N,N-diisopropylethylamine (272 mg, 2.10 mmol) were added in sequence to 10 mL of Schlenk. After the addition was complete, the mixture was reacted overnight at 65°C under nitrogen protection. TLC (*V*_{dichloromethane} : *V*ₘₑₜₕₐₙₒₗ = 10:1) monitored that the starting materials have completely reacted. The reaction system was filtered, and the filter cake was washed with tetrahydrofuran (10 mL), and dried to obtain 162 mg of white solid **Example 6,** with a yield of 44.0%.

¹H NMR (400 MHz, DMSO-*d*₆) *δ* (ppm): 10.22 (s, 1H, NH), 8.67 (s, 2H, ArH), 7.36 (s, 1H, NH), 4.84 (t, *J*=5.7 Hz, 1H, OH), 4.66 (d, *J=*13.0 Hz, 2H, **CH₂**OH), 3.79-3.68 (m, 2H, SCH₂), 3.47-3.37 (m, 1H, H of SCH₂**CH₂**), 3.26-3.16 (m, 1H, H of SCH₂**CH₂**), 3.08-2.98 (m, 2H, NCH₂), 2.97-2.77 (m, 3H, NCH₂ and CH), 2.41-2.25 (m, 2H, CH**CH₂**), 2.23-2.11 (m, 2H, CH**CH₂**), 1.89 (d, *J*=12.9 Hz, 2H, **CH₂**CH₂CH₂), 1.83-1.69 (m, 2H, CH₂CH₂**CH₂**), 1.64-1.49 (m, 2H, CH₂**CH₂**CH₂).

HRMS (ESI): m/z [M+H]⁺ Calcd for C₂₂H₂₆Cl₂N₃O₃S: 525.124; Found: 525.121.

### Example 7

### Step 1: Synthesis of tert-butyl 4-(2-hydroxyl-5-methylbenzamido)piperidine-1-carboxylate (3)

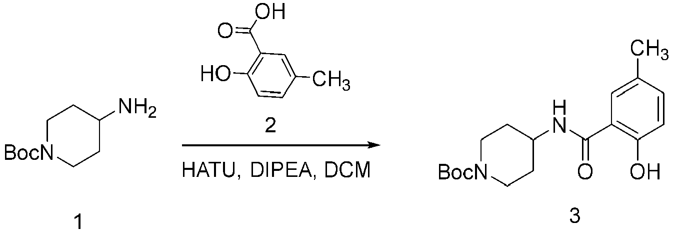

**Compound** 1 (1.00 g, 6.57 mmol), **compound 2** (1.58 g, 7.88 mmol), HATU (3.00 g, 7.88 mmol), DIPEA (1.70 g, 13.17 mmol) and dichloromethane (20 mL) were added in sequence to a 100 mL single-necked flask. The reaction was carried out at room temperature for 1 h under nitrogen protection. After the reaction was completed monitored by TLC (*V*_{petroleum ether} : *V*_{ethyl acetate} = 2:1), water (20 mL) was added to the reaction solution, and the mixture was extracted with dichloromethane (50 mL × 3), and the organic phase was collected. The organic phase was washed with saturated brine (50 mL), dried over anhydrous sodium sulfate, filtered, concentrated, and purified by flash preparative chromatography (40 g, *V*_{petroleum ether} : *V*_{ethyl acetate} = 3:1) to obtain 350 mg of white solid **intermediate 3,** with a yield of 15.9%.

¹H NMR (400 MHz, DMSO-*d*₆) *δ* (ppm): 12.34 (s, 1H, OH), 8.53 (d, *J*=7.8 Hz, 1H, ArH), 7.68 (d, *J*=2.1 Hz, 1H, HN), 7.21 (dd, *J*₁=8.4 Hz, *J*₂=2.1 Hz, 1H, ArH), 6.78 (d, *J*=8.3 Hz, 1H, ArH), 4.07-3.91 (m, 3H, CH₂, CH), 2.94-2.74 (m, 2H, CH₂), 2.24 (s, 3H, ArCH₃), 1.83-1.76 (m, 2H, CH₂), 1.41 (s, 9H, C(CH₃)₃).

### Step 2 Synthesis of 2-hydroxyl-5-methyl- N- (piperidin-4-yl)benzamide trifluoroacetate (4)

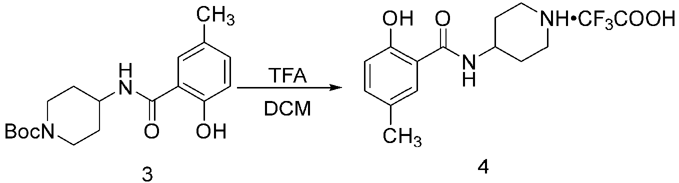

**Intermediate 3** (0.2 g, 0.6 mmol) and dichloromethane (3 mL) were added in sequence to a 25 mL single-necked flask. The temperature was lowered to 0°C, and TFA (3 mL) was slowly added dropwise. After the addition was complete, the mixture was allowed to react at room temperature for 1 h. After TLC (*V*_{petroleum ether} : *V*_{ethyl acetate} = 2:1) monitored that the reaction was complete, the mixture was concentrated to obtain 206 mg of yellow oily **intermediate 4,** with a yield of 99%, which was directly used in the next step.

### Step 3 Synthesis of 2-hydroxyl- N- (1-(4-((1-(hydroxymethyl)cyclobutyl)amino)-5-oxo-6,7-dihydrothiapheno[3,2-d]pyrimidin-2-yl)piperidin-4-yl)-5-methylbenzamide (Example 7)

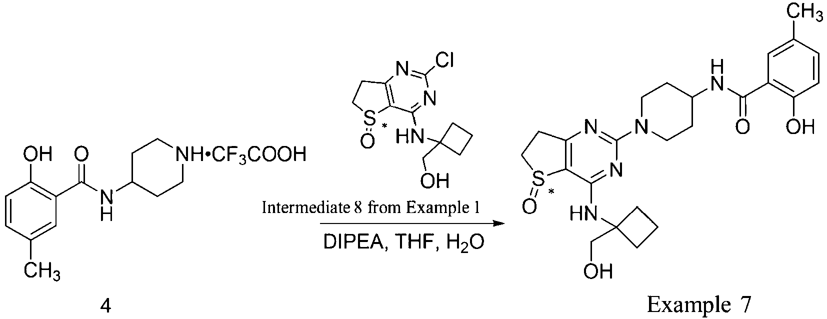

**Intermediate 4** (0.10 g, 0.30 mmol), **Intermediate 8 from Example** 1 (0.086 g, 0.3 mmol), DIPEA (0.116 g, 0.90 mmol), THF (4 mL) and H₂O (1 mL) were added in sequence to a 50 mL Schlenk reaction tube. The reaction was carried out overnight at 65°C under nitrogen protection. After TLC (*V*_{dichloromethane} : *V*ₘₑₜₕₐₙₒₗ = 10:1) monitored that the reaction was complete, the mixture was concentrated and purified by preparative plate (*V*_{dichloromethane} : *V*ₘₑₜₕₐₙₒₗ = 10:1) to obtain 105 mg of white solid **Example 7,** with a yield of 71.4%.

¹H NMR (400 MHz, DMSO-*d*₆) *δ* (ppm): 12.40 (s, 1H, ArOH), 8.51 (d, *J*=7.9 Hz, 1H, NH), 7.67 (d, *J*=2.4 Hz, 1H, NH), 7.40 (s, 1H, ArH), 7.20 (dd, *J*₁=8.4 Hz, *J*₂=2.1 Hz, 1H, ArH), 6.78 (d, *J*=8.3 Hz, 1H, ArH), 4.85 (t, *J=5.6* Hz, 1H, OH), 4.69-4.56 (m, 2H, CH₂), 4.24-4.12 (m, 1H, CH), 3.78-3.68 (m, 2H, CH₂), 3.51-3.39 (m, 1H, H of CH₂), 3.26-3.16 (m, 1H, H of CH₂), 2.98-2.84 (m, 2H, CH₂), 2.41-2.26 (m, 2H, CH₂), 2.22 (s, 3H, CH₃), 2.20-2.12 (m, 2H, CH₂), 1.87-1.71 (m, 4H, CH₂CH₂), 1.56-1.43 (m, 2H, CH₂).

¹³C NMR (101 MHz, MeOD) *δ* (ppm): 175.89, 168.94, 162.44, 158.13, 157.56, 134.11, 127.97, 127.52, 116.86, 115.25, 107.24, 64.21, 58.72, 43.14, 32.09, 31.24, 31.20, 29.85, 29.72, 19.14, 14.45.

HRMS (ESI): m/z [M+H]⁺ Calcd for C₂₄H₃₁N₅O₄S: 486.2175; Found: 486.2161.

### Example 8

### Step 1 Synthesis of tert-butyl 6-(5-chloropyrimidin-2-yl)-2,6-diazaspiro[3.3]heptane-2-carboxylate (2)

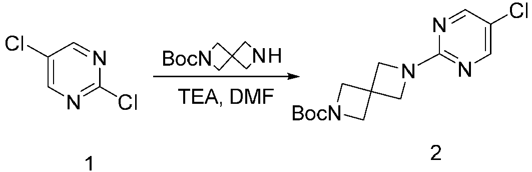

**Compound 1** (700 mg, 3.53 mmol), tert-butyl 2,6-diazaspiro[3.3]heptan-2-ylcarboxylate (848 mg, 3.53 mmol), tetrahydrofuran (20 mL) and triethylamine (429 mg, 4.24 mmol) were added in sequence to a 50 mL single-necked flask. After the addition was complete, the mixture was allowed to react overnight at room temperature. TLC (*V*_{petroleum ether} : *V*_{ethyl acetate} = 4:1) monitored that the starting materials have completely reacted. The reaction solution was directly concentrated and purified by flash preparative chromatography (20 g, *V*_{petroleum ether} : *V*_{ethyl acetate} = 4:1) to obtain 825 mg of white solid **intermediate 2,** with a yield of 75.2%.

¹H NMR (400 MHz, DMSO-*d*₆) *δ* (ppm): 8.40 (s, 2H, ArH), 4.16 (s, 4H, N(CH₂)₂), 4.02 (s, 4H, N(CH₂)₂), 1.37 (s, 9H, C(CH₃)₃).

### Step 2 Synthesis of 2-(5-chloropyrimidin-2-yl)-2,6-diazaspiro[3.3]heptane (3)

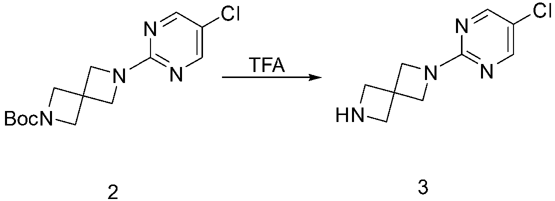

**Intermediate 2** (500 mg, 1.61 mmol), tetrahydrofuran (8 mL), and trifluoroacetic acid (2 mL) were added in sequence to a 50 mL single-necked flask. After the addition was complete, the mixture was allowed to react at room temperature for 2 h. TLC (*V*_{dichloromethane} : *V*ₘₑₜₕₐₙₒₗ = 10:1) monitored that the starting materials have completely reacted. The reaction solution was concentrated, and the residue was added to a saturated sodium bicarbonate aqueous solution (20 mL). After the addition was complete, the mixture was extracted with dichloromethane (20 mL × 3). The organic phases were combined, washed with saturated brine (100 mL), dried over anhydrous sodium sulfate, filtered, and concentrated to obtain 320 mg of white solid **intermediate 3,** which was used directly in the next step without purification.

### Step 3 Synthesis of 2-(6-(5-chloropyrimidin-2-yl)-2,6-diazaspiro[3.3]heptan-2-yl)-4-((1-(hydroxymethyl)cyclobutyl)amino)-6,7-dihydrothiopheno[3,2-d|pyrimidine 5-oxide (Example 8)

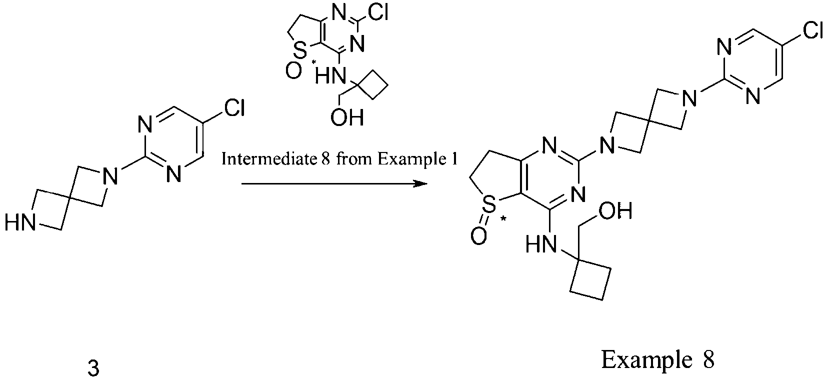

**Intermediate** 8 (228 mg, 0.79 mmol) from **Example 1, intermediate 3** (200 mg, 0.95 mmol), N,N-diisopropylethylamine (306 mg, 2.37 mmol), and tetrahydrofuran (10 mL) were added in sequence to a 50 mL single -necked flask. The mixture was then reacted overnight at 80°C under nitrogen protection. TLC (*V*_{dichloromethane} : *V*ₘₑₜₕₐₙₒₗ = 10:1) monitored that the starting materials have completely reacted. The reaction solution was directly concentrated and purified by flash preparative chromatography (20 g, *V*_{dichloromethane} : *V*ₘₑₜₕₐₙₒₗ = 10:1) to obtain 210 mg of white solid **(Example 8),** with a yield of 44.1%.

¹H NMR (400 MHz, DMSO-*d*₆) *δ* (ppm): 8.41 (s, 2H, ArH), 7.36 (s, 1H, NH), 4.85 (t, *J=5.6* Hz, 1H, OH), 4.23 (s, 4H, N(CH₂)₂), 4.19 (s, 4H, N(CH₂)₂), 3.70 (d, J=5.7 Hz, 2H, SCH₂), 3.45-3.34 (m, 1H, H of CH₂OH), 3.22-3.14 (m, 1H, H of CH₂OH), 2.97-2.82 (m, 2H, SCH₂CH₂), 2.42-2.28 (m, 2H, H of CH₂CH₂CH₂), 2.17-2.06 (m, 2H, H of CH₂CH₂CH₂), 1.85-1.69 (m, 2H, H of CH₂CH₂CH₂).

¹³C NMR (100 MHz, DMSO-*d*₆) *δ* (ppm): 175.21, 163.44, 161.08, 158.30, 156.47, 118.79, 110.66, 64.63, 60.60, 60.12, 59.10, 48.97, 33.68, 32.77, 30.32, 30.15, 14.77.

HRMS (ESI): m/z [M+H]⁺ Calcd for C₂₀H₂₄ClN₇O₂S: 462.1479; Found: 462.1474.

### Example 9

### Step 1 Synthesis of 2-chloro- N- (3,5-dichloropyridin-4-yl)-7,8-dihydro- 6H - thiapyrano[3,2-d]pyrimidine-4-amine (2)

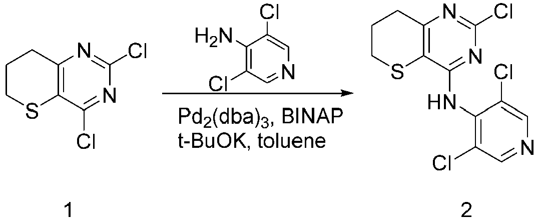

**Compound** 1 (500 mg, 2.27 mmol), 4-amino-3,5-dichloropyridine (740 mg, 4.54 mmol), Pd₂(dba)₃ (211 mg, 0.23 mmol), BINAP (143 mg, 0.23 mmol), potassium tert-butoxide (509 mg, 4.54 mmol), and anhydrous toluene (20 mL) were added in sequence to a 100 mL Schlenk reaction tube. The reaction was carried out at 100°C for 15 h under nitrogen protection. TLC (*V*_{petroleum ether} : *V*_{ethyl acetate} = 3: 1) monitored that the reaction was completed, and the reaction solution was cooled to room temperature. The mixture was filtered, concentrated, and purified by flash preparative chromatography (20 g, *V*_{petroleum ether} : *V*_{ethyl acetate} = 4:1) to obtain 130 mg of yellow solid **intermediate 2,** with a yield of 16.5%.

¹H NMR (400MHz, CDCl₃) *δ* (ppm): 8.54 (s, 2H, ArH), 6.57 (s, 1H, NH), 3.19-3.15 (m, 2H, SCH₂), 2.97-2.92 (m, 2H, CH₂CH₂C**H₂**), 2.35-2.29 (m, 2H, CH₂C**H₂**CH₂).

HRMS (ESI): m/z [M+H]⁺ Calcd for C₁₂H₉Cl₃N₄S: 346.9692; Found: 346.9670.

### Step 2 Synthesis of 2-chloro-4-((3,5-dichloropyridin-4-yl)amino)-7,8-dihydro- 6H -thiapyrano[3,2-d]pyrimidine 5,5-dioxide (3)

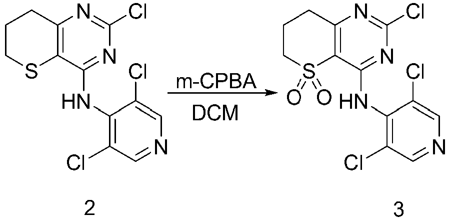

**Intermediate 2** (600 mg, 1.73 mmol), m-CPBA (85%, 879 mg, 4.33 mmol), and dichloromethane (20 mL) were added in sequence to a 100 mL single-necked flask. The mixture was stirred at room temperature for 5 h under N₂ protection. TLC (*V*_{petroleum ether} : *V*_{ethyl acetate} = 3 : 1) monitored that the reaction was completed. 100 mL of saturated sodium thiosulfate aqueous solution was added, and after extracting with dichloromethane (100 mL × 3), the organic phase was washed with saturated sodium bicarbonate aqueous solution (100 mL × 3). The organic phase was combined, dried over anhydrous sodium sulfate, filtered, concentrated, and purified by flash preparative chromatography (12 g, *V*_{petroleum ether} : *V*_{ethyl acetate} = 9: 1~1: 1) to obtain 110 mg of white solid **intermediate 3,** with a yield of 16.7%.

¹H NMR (400MHz, CDCl₃) *δ* (ppm): 8.65 (s, 1H, NH), 8.59 (s, 2H, ArH), 3.54-3.42 (m, 2H, SO₂CH₂), 3.13-3.04 (m, 2H, CH₂CH₂CH₂), 2.66-2.53 (m, 2H, CH₂CH₂CH₂).

HRMS (ESI): m/z [MH]⁻ Calcd for C₁₂H₉Cl₃N₄O₂S: 376.9434; Found: 376.9456.

### Step 3 Synthesis of 2-(4-(5-chloropyrimidin-2-yl)piperidin-1-yl)-4-((3,5-dichloropyridin-4-yl)amino)-7,8-dihydro- 6H -thiapyrano[3,2-d]pyrimidine 5,5-dioxide (Example 9)

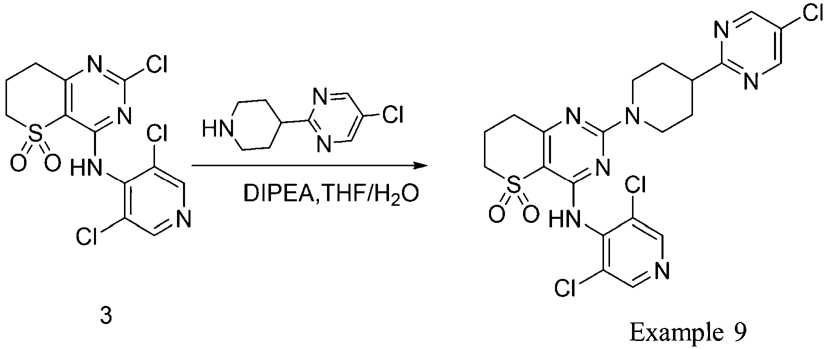

**Intermediate 3** (110 mg, 0.29 mmol), 5-chloro-2-(piperidin-4-yl)pyrimidine (57 mg, 0.29 mmol), tetrahydrofuran (8 mL), water (2 mL), and DIPEA (112 mg, 0.87 mmol) were added in sequence to a 100 mL single-necked flask. The reaction was carried out at 65°C for 3 h under nitrogen protection. TLC (*V*_{dichloromethane} : *V*ₘₑₜₕₐₙₒₗ = 50: 1) mointored that the reaction was completed. The mixture was concentrated and purified by flash preparative chromatography (12 g, *V*_{petroleum ether} : *V*_{ethyl acetate} = 1: 1) to obtain 93 mg of white solid **Example 9,** with a yield of 59.2%.

¹H NMR (400MHz, CDCl₃) *δ* (ppm): 8.61 (s, 2H, ArH), 8.58 (s, 1H, NH), 8.51 (s, 2H, ArH), 4.87 (br, 1H, H of SO₂CH₂), 4.39 (br, 1H, H of SO₂CH₂), 3.45-3.34 (m, 2H, NCH₂), 3.16-3.07 (m, 1H, CH), 3.05-2.83 (m, 4H, NCH₂, CH₂CH₂CH₂), 2.54-2.43 (m, 2H, CH₂CH₂CH₂), 2.03-1.87 (m, 2H, CHCH₂), 1.82-1.69 (m, 2H, CHCH₂).

¹³C NMR (101 MHz, CDCl₃) *δ* (ppm): 170.43, 166.25, 159.21, 155.62, 155.54, 148.02, 141.17, 129.14, 128.73, 106.06, 51.77, 44.64, 43.67, 31.78, 30.66, 18.72.

HRMS (ESI): m/z [M+Na]⁺ Calcd for C₂₁H₂₀Cl₃N₇O₂S: 562.0363; Found: 562.0380.

### Example 11

### Step 1 Synthesis of methyl 2-(4-((2-chloro-7,8-dihydro- 6H -thiapyrano[3,2-d]pyrimidin-4-yl)amino)-2-fluorophenyl)acetate (2)

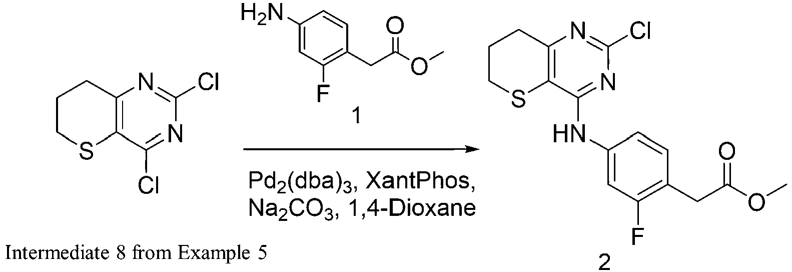

**Intermediate 8** (150 mg, 0.66 mmol) from **Example 5, compound 1** (0.10 mg, 0.55 mmol), Xantphos (48 mg, 0.083 mmol), Na₂CO₃ (120 mg, 1.10 mmol), and 1,4-dioxane (2 mL) were added in sequence to a 100 mL single-necked flask. After completely purging with nitrogen, Pd₂(dba)₃ (25 mg, 0.028 mmol) was added. After the addition is complete, purging with nitrogen was conducted for three more times. The reaction was carried out overnight at 70°C. TLC (*V*_{petroleum ether} : *V*_{ethyl acetate} = 2:1) monitored that the reaction was completed, ethyl acetate (10 mL) was added to the reaction solution, filtered, concentrated and purified by flash preparative chromatography (40 g, *V*_{petroleum ether} : *V*_{ethyl acetate} = 1:1) to obtain 200 mg of white solid **intermediate 2,** with a yield of 41.3%.

¹H NMR (400 MHz, DMSO-*d*₆) *δ* (ppm): 8.71 (s, 1H, NH), 7.46 (dd, *J*₁=12.2 Hz, *J*₂=2.1 Hz, 1H, ArH), 7.37-7.33 (m, 1H, ArH), 7.29 (t, *J*=8.4 Hz, 1H, ArH), 3.70 (s, 2H, ArCH₂), 3.63 (s, 3H, OCH₃), 3.17-3.12 (m, 2H, CH₂), 2.79 (t, *J*=6.4 Hz, 2H, CH₂), 2.17-2.10 (m, 2H, CH₂).

### Step 2 Synthesis of methyl 2-(4-(((2-chloro-5,5-dioxo-7,8-dihydro- 6H - thiapyrano[3,2-d]pyrimidin-4-yl)amino)-2-fluorophenyl)acetate (3)

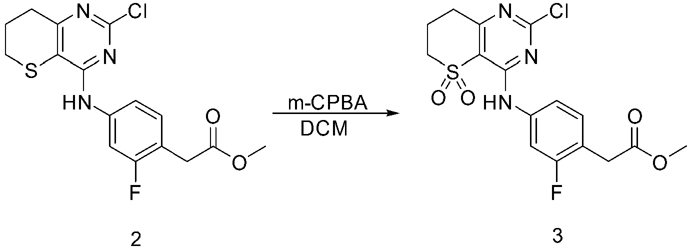

**Intermediate** 2 (280 mg, 0.76 mmol) and DCM (10 mL) were added in sequence to a 25 mL single-necked flask. At room temperature, m-CPBA (85%, 390 mg, 1.90 mmol) was added in two batches. After the addition was complete, the reaction was carried out at room temperature for 2.5 h under nitrogen protection. After TLC (*V*_{petroleum ether} : *V*_{ethyl acetate} = 1:1) monitored that the reaction was completed, the reaction solution was quenched with saturated sodium thiosulfate solution (10 mL), extracted with DCM (30 mL × 3), and the organic phase was collected. The organic phase was washed successively with saturated sodium bicarbonate solution (30 mL × 3) and saturated saline solution (30 mL × 3), dried over anhydrous sodium sulfate, filtered, concentrated and purified by flash preparative chromatography (20 g, *V*_{petroleum ether} : *V*_{ethyl} acetate = 1:1) to obtain 202 mg of white solid intermediate 3, with a yield of 72.4%.

HRMS (ESI): m/z [M+H]⁺ Calcd for C₁₆H₁₅ClFN₃O₄S: 400.0530; Found: 400.0534.

### Step 3 Synthesis of methyl 2-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2-fluorophenyl)acetate (4)

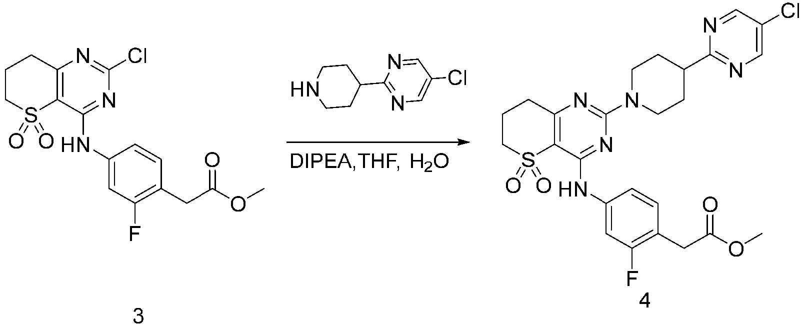

**Intermediate** 3 (210 mg, 0.53 mmol), 5-chloro-2-(piperidin-4-yl)pyrimidine (114 mg, 0.58 mmol), DIPEA (205 g, 1.59 mmol), THF (4 mL) and H₂O (1 mL) were added in sequence to a 25 mL single-necked flask. The reaction was carried out overnight at 65°C under nitrogen protection. TLC (*V*_{dichloromethane} : *V*ₘₑₜₕₐₙₒₗ = 30:1) monitored that the reaction was completed. The mixture was concentrated and slurried with tetrahydrofuran (1 mL) to obtain 130 mg of white solid intermediate 4, with a yield of 87.6%.

¹H NMR (400 MHz, DMSO-*d*₆) *δ* (ppm): 8.87 (s, 2H, ArH), 8.68 (s, 1H, NH), 7.61 (dd, *J*₁=12.3 Hz, *J*₂=2.0 Hz, 1H, ArH), 7.31 (t, J=8.4 Hz, 1H, ArH), 7.26-7.22 (m, 1H, ArH), 4.80-4.51 (m, 2H, CH₂), 3.70 (s, 2H, ArCH₂), 3.62 (s, 3H, OCH₃), 3.59-3.55 (m, 2H, CH₂), 3.23-3.12 (m, 3H, CH₂CH₂), 2.82 (t, J=6.4 Hz, 2H, CH₂), 2.31-2.26 (m, 2H, CH₂), 2.05-2.00 (m, 2H, CH₂), 1.75-1.64 (m, 2H, CH₂).

HRMS (ESI): m/z [M+H]⁺ Calcd for C₂₅H₂₆ClFN₆O₄S: 561.1487; Found: 561.1479.

### Step 4 Synthesis of 2-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2-fluorophenyl)acetic acid (Example 11)

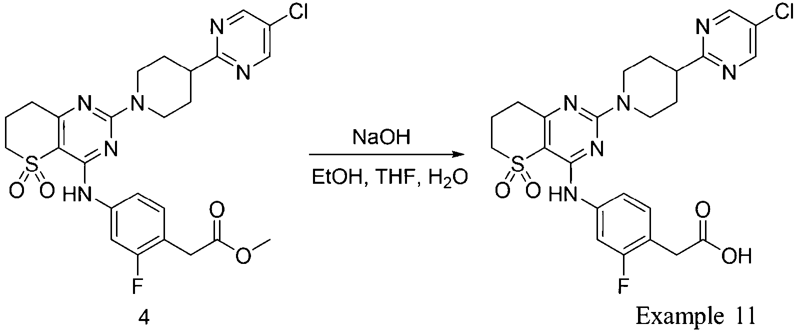

**Intermediate** 4 (150 mg, 0.28 mmol), ethanol (1 mL), tetrahydrofuran (1 mL), and water (0.5 mL) were added in sequence to a 25 mL single-necked flask. The reaction was carried out at room temperature for 2 h under N₂ protection. TLC (*V*_{dichloromethane} : *V*ₘₑₜₕₐₙₒₗ = 30 : 1) monitored that the reaction was completed. The pH of the solution was adjusted to 7 with 1M hydrochloric acid and then concentrated. H₂O (2 mL) was added, and the pH of the solution was adjusted to 5 with 1M hydrochloric acid, filtered, and the filter cake was dried to obtain 132 mg of white solid Example 11, with a yield of 90.7%.

¹H NMR(400 MHz, DMSO-*d*₆) *δ* (ppm): 12.47 (s, 1H, COOH), 8.87 (s, 2H, ArH), 8.67 (s, 1H, NH), 7.59 (dd, *J*₁=12.2 Hz, *J*₂=2.1 Hz, 1H, ArH), 7.30 (t, J=8.4 Hz, 1H, ArH), 7.24-7.20 (m, 1H, ArH), 4.76-4.48 (m, 2H, CH₂), 3.59-3.54 (m, 4H, CH₂, CH₂), 3.24-3.12 (m, 3H, CH₂CH₂), 2.82 (t, J=6.4 Hz, 2H, CH₂), 2.32-2.25 (m, 2H, CH₂), 2.06-2.00 (m, 2H, CH₂), 1.78-1.63 (m, 2H, CH₂).

¹³C NMR (101 MHz, DMSO-*d*₆) *δ* (ppm): 172.33, 170.55, 167.29, 160.75 (d, *J_{C- F}*=243.4 Hz), 156.23, 156.14, 138.91 (d, *J_{C-F}*=11.1 Hz), 132.31 (d, *J_{C-F}*=6.1 Hz), 129.01, 117.78 (d, *J_{C-F}*=17.2 Hz), 117.17 (d, *J_{C-F}*=3.0 Hz), 108.48 (d, *J_{C-F}*=27.3 Hz), 106.03, 51.36, 44.11, 43.91, 34.30, 31.92, 30.63, 18.77.

HRMS (ESI): m/z [M+H]⁺ Calcd for C₂₄H₂₄ClFN₆O₄S: 547.1331; Found: 547.1300.

### Example 12

### Step 1 Synthesis of 4-azido-3,5-dichloropyridine (2)

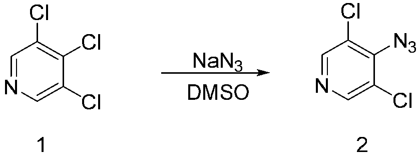

**Compound 1** (500 mg, 2.7 mmol), DMSO (5 mL), and sodium azide (356 mg, 5.4 mmol) were added in sequence to a 25 mL single-necked flask under nitrogen protection, and the reaction was carried out at 30°C for 36 h. TLC (*V*_{petroleum ether} : *V*_{ethyl acetate} = 5:1) monitored that the starting materials have completely reacted. The reaction was cooled and quenched by adding water (15 mL), and the solid precipitated. After filtration and drying, 450 mg of blue-white solid **intermediate 2** was obtained, with a yield of 86.9%.

¹ H NMR (400 MHz, CDCl₃) *δ* (ppm): 8.40 (s, 2H, ArH).

### Step 2 Synthesis of 4-((1-(hydroxymethyl)cyclobutyl)amino)-2-((trimethylsilyl)ethynyl)-7,8-dihydro-6H - thiapyrano[3,2-d]pyrimidine 5,5-dioxide (3)

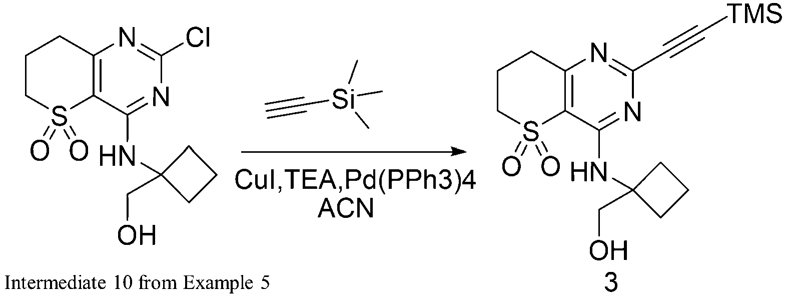

**Intermediate 10 from Example 5** (600 mg, 1.89 mmol), acetonitrile (30 mL), trimethylethynylsilane (1.11 g, 11.3 mmol), cuprous iodide (54 mg, 0.28 mmol), triethylamine (0.8 mL, 5.66 mmol), and tetrakis(triphenylphosphine)palladium (327 mg, 0.28 mmol) were added in sequence to a 100 mL single-necked flask. The mixture was then reacted at 80°C for 2 h under nitrogen protection. TLC (*V*_{petroleum ether} : *V*_{ethyl acetate} = 1:1) monitored that the starting materials have completely reacted. The mixture was cooled, directly concentrated and purified by column chromatography (*V*_{petroleum ether} : *V*_{ethyl acetate} = 1:1) to obtain 520 mg of yellow solid **intermediate 3,** with a yield of 72.6%.

¹H NMR (400 MHz, DMSO-*d*₆) *δ* (ppm): 6.93 (s, 1H, NH), 5.02 (t, *J=5.6* Hz, 1H, OH), 3.66 (d, *J=5.6* Hz, 2H, CH₂OH), 3.63-3.56 (m, 2H, SO₂CH₂), 2.85 (t, J=6.4 Hz, 2H, SO₂CH₂CH₂), 2.33-2.23 (m, 4H, SO₂CH₂CH₂CH₂, cyclobutane-CH₂), 2.17-2.09 (m, 2H, cyclobutane-CH₂), 1.92-1.82 (m, 1H, cyclobutane-CH₂), 1.80-1.72 (m, 1H, cyclobutane-CH₂), 0.24 (s, 9H, (CH₃)₃).

### Step 3 Synthesis of 2-ethynyl-4-((1-(hydroxymethyl)cyclobutyl)amino)-7,8-dihydro- 6H-thiapyrano[3,2-d]pyrimidine 5,5-dioxide (4)

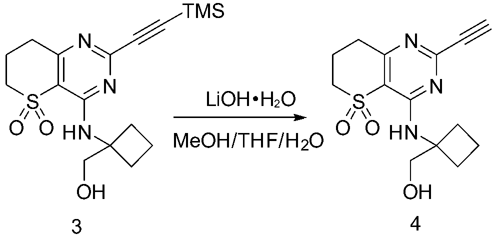

**Intermediate** 3 (520 mg, 1.37 mL), methanol (15 mL), and tetrahydrofuran (15 mL) were added to a 100 mL single-necked flask in sequence. An aqueous solution (15mL) of lithium hydroxide monohydrate (58 mg, 1.37) was add dropwise under an ice-water bath. After the addition was complete, reaction was carried out at room temperature for 30 min. TLC (*V*_{petroleum ether} : *V*_{ethyl acetate} = 1:2) monitored that the starting materials have completely reacted. The mixture was diluted with water (30 mL), extracted with ethyl acetate (30 mL × 3), and the organic phases were combined, dried over anhydrous sodium sulfate, filtered, concentrated, and purified by column chromatography (*V*_{petroleum ether} : *V*_{ethyl acetate} = 1:2) to obtain 390 mg of yellow solid **intermediate 4,** with a yield of 92.6%.

¹H NMR (400 MHz, DMSO-*d*₆) *δ* (ppm): 6.94 (s, 1H, NH), 5.01 (t, *J=5.6* Hz, 1H, OH), 4.31 (s, 1H, CH), 3.67 (d, *J=5.2* Hz, 2H, CH₂OH), 3.64-3.56 (m, 2H, SO₂CH₂), 2.85 (t, J=6.4 Hz, 2H, SO₂CH₂CH₂), 2.32-2.24(m, 4H, SO₂CH₂CH₂CH₂, cyclobutane-CH₂), 2.17-2.09 (m, 2H, cyclobutane-CH₂), 1.93-1.82 (m, 1H, cyclobutane-CH₂), 1.80-1.73 (m, 1H, cyclobutane-CH₂).

### Step 4 Synthesis of 2-(1-(3,5-dichloropyridin-4-yl)-1H - 1,2,3-triazol-4-yl)-4-((1-(hydroxymethyl)cyclobutyl)amino)-7,8-dihydro-6H - thiapyrano[3,2-d]pyrimidine 5,5-dioxide (Example 12)

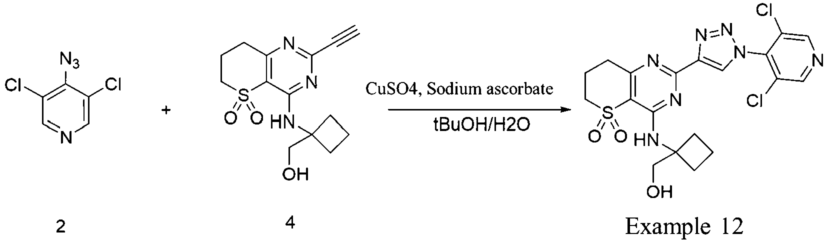

**Intermediate** 2 (231 mg, 1.22 mmol), **Intermediate** 4 (250 mg, 0.81 mmol), tert-butanol (8 mL), water (2 mL), anhydrous copper sulfate (20 mg, 0.08 mmol), and sodium ascorbate (32 mg, 0.16 mmol) were added in sequence to a 25 mL single-necked flask. The mixture was then reacted overnight at 60°C under nitrogen protection. TLC (*V*_{petroleum ether} : *V*_{ethyl acetate} = 1:2) monitored that the starting materials have completely reacted. After cooling to room temperature, the mixture was diluted with water (20 mL), extracted with dichloromethane (15 mL × 3), and the organic phases were combined, dried over anhydrous sodium sulfate, filtered, concentrated, and purified by column chromatography (*V*_{dichloromethane} : *V*ₘₑₜₕₐₙₒₗ = 100:1) to obtain 160 mg of white solid **Example 12,** with a yield of 39.6%.

¹H NMR (400 MHz, DMSO-*d*₆) *δ* (ppm): 9.19 (s, 1H, ArH), 9.03 (s, 2H, ArH), 7.01 (s, 1H, NH), 5.03 (s, 1H, OH), 3.77 (d, *J=5.6* Hz, 2H, CH₂OH), 3.67-3.60 (m, 2H, SO₂CH₂), 2.98 (t, J=6.4 Hz, 2H, SO₂CH₂CH₂), 2.46-2.31 (m, 4H, SO₂CH₂CH₂CH₂, cyclobutane-CH₂), 2.26-2.18 (m, 2H, cyclobutane-CH₂), 2.03-1.92 (m, 1H, cyclobutane-CH₂), 1.85-1.73 (m, 1H, cyclobutane-CH₂).

¹³C NMR (101 MHz, DMSO-*d*₆) *δ* (ppm): 165.66, 157.56, 156.74, 149.58, 146.84, 139.78, 129.89, 129.67, 114.37, 64.36, 59.33, 55.38, 51.01, 31.59, 30.21, 18.79, 14.45.

HRMS (ESI): m/z [M+H]⁺ Calcd for C₁₉H₁₉Cl₂N₇O₃S: 496.0725; Found: 496.0711.

### Example 13

### Step 1: Synthesis of tert-butyl 5-(((trifluoromethyl)sulfonyl)oxy)-2-azabicyclo[2.2.2]oct-5-ene-2-carboxylate (2)

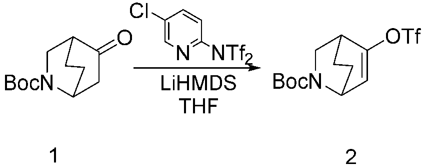

**Compound 1** (1.30 g, 5.77 mmol) and tetrahydrofuran (40 mL) were added in sequence to a 50 mL single-necked flask under nitrogen protection. LiHMDS (11.5 mL, 11.5 mmol, 1 M in THF) was added dropwise at -78°C and kept at this temperature for 30 min. Then, a solution of 2-[N, n-bis(trifluoromethanesulfonyl)amino]-5-chloropyridine (3.40 g, 8.66 mmol) in tetrahydrofuran (10 mL) was added dropwise. After the addition was complete, the mixture was slowly brought to room temperature and reacted overnight. TLC (*V*_{petroleum ether} : *V*_{ethyl acetate} = 5:1) monitored that the starting materials have completely reacted. The reaction was quenched with water (30 mL), extracted with ethyl acetate (30 mL × 3), and the organic phases were combined. The mixture was washed with saturated sodium chloride solution (50 mL), dried over anhydrous sodium sulfate, filtered, concentrated, and purified by column chromatography (*V*_{petroleum ether} : *V*_{ethyl acetate} = 20:1) to obtain 1.96 g of yellow oily **intermediate** 2, with a yield of 95.0%.

### Step 2 Synthesis of tert-butyl 5-(4,4,5,5-tetramethyl-1,3,2-dioxaborolan-2-yl)-2-azabicyclo[2.2.2]oct-5-ene-2-carboxylate (3)

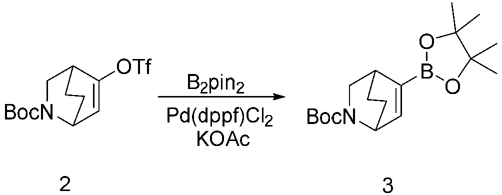

**Intermediate 2** (2.30 g, 6.44 mmol), 1,4-dioxane (45 mL), pinacol diborate (1.96 g, 7.72 mmol), potassium acetate (1.90 g, 19.3 mmol), and Pd(dppf)Cl₂ (470 mg, 0.64 mmol) were added in sequence to a 100 mL single-necked flask. The mixture was then reacted at 60°C for 3 h under nitrogen protection. TLC (*V*_{petroleum ether} : *V*_{ethyl acetate} = 5:1) monitored that the starting materials have completely reacted. The mixture was cooled, and the reaction was quenched with water (30 mL). The mixture was extracted with ethyl acetate (30 mL × 3). The organic phases were combined, washed with saturated sodium chloride solution (50 mL), dried over anhydrous sodium sulfate, filtered, and concentrated to obtain 2.70 g of black oily **intermediate 3,** which was used directly in the next step at a purity of 80%.

### Step 3 Synthesis of tert-butyl 5-(5-chloropyrimidin-2-yl)-2-azabicyclo[2.2.2]oct-5-ene-2-carboxylate (4)

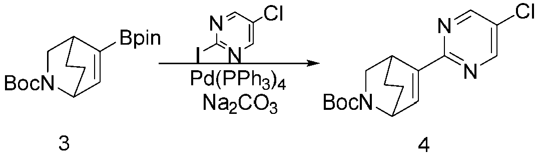

**Intermediate 3** (2.70 g, 6.44 mmol, 80% wt), 1,4-dioxane/water (40 mL/5 mL), 5-chloro-2-iodopyrimidine (1.86 g, 7.72 mmol), sodium carbonate (2.05 g, 19.3 mmol), and tetrakis(triphenylphosphine)palladium (675 mg, 0.64 mmol) were added in sequence to a 50 mL single-necked flask. The mixture was then reacted overnight at 90°C under nitrogen protection. TLC (*V*_{petroleum ether} : *V*_{ethyl acetate} = 5:1) monitored that the starting materials have completely reacted. After cooling, the reaction was quenched with water (30 mL), extracted with ethyl acetate (30 mL × 3), and the organic phases were combined. The mixture was washed with saturated sodium chloride solution (50 mL), dried over anhydrous sodium sulfate, filtered, concentrated, and purified by column chromatography (*V*_{petroleum ether} : *V*_{ethyl acetate} = 10:1) to obtain 1.40 g of yellow oily **intermediate 4,** with a two-step yield of 67.7%.

¹H NMR (400 MHz, CDCl₃) *δ* (ppm): 8.64 (s, 2H, ArH), 7.60-7.52 (m, 1H, ArH), 5.00-4.78 (br, 1H, NCH), 3.88-3.80 (m, 1H, NCH₂CH), 3.39-3.34 (m, 1H, H of NCH₂), 3.12-3.02 (m, 1H, H of NCH₂), 2.10-2.04 (m, 1H, H of CH₂CH₂), 1.82-1.74 (m, 1H, H of CH₂CH₂), 1.48-1.46 (m, 2H, H of CH₂CH₂), 1.45-1.40 (m, 9H, (CH₃)₃).

### Step 4 Synthesis of tert-butyl 5-(5-chloropyrimidin-2-yl)-2-azabicyclo[2.2.2]octane-2-carboxylate (5)

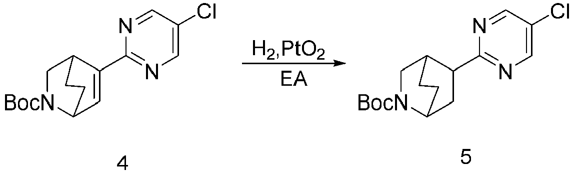

**Intermediate** 4 (0.90 g, 2.80 mmol), ethyl acetate (30 mL), and platinum dioxide (90 mg) were added in sequence to a 50 mL single-necked flask, and the mixture was reacted overnight at room temperature under a hydrogen atmosphere (15 psi). TLC (*V*_{petroleum ether} : *V*_{ethyl acetate} = 5:1) monitored that the starting materials have completely reacted. After cooling, the mixture was filtered through diatomaceous earth, concentrated, and purified by column chromatography (*V*_{petroleum ether} : *V*_{ethyl acetate} = 15:1) to obtain 390 g of white solid **intermediate 5,** with a yield of 43.0%.

¹H NMR (400 MHz, CDCl₃) *δ* (ppm): 8.66-8.58 (m, 2H, ArH), 4.19 (s, 0.5H, NCH), 4.06 (s, 0.5H, NCH), 3.38-3.23 (m, 2H, NCH₂), 3.24-3.15 (m, 1H, NCH₂CH), 2.64-2.56 (m, 1H, CH), 2.41-2.32 (m, 1H, H of CH₂CH₂), 2.14-1.90 (m, 2H, CH₂), 1.87-1.66 (m, 3H, H of CH₂CH₂), 1.45 (d, J=11.2 Hz, 9H, (CH₃)₃).

### Step 5 Synthesis of 5-(5-chloropyrimidin-2-yl)-2-azabicyclo[2.2.2]octane hydrochloride (6)

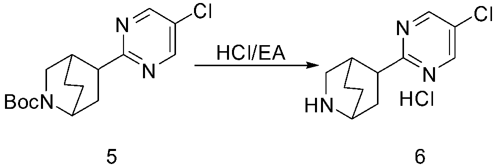

**Intermediate 5** (200 mg, 0.62 mmol) and 4 N HCl/EA (5 mL) were added in sequence to a 50 mL single-necked flask under nitrogen protection and the reaction was carried out at room temperature for 1 h. TLC (*V*_{petroleum ether} : *V*_{ethyl acetate} = 5:1) monitored that the starting materials have completely reacted. The mixture was then directly concentrated to obtain 137 mg of white solid **intermediate 6,** which was used directly in the next step without purification.

### Step 6 Synthesis of 2-(5-(5-chloropyrimidin-2-yl)-2-azabicyclo[2.2.2]octan-2-yl)-4-((1-(hydroxymethyl)cyclobutyl)amino)-6,7-dihydrothiopheno[3,2-d]pyrimidine 5-oxide (Example 13)

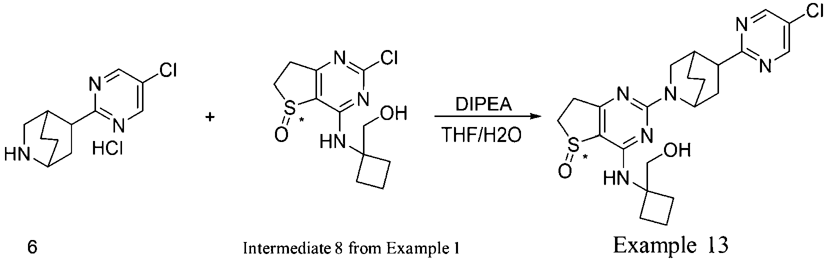

**Intermediate 6** (137 mg, 0.61 mmol), **Intermediate 8** (177 mg, 0.61 mmol) from **Example 1,** tetrahydrofuran/water (6 mL/1.5 mL), and DIPEA (238 mg, 1.84 mmol) were added in sequence to a 50 mL single-necked flask. The mixture was then reacted overnight at 65°C under nitrogen protection. TLC (*V*_{dichloromethane} : *V*ₘₑₜₕₐₙₒₗ = 10:1) monitored that the starting materials have completely reacted. After cooling, water (20 mL) was added, and the mixture was extracted with ethyl acetate (30 mL × 3). The organic phases were combined, washed with saturated sodium chloride solution (50 mL), dried over anhydrous sodium sulfate, filtered, concentrated, purified by column chromatography (*V*_{dichloromethane}: *V*ₘₑₜₕₐₙₒₗ = 50:1), and then purified by preparative plate (*V*_{dichloromethane} : *V*ₘₑₜₕₐₙₒₗ= 10:1) to obtain 175 mg of white solid **Example 13,** with a two-step yield of 59.7%.

¹H NMR (400 MHz, DMSO-*d*₆) *δ* (ppm): 8.95-8.80 (m, 2H, ArH), 7.19 (s, 1H, NH), 4.84-4.82 (m, 1H, OH), 4.79-4.69 (m, 1H, NCH), 3.72-3.71 (m, 1H, NCH₂CH), 3.66-3.61 (m, 1H, CH), 3.42-3.32 (m, 4H, CH₂OH, NCH₂CH), 3.29-3.09 (m, 2H, SOCH₂CH₂), 2.94-2.79 (m, 2H, SOCH₂CH₂), 2.41-2.22 (m, 3H, azabicyclo-octane-CH₂), 2.17-2.00 (m, 3H, azabicyclo-octane-CH₂), 1.90-1.63 (m, 6H, cyclobutane-CH₂).

¹³C NMR (101 MHz, DMSO-*d*₆) *δ* (ppm): 175.42, 175.00, 170.35, 161.48, 158.17, 157.81, 155.92, 128.80, 109.55, 109.09, 64.29, 58.90, 48.92, 46.18, 43.77, 43.03, 42.52, 32.78, 31.94, 31.03, 30.13, 25.59, 14.85.

HRMS (ESI): m/z [M+H]⁺ Calcd for C₂₂H₂₇ClN₆O₂S: 475.1683; Found: 475.1696

### Example 14

### Step 1 Synthesis of methyl 2-(4-((2-chloro-6,7-dihydrothiopheno[3,2-d]pyrimidin-4-yl)amino)-2-fluorophenyl)acetate (3)

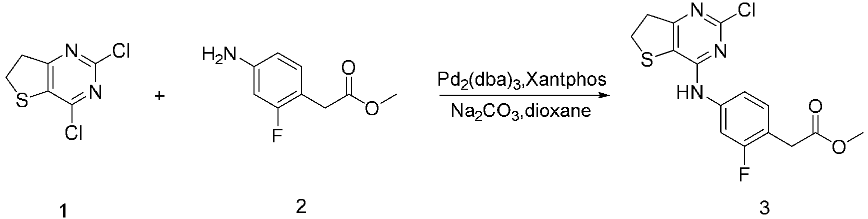

**Compound 1** (6.90 g, 33.3 mmol), **Compound 2** (6.10 g, 33.3 mmol), 1,4-dioxane (130 mL), Pd₂(dba)₃ (1.14 g, 1.25 mmol), Xantphos (1.45 g, 2.50 mmol), and sodium carbonate (7.06 g, 66.6 mmol) were added in sequence to a 500 mL single-necked flask. The mixture was then reacted overnight at 75°C under nitrogen protection. TLC (*V*_{petroleum ether} : *V*_{ethyl acetate} = 3:1) monitored that the starting materials have completely reacted. After cooling, the mixture was filtered, concentrated, and purified by column chromatography (*V*_{petroleum ether} : *V*_{ethyl acetate} = 3:1) to obtain 6.50 g of white solid **intermediate 3,** with a yield of 55.1%.

¹H NMR (400 MHz, DMSO-*d*₆) *δ* (ppm): 9.29 (s, 1H, NH), 7.52 (dd, *J*₁=12.4 Hz, *J*₂=2.0 Hz, 1H, ArH), 7.36 (dd, *J*₁=8.4 Hz, *J*₂=2.0 Hz, 1H, ArH), 7.29 (t, J=8.4 Hz, 1H, ArH), 3.69 (s, 2H, CH₂COOCH₃), 3.63 (s, 3H, COOCH₃), 3.46-3.39 (m, 2H, SCH₂), 3.25 (t, J=8.4 Hz, 2H, SCH₂CH₂).

### Step 2 Synthesis of methyl 2-(4-((2-chloro-5-oxo-6,7-dihydrothiopheno[3,2-d]pyrimidin-4-yl)amino)-2-fluorophenyl)acetate (4)

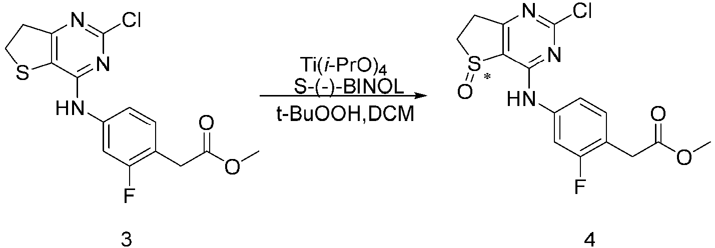

**Intermediate 3** (2.00 g, 5.6 mmol), dichloromethane (15 mL), S-(-)-BINOL (160 mg, 0.56 mmol), tetraisopropyl titanate (80 mg, 0.28 mmol), and water (100 mg, 5.6 mmol) were added in sequence to a 50 mL single-necked flask. The mixture was reacted at room temperature for 1 h under nitrogen protection. Then, 70% wt peroxytert-butanol (800 mg, 6.1 mmol) was added dropwise. After the addition was complete, the reaction was continued for 2 h. TLC (*V*_{dichloromethane} : *V*ₘₑₜₕₐₙₒₗ = 20:1) monitored that the starting materials have completely reacted. The sample was uploaded by wet method and purified by column chromatography (*V*_{dichloromethane} : *V*ₘₑₜₕₐₙₒₗ = 100:1) to obtain 1.85 g of yellow solid **intermediate 4,** with a yield of 88.4%.

¹H NMR (400 MHz, DMSO-*d*₆) *δ* (ppm): 10.56 (s, 1H, NH), 7.55 (dd, *J*₁=12.0 Hz, *J*₂=2.0 Hz, 1H, ArH), 7.44-7.33 (m, 2H, ArH), 3.73 (s, 2H, CH₂COOCH₃), 3.72-3.65 (m, 1H, H of SOCH₂), 3.64 (s, 3H, COOCH₃), 3.49-3.42 (m, 1H, H of SOCH₂), 3.30-3.23 (m, 1H, H of SOCH₂CH₂), 3.17-3.11 (m, 1H, H of SOCH₂CH₂).

### Step 3 Synthesis of methyl 2-(4-((2-(4-(5-chloropyrimidin-2-yl)-3,6-dihydropyridin-1(2H)-yl)-5-0x0-6,7-dihydrothiopheno[3,2-d]pyrimidin-4-yl)amino)-2-fluorophenyl)acetate (5)

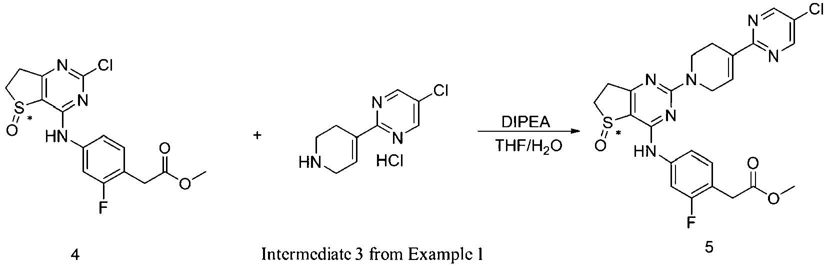

**Intermediate 4** (2.00 g, 5.4 mmol), **Intermediate 3** (1.32 g, 5.7 mmol) from **Example 1,** tetrahydrofuran/water (40 mL/10 mL), and DIPEA (2.9 mL, 16.3 mmol) were added in sequence to a 250 mL single-necked flask. The mixture was then reacted overnight at 65°C under nitrogen protection. TLC (*V*_{dichloromethane} : *V*ₘₑₜₕₐₙₒₗ = 15:1) monitored that the starting materials have completely reacted. After cooling, the mixture was concentrated, purified by column chromatography (*V*_{dichloromethane} : *V*ₘₑₜₕₐₙₒₗ = 100:1), and further purified by two slurries (30 mL × 2, *V*_{petroleum ether} : *V*_{ethyl acetate} = 1:1, *V*_{ethyl acetate} : *V*_{dichloromethane} = 5:1) to obtain 930 mg of white solid **intermediate 5,** with a yield of 32.5%.

¹H NMR (400 MHz, DMSO-*d*₆) *δ* (ppm): 9.72 (s, 1H, NH), 8.90 (s, 2H, ArH), 7.67 (d, *J=10.4* Hz, 1H, ArH), 7.56 (d, J=8.4 Hz, 1H, ArH), 7.35-7.28 (m, 2H, ArH and C=CH), 4.50 (br, 2H, NCH₂CH), 4.01 (br, 2H, NCH₂CH₂), 3.71 (s, 2H, CH₂COOCH₃), 3.64 (s, 3H, COOCH₃), 3.59-3.51 (m, 1H, H of SOCH₂), 3.30-3.26 (m, 1H, H of SOCH₂), 3.12-3.06 (m, 1H, H of SOCH₂CH₂), 3.03-2.96 (m, 1H, H of SOCH₂CH₂), 2.70 (br, 2H, NCH₂CH₂).

### Step 4 Synthesis of 2-(4-((2-(4-(5-chloropyrimidin-2-yl)-3,6-dihydropyridin-1(2H)-yl)-5-oxo-6,7-dihydrothiopheno[3,2-d]pyrimidin-4-yl)amino)-2-fluorophenyl)acetic acid (Example 14)

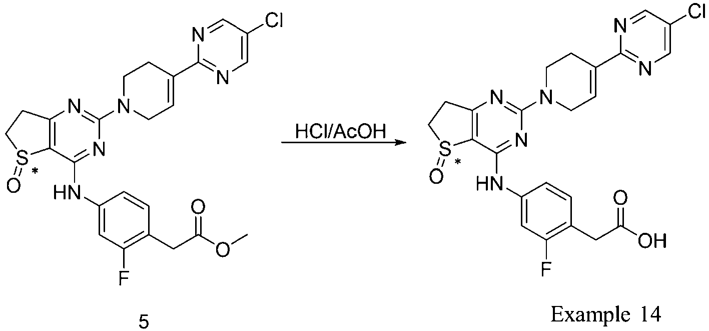

**Intermediate 5** (450 mg, 0.85 mmol), acetic acid (4.5 mL), and 37% concentrated hydrochloric acid (1.5 mL) were added in sequence to a 25 mL single-necked flask. The mixture was then reacted at 60°C for 4 h under nitrogen protection. TLC (*V*_{dichloromethane}: *V*ₘₑₜₕₐₙₒ = 20:1) monitored that the starting materials have completely reacted. After cooling, the mixture was directly concentrated, water (20 mL) was added, and the solid precipitated. The solid was filtered and then purified by slurrying (15 mL, *V*_{dichloromethane}: *V*ₘₑₜₕₐₙₒₗ = 50:1) to obtain 250 mg of white solid **Example 14,** with a yield of 57.0%.

¹H NMR (400 MHz, DMSO-*d*₆) *δ* (ppm): 12.43 (s, 1H, COOH), 9.71 (s, 1H, NH), 8.90 (s, 2H, ArH), 7.65 (d, *J=10.4* Hz, 1H, ArH), 7.54 (d, J=8.4 Hz, 1H, ArH), 7.33-7.28 (m, 2H, ArH and C=CH), 4.50 (br, 2H, NCH₂CH), 4.02 (br, 2H, NCH₂CH₂), 3.59 (s, 2H, CH₂COOH), 3.59-3.51 (m, 1H, H of SOCH₂), 3.30-3.26 (m, 1H, H of SOCH₂), 3.13-3.06 (m, 1H, H of SOCH₂CH₂), 3.02-2.96 (m, 1H, H of SOCH₂CH₂), 2.70 (br, 2H, NCH₂CH₂).

### Example 15

### Step 1 Synthesis of methyl 2-(4-((2-(4-(5-chloropyrimidin-2-yl)piperazin-1-yl)-5-oxo-6,7-dihydrothiopheno[3,2-d]pyrimidin-4-yl)amino)-2-fluorophenyl)acetate (2)

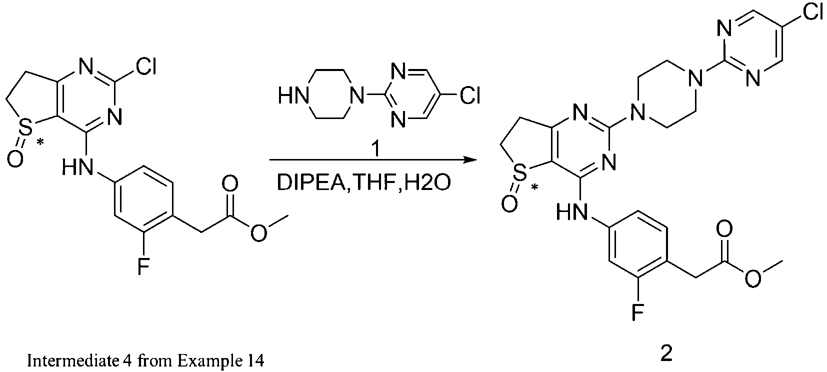

**Intermediate 4** (307 mg, 0.83 mmol) from **Example 14,** THF (8 mL), H₂O (2 mL), 5-chloro-2-(piperazin-1-yl)pyrimidine **(1,** 180 mg, 0.90 mmol), and DIPEA (322 mg, 2.49 mmol) were added in sequence to a 25 mL single-necked flask. After addition, th e mixture was reacted overnight at 65°C under nitrogen protection. TLC (*V*_{dichloromethane} : *V*ₘₑₜₕₐₙₒₗ = 15:1) monitored that the starting materials have completely reacted. The r eaction solution was concentrated and purified by flash preparative chromatography (1 2 g, *V*_{dichloromethane} : *V*ₘₑₜₕₐₙₒₗ = 50 : 1) to obtain 395 mg of white solid **intermediate 2,** w ith a yield of 90.2%.

¹H NMR (400 MHz, DMSO-*d*₆) *δ* (ppm): 9.73 (s, 1H, NH), 8.44 (s, 2H, ArH), 7.59 (d, J=12.8 Hz, 1H, ArH), 7.51 (d, J=8.4 Hz, 1H, ArH), 7.30 (t, J=8.8 Hz, 1H, ArH), 3.91-3.76 (m, 8H, N(CH₂CH₂)₂), 3.69 (s, 2H, CH₂COOCH₃), 3.63 (s, 3H, COOCH₃), 3.57-3.48 (m, 1H, H of SOCH₂), 3.27-3.25 (m, 1H, H of SOCH₂), 3.11-3.03 (m, 1H, H of SOCH₂CH₂), 3.01-2.95 (m, 1H, H of SOCH₂CH₂).

### Step 2 Synthesis of 2-(4-((2-(4-(5-chloropyrimidin-2-yl)piperazin-1-yl)-5-oxo-6,7-dihydrothiopheno[3,2-d]pyrimidin-4-yl)amino)-2-fluorophenyl)acetic acid (Example 15)

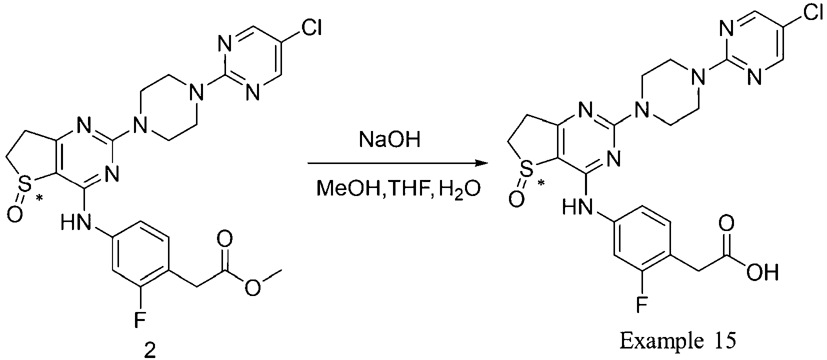

**Intermediate** 2 (300 mg, 0.58 mmol), MeOH (3.7 mL), THF (3.7 mL), H₂O (2.7 mL), and NaOH (108 mg, 2.70 mmol) were added in sequence to a 50 mL single-necked flask. The mixture was then reacted at 35°C for 4 h under nitrogen protection. TLC (*V*_{dichloromethane} : *V*ₘₑₜₕₐₙₒₗ = 10:1) monitored that the starting materials have completely reacted. Water (10 mL) was added to quench the reaction, and the pH was adjusted to 5 with diluted HCl (1M). A pale yellow solid precipitated out. After filtration, the solid was slurried with MeOH (10 mL) for 4 h, and 200 mg of pale yellow solid was obtained by filtration **(Example 15,** with a yield of 68.41%).

¹H NMR (400 MHz, DMSO-*d*₆) *δ* (ppm): 12.42 (s, 1H, COOH), 9.73 (s, 1H, NH), 8.46 (s, 2H, ArH), 7.57(d, J=12.8 Hz, 1H, ArH), 7.50 (d, J=6.4 Hz, 1H, ArH), 7.29 (t, J=8.6 Hz, 1H, ArH), 3.95-3.77 (m, 8H, N(CH₂CH₂)₂), 3.59 (s, 2H, CH₂COOH), 3.57-3.50 (m, 1H, H of SOCH₂), 3.28-3.26 (m, 1H, H of SOCH₂), 3.11-3.05 (m, 1H, H of SOCH₂CH₂), 3.02-2.97 (m, 1H, H of SOCH₂CH₂).

### Example 16

### Step 1: Synthesis of sodium 2-(4-((2-(4-(5-chloropyrimidin-2-yl)piperazin-1-yl)-5-oxo-6,7-dihydrothiopheno[3,2-d]pyrimidin-4-yl)amino)-2-fluorophenyl)acetate (Example 16)

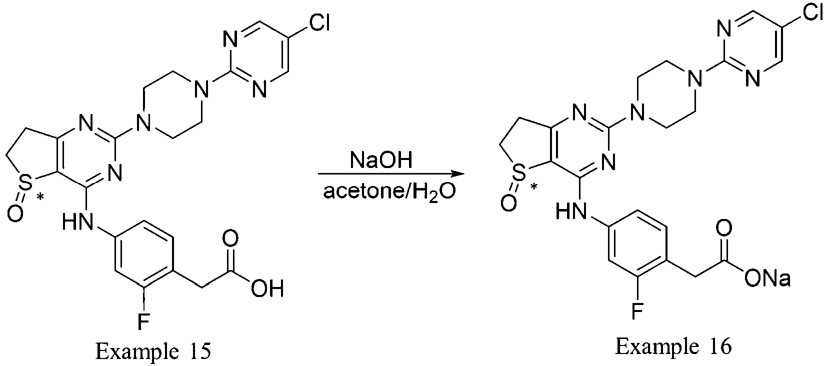

**Example 15** (150 mg, 0.29 mmol), water (2.5 mL), and sodium hydroxide (13 mg, 0.32 mmol) were added in sequence to a 25 mL single-necked flask. The reaction was carried out at room temperature for 0.5 h under nitrogen protection. The solid was slowly dissolved. The mixture was cooled in an ice-water bath. After 30 min, acetone (15 mL) was added. A white solid precipitated and was stirred for further 30 min. The precipitate was then filtered and dried to obtain 105 mg of white solid, **Example 16,** with a yield of 67.2%.

¹H NMR (400 MHz, DMSO-*d*₆) *δ* (ppm): 9.68 (s, 1H, NH), 8.45 (s, 2H, ArH), 7.43 (d, J=12.4 Hz, 1H, ArH), 7.36 (d, J=8.4 Hz, 1H, ArH), 7.20 (t, J=8.4 Hz, 1H, ArH), 3.91-3.78 (m, 8H, N(CH₂CH₂)₂), 3.57-3.49 (m, 1H, H of SOCH₂), 3.30-3.24 (m, 1H, H of SOCH₂), 3.18 (s, 2H, CH₂COONa), 3.09-3.03 (m, 1H, H of SOCH₂CH₂), 3.00-2.95 (m, 1H, H of SOCH₂CH₂).

### Example 17

### Step 1 Synthesis of methyl 2-(4-((2-(4-(5-chloropyrimidin-2-yl)piperidin-1-yl)-5-oxo-6,7-dihydrothiopheno[3,2-d]pyrimidin-4-yl)amino)-2-fluorophenyl)acetate (2)

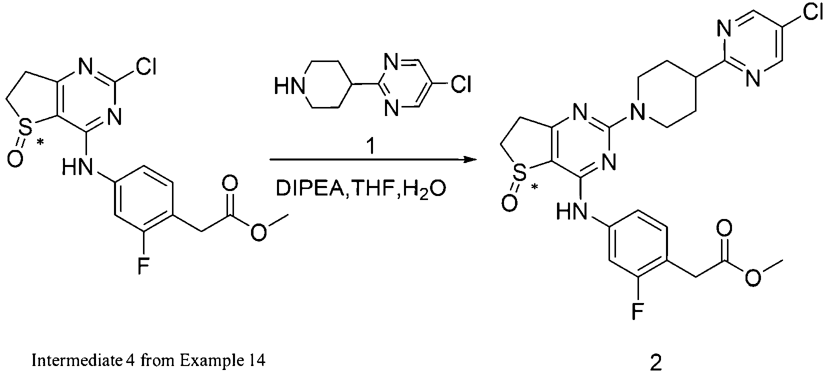

**Intermediate 4** (307 mg, 0.83 mmol) from **Example 14,** THF (8 mL), H₂O (2 mL), 5-chloro-2-(piperidin-4-yl)pyrimidine **(1,** 180 mg, 0.91 mmol), and DIPEA (322 mg, 2.49 mmol) were added in sequence to a 25 mL single-necked flask. After addition, th e mixture was reacted overnight at 65°C under nitrogen protection. TLC (*V*_{dichloromethane} : *V*ₘₑₜₕₐₙₒₗ = 15:1) monitored that the starting materials have completely reacted. The r eaction solution was concentrated and purified by flash preparative chromatography (1 2 g, *V*_{dichloromethane} : *V*ₘₑₜₕₐₙₒₗ = 50 : 1) to obtain 320 mg of yellow solid intermediate 2, with a yield of 73.85%.

### Step 2 Synthesis of 2-(4-((2-(4-(5-chloropyrimidin-2-yl)piperidin-1-yl)-5-oxo-6,7-dihydrothiopheno[3,2-d]pyrimidin-4-yl)amino)-2-fluorophenyl)acetic acid (Example 17)

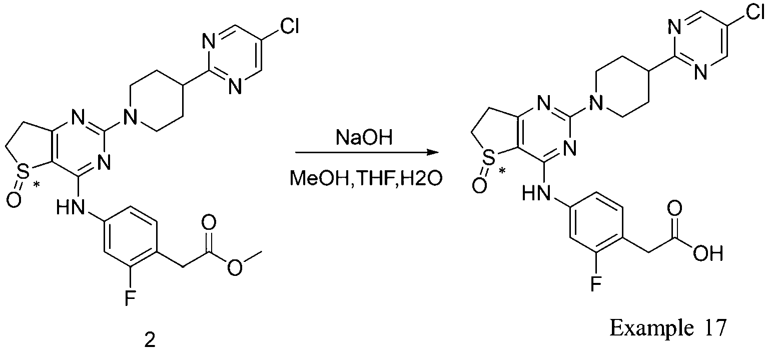

**Intermediate 2** (200 mg, 0.39 mmol), MeOH (2.5 mL), THF (2.5 mL), H₂O (1.8 mL), and NaOH (71 mg, 1.78 mmol) were added in sequence to a 25 mL single-necked flask. After the addition was complete, the mixture was then reacted at 35°C for 4 h under nitrogen protection. TLC (*V*_{dichloromethane} : *V*ₘₑₜₕₐₙₒₗ = 10:1) monitored that the starting materials have completely reacted. Water (10 mL) was added to quench the reaction, and the pH was adjusted to 5 with diluted HCl (1 N). A pale yellow solid precipitated out. After filtration, the mixture was slurried with MeOH (10 mL) for 4 h, filtered and dried, and 115 mg of pale yellow solid was obtained **(Example 17,** with a yield of 60.52%).

¹H NMR (400 MHz, DMSO-*d*₆) *δ* (ppm): 12.40 (s, 1H, COOH), 9.68 (s, 1H, NH), 8.87 (s, 2H, ArH), 7.62 (d, J=12.8 Hz, 1H, ArH), 7.47 (d, J=8.4 Hz, 1H, ArH), 7.26 (t, J=8.4 Hz, 1H, ArH), 4.69 (br, 2H, SOCH₂), 3.57 (s, 2H, CH₂COOH), 3.55-3.48 (m, 1H, CH), 3.29-3.13 (m, 4H, CH₂NCH₂), 3.09-3.02 (m, 1H, H of SOCH₂CH₂), 3.01-2.95 (m, 1H, H of SOCH₂CH₂), 2.07-1.98 (m, 2H, NCH₂CH₂), 1.78-1.64 (m, 2H, NCH₂CH₂).

HRMS (ESI): m/z [M+H]⁺ Calcd for C₂₂H₂₄ClFN₆O₃S: 517.1225; Found: 517.1194.

### Example 18

### Step 1 Synthesis of methyl 2-(4-((2-(4-(5-chloropyrimidin-2-yl)-3,6-dihydropyridin-1(2H)-yl)-5,5-dioxo-7,8-dihydro- 6H -thiapyrano[3,2-d]pyrimidin-4-yl)amino)-2-fluorophenyl)acetate (2)

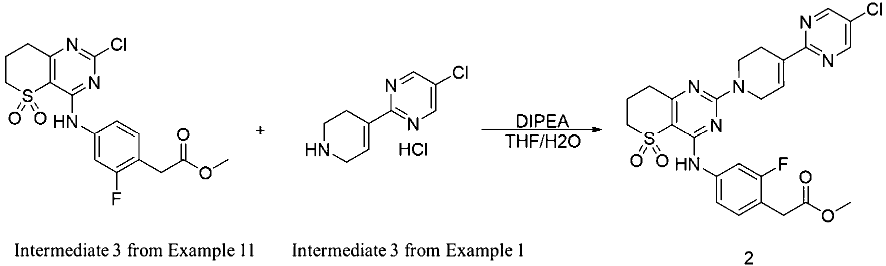

**Intermediate 3** (2.00 g, 5.0 mmol) from **Example 11, Intermediate 3** (1.28 g, 5.5 mmol) from **Example 1,** tetrahydrofuran/water (40 mL/4 mL), and DIPEA (2.7 mL, 15.0 mmol) were added in sequence to a 250 mL single-necked flask. The mixture was then reacted overnight at 65°C under nitrogen protection. TLC (*V*_{dichloromethane} : *V*ₘₑₜₕₐₙₒₗ = 20:1) monitored that the starting materials have completely reacted. After cooling, water (20 mL) was added, followed by extraction with ethyl acetate (30 mL × 3). The organic phases were combined, washed with saturated sodium chloride solution (50 mL), dried over anhydrous sodium sulfate, filtered, concentrated, purified by column chromatography (*V*_{dichloromethane} : Vmethanol = 100:1), and then slurried (150 mL, *V*_{petroleum ether} : *V*_{ethyl acetate} = 2:1) to obtain 850 mg of white solid **intermediate 2,** with a yield of 30.4%.

¹H NMR (400 MHz, DMSO-*d*₆) *δ* (ppm): 8.89 (s, 2H, ArH), 8.70 (s, 1H, NH), 7.63 (d, *J*=12.0 Hz, 1H, CH), 7.38-7.25 (m, 3H, ArH), 4.46 (br, 2H, NCH₂), 3.96 (br, 2H, NCH₂), 3.72 (s, 2H, COCH₂), 3.64 (s, 3H, OCH₃), 3.58-3.55 (m, 2H, SO₂CH₂), 2.84 (t, J=6.4 Hz, 2H, SO₂CH₂CH₂), 2.68 (s, 2H, SO₂CH₂CH₂CH₂), 2.33-2.25 (m, 2H, NCH₂CH₂).

### Step 2 Synthesis of 2-(4-((2-(4-(5-chloropyrimidin-2-yl)-3,6-dihydropyridin-1(2H)-yl)-5,5-dioxo-7,8-dihydro- 6H -thiapyrano[3,2-d]pyrimidin-4-yl)amino)-2-fluorophenyl)acetic acid (Example 18)

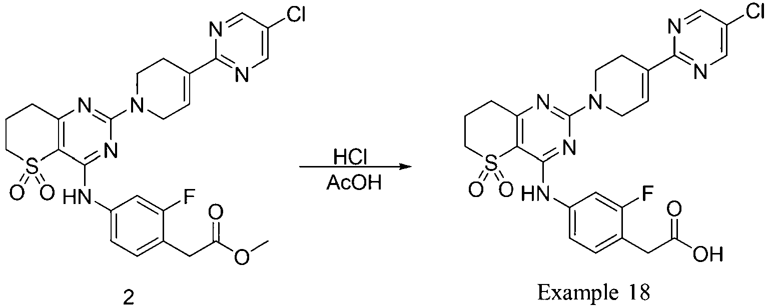

**Intermediate 2** (50 mg, 0.09 mmol), acetic acid (2.0 mL), and 37% concentrated hydrochloric acid (1.0 mL) were added in sequence to a 25 mL single-necked flask. The mixture was then reacted at 80°C for 1 h under nitrogen protection. TLC (*V*_{dichloromethane} : *V*ₘₑₜₕₐₙₒₗ = 20:1) monitored that the starting materials have completely reacted. After cooling, the mixture was directly concentrated, water (10 mL) was added, and the solid precipitated. After filtration and drying, 35 mg of white solid was obtained **(Example 18,** with a yield of 71.8%).

¹H NMR (400 MHz, DMSO-*d*₆) *δ* (ppm): 12.44 (s, 1H, COOH), 8.89 (s, 2H, ArH), 8.70 (s, 1H, NH), 7.62 (d, J=12.0 Hz, 1H, CH), 7.35-7.24 (m, 3H, ArH), 4.46 (br, 2H, NCH₂), 3.97 (br, 2H, NCH₂), 3.64-3.54 (m, 4H, SO₂CH₂), 2.84 (t, J=6.4 Hz, 2H, SO₂CH₂CH₂), 2.68 (d, J=6.4 Hz, 2H, SO₂CH₂CH₂CH₂), 2.33-2.25 (m, 2H, NCH₂CH₂).

¹³C NMR (101 MHz, DMSO-*d*₆) *δ* (ppm): 172.24, 167.11, 160.70 (d, J=240.5 Hz), 156.09, 155.89, 138.91, 132.29, 128.83, 117.71 (d, *J=13.4* Hz), 117.27, 108.55 (d, *J=27.0* Hz), 106.78, 51.29, 44.61, 34.20, 31.85, 25.18, 18.71.

HRMS (ESI): m/z [M+H]⁺ Calcd for C₂₄H₂₂ClFN₆O₄S: 545.1174; Found: 545.1160.

### Example 19

### Step 1: Synthesis of ammonium 2-(4-((2-(4-(5-chloropyrimidin-2-yl)-3,6-dihydropyridin-1(2H)-yl)-5,5-dioxo-7,8-dihydro- 6H -thiapyrano[3,2-d]pyrimidin-4-yl)amino)-2-fluorophenyl) acetate(Example 19)

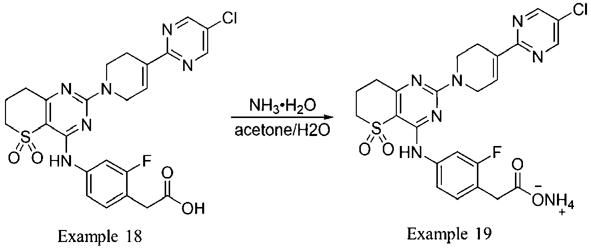

80 mg (0.15 mmol) of **Example 18** and 5 mL of water were added to a 25 mL single-necked flask. The solid was not dissolved. The pH was adjusted to 8 by adding 27% ammonia dropwise in an ice-water bath. After stirring for 15 min, the system became clear. After addition of 10 mL of acetone, it was stirred continually, and a white solid slowly precipitated out. After 30 min, it is filtered and dried to obtain 40 mg of white solid **of Example 19** with a yield of 48.5%.

¹H NMR (400 MHz, DMSO-*d*₆) *δ* (ppm): 8.89 (s, 2H, ArH), 7.58 (d, J=12.1 Hz, 1H, CH), 7.35-7.22 (m, 3H, ArH), 4.46 (br, 2H, NCH₂), 3.96 (br, 2H, NCH₂), 3.61-3.53 (m, 2H, COCH₂), 3.49 (s, 2H, SO₂CH₂), 2.84 (t, J=6.4 Hz, 2H, SO₂CH₂CH₂), 2.68 (s, 2H, SO₂CH₂CH₂CH₂), 2.29 (t, *J=6.0* Hz, 2H, NCH₂CH₂).

### Example 20

### Step 1: Synthesis of ammonium 2-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2-fluorophenyl) acetate (Example 20)

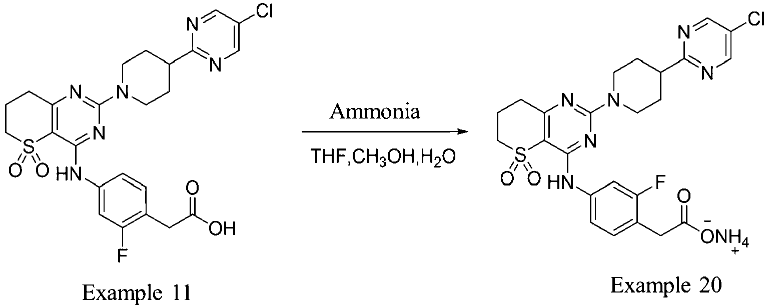

**Example** 11 (300 mg, 0.55 mmol), THF (4.5 mL), and methanol (4.5 mL) were added to a 50 mL single-necked flask in sequence, and stirred at room temperature until homogeneous. Ammonia water (0.1 mL) was slowly added dropwise to the above reaction system, and the reaction was allowed to proceed overnight at room temperature. The system gradually became turbid from clear. After filtration, the filter cake was rinsed with water (2 mL × 3) and collected. After drying at 50°C overnight with forced air, 200 mg of white solid was obtained **(Example 20,** with a yield of 64.5%).

¹H NMR (400 MHz, DMSO-*d*₆) *δ* (ppm): 8.86 (s, 2H, ArH), 8.63 (s, 1H, NH), 7.56 (dd, *J*₁=12.2 Hz, *J*₂=2.1 Hz, 1H, ArH), 7.27 (t, J=8.4 Hz, 1H, ArH), 7.19 (dd, *J*₁=8.3 Hz, *J*₂=2.1 Hz, 1H, ArH), 4.60 (br, 2H, SO₂CH₂), 3.58-3.54 (m, 2H, NCH₂), 3.52 (s, 2H, ArCH₂CO), 3.23-3.11 (m, 3H, NCH₂ and CH), 2.81 (t, J=6.4 Hz, 2H, SO₂CH₂CH₂CH₂), 2.31-2.24 (m, 2H, SO₂CH₂CH₂), 2.04-2.00 (m, 2H, NCH₂CH₂), 1.70-1.67 (m, 2H, NCH₂CH₂).

HRMS (ESI): m/z [M+H]⁺ Calcd for C₂₄H₂₄ClFN₆O₄S: 547.1331; Found: 547.1342.

### Example 21

### Step 1 Synthesis of dimethyl 2-(2-chloro-4-nitrophenyl)malonate (2)

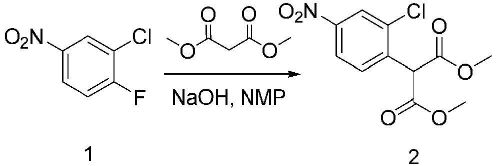

**Compound 1** (5.00 g, 28.51 mmol)), NMP (100 mL), dimethyl malonate (4.78 g, 36.22 mmol) and sodium hydroxide (2.40 g, 59.85 mmol) were added in sequence to a 250 mL reaction flask. The mixture was then reacted overnight at 80°C for 2 h under nitrogen protection. TLC (*V*_{petroleum ether} : *V*_{ethyl acetate} = 10:1)monitored that the reaction was completed. The reaction solution was cooled to room temperature and quenched with water (300 mL). The pH was adjusted to 3 with diluted hydrochloric acid (1 N) in an ice bath, and a solid precipitated. The solid was filtered, and the filter cake was rinsed with water (50 mL). The filter cake was collected and dried under vacuum for 2 h to obtain 7.90 g of yellow solid **intermediate 2,** with a yield of 85.4%.

¹H NMR (400 MHz, CDCl₃) *δ* (ppm): 8.30 (d, J=2.4 Hz, 1H, ArH), 8.15 (dd, *J*₁=8.6 Hz, *J*₂=2.4 Hz, 1H, ArH), 7.74 (d, J=8.6 Hz, 1H, ArH), 5.31 (s, 1H, CH), 3.81 (s, 6H, 2CH₃).

### Step 2 Synthesis of methyl 2-(2-chloro-4-nitrophenyl)acetate (3)

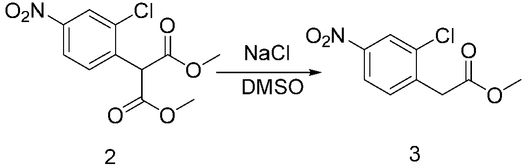

**Intermediate 2** (1.00 g, 3.48 mmol), DMSO (10 mL), water (0.06 mL), and sodium chloride (366 mg, 6.26 mmol) were added in sequence to a 50 mL reaction flask and the reaction was carried out at 110°C for 16 h under nitrogen protection. TLC (*V*_{petroleum ether} : *V*_{ethyl acetate} = 5:1) monitored that the reaction was completed. The reaction solution was cooled to room temperature, quenched with water (30 mL), extracted with ethyl acetate (50 mL × 3), the organic phases were combined, washed with saturated brine (50 mL × 4), the organic phase was collected, dried over anhydrous sodium sulfate, filtered, concentrated and purified by flash preparative chromatography (40 g, *V*_{petroleum ether} : *V*_{ethyl acetate} = 5:1) to obtain 320 mg of yellow oily **intermediate** 3, with a yield of 40.1%.

¹H NMR (400 MHz, CDCl₃) *δ* (ppm): 8.28 (d, J=2.4 Hz, 1H, ArH), 8.11 (dd, *J*₁=8.4 Hz, *J*₂=2.4 Hz, 1H, ArH), 7.50 (d, J=8.4 Hz, 1H, ArH), 3.88 (s, 2H, CH₂), 3.74 (s, 3H, CH₃).

### Step 3 Synthesis of methyl 2-(4-amino-2-chlorophenyl)acetate (4)

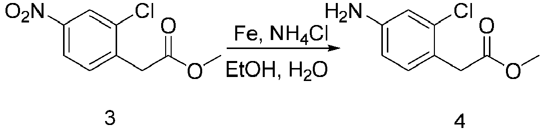

**Intermediate** 3 (320 mg, 1.39 mmol), ethanol (4 mL), water (1 mL), iron powder (389 mg, 6.97 mmol), and ammonium chloride (38 mg, 0.70 mmol) were added in sequence to a 25 mL reaction flask. The reaction was carried out at 85°C for 1 h under nitrogen protection. TLC (*V*_{petroleum ether} : *V*_{ethyl acetate} = 3:1) monitored that the starting materials have completely reactedd. The reaction solution was filtered through diatomaceous earth, the filter cake was rinsed with ethyl acetate (50 mL), the filtrate was collected, concentrated and purified by flash preparative chromatography (12 g, *V*_{petroleum ether} : *V*_{ethyl acetate} = 3:1) to obtain 220 mg of yellow oily **intermediate 4,** with a yield of 75.5%.

### Step 4 Synthesis of methyl 2-(2-chloro-4-((2-chloro-7,8-dihydro- 6H - thiapyrano[3,2-d]pyrimidin-4-yl)amino)phenyl)acetate (5)

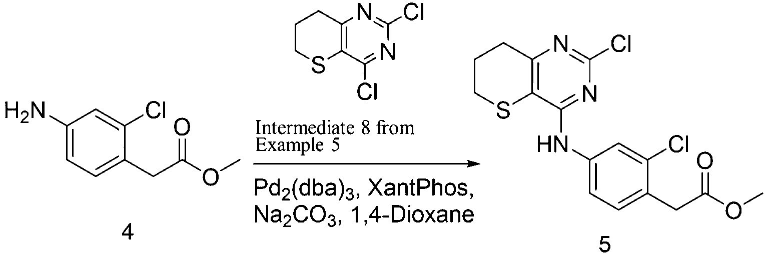

**Intermediate 4** (210 mg, 1.05 mmol), 1,4-dioxane (5 mL), **Intermediate** 8 (280 mg, 1.26 mmol) from **Example 5,** sodium carbonate (223 mg, 2.10 mmol), Xantphos (91 mg, 0.16 mmol), and Pd₂(dba)₃ (72 mg, 0.08 mmol) were added in sequence to a 25 mL single-necked flask. The reaction was carried out at 70°C for 16 h under nitrogen protection. TLC (*V*_{ethyl acetate} : *V*_{petroleum ether} = 3 : 1) monitored that the starting materials have completely reacted. The reaction solution was filtered through diatomaceous earth, the filter cake was rinsed with ethyl acetate (50 mL), the filtrate was collected, concentrated and purified by flash preparative chromatography (12 g, *V*_{petroleum ether} : *V*_{ethyl acetate} = 3:1) to obtain 290 mg of white solid **intermediate 5,** with a yield of 57.6%.

¹H NMR (400 MHz, DMSO-*d*₆) *δ* (ppm): 8.70 (s, 1H, NH), 7.69 (d, J=2.2 Hz, 1H, ArH), 7.50 (dd, *J*₁=8.4 Hz, *J*₂=2.2 Hz, 1H, ArH), 7.36 (d, J=8.4 Hz, 1H, ArH), 3.79 (s, 2H, CH₂COOCH₃), 3.64 (s, 3H, COOCH₃), 3.16-3.09 (m, 2H, SCH₂), 2.79 (t, *J=* 6.3 Hz, 2H, SCH₂CH₂CH₂), 2.16-2.10 (m, 2H, SCH₂CH₂).

### Step 5 Synthesis of methyl 2-(2-chloro-4-((2-chloro-5,5-dioxo-7,8-dihydro- 6H - thiapyrano[3,2-d]pyrimidin-4-yl)amino)phenyl)acetate (6)

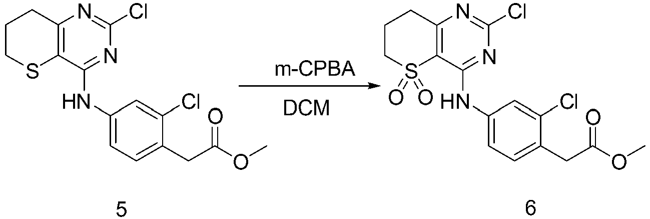

**Intermediate 5** (280 mg, 0.73 mmol), DCM (10 mL), and m-chloroperoxybenzoic acid (85%, 371 mg, 3.84 mmol) were added in sequence to a 50 mL single-necked flask and reacted at room temperature for 2 h under nitrogen protection. TLC (*V*_{ethyl acetate} : *V*_{petroleum ether} = 2 : 1) monitored that the starting materials have completely reacted. The reaction was quenched by addition of saturated sodium thiosulfate (10 mL), extracted with dichloromethane (30 mL × 3). The organic phases were combined, washed with saturated brine (50 mL × 3), collected, dried, filtered, concentrated, and purified by flash preparative chromatography (12 g, *V*_{dichloromethane} : *V*ₘₑₜₕₐₙₒₗ = 100: 1) to obtain 230 mg of white solid **intermediate** 6, with a yield of 75.9%.

### Step 6 Synthesis of methyl 2-(2-chloro-4-((2-(4-(5-chloropyrimidin-2-yl)piperidin-1-yl)-5,5-dioxo-7,8-dihydro- 6H -thiapyrano[3,2-d]pyrimidin-4-yl)amino)phenyl)acetate (7)

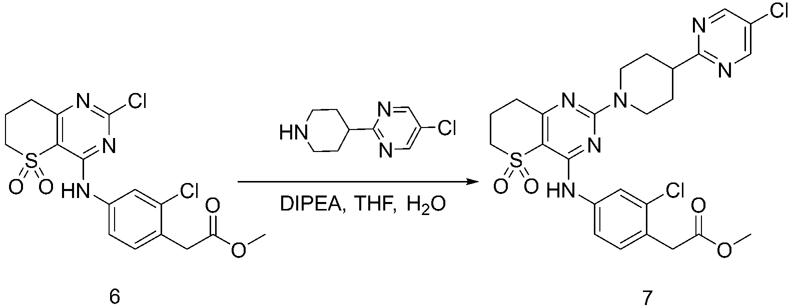

**Intermediate** 6 (230 mg, 0.55 mmol), tetrahydrofuran (3 mL), water (0.6 mL), 5-chloro-2-(piperidin-1-yl)pyrimidine (121 mg, 0.61 mmol) and DIPEA (215 mg, 1.66 mmol) were added in sequence to a 25 mL single-necked flask, and the reaction was carried out at 65°C for 16 h under a nitrogen atmosphere. TLC (*V*_{dichloromethane} : *V*ₘₑₜₕₐₙₒₗ = 20:1) monitored that the starting materials have completely reacted. The reaction solution was cooled to room temperature, directly mixed, concentrated, and purified by flash preparative chromatography (12 g, *V*_{dichloromethane} : *V*ₘₑₜₕₐₙₒₗ = 100:1) to obtain 280 mg of white solid **intermediate 7,** with a yield of 91.5%.

¹H NMR (400 MHz, DMSO-*d*₆) *δ* (ppm): 8.87 (s, 2H, ArH), 8.66 (s, 1H, ArH), 7.92 (s, 1H, NH), 7.37 (s, 2H, ArH), 4.56 (br, 2H, SO₂CH₂), 3.78 (s, 2H, CH₂COOCH₃), 3.62 (s, 3H, CH₂COOCH₃), 3.59-3.52 (m, 2H, NCH₂), 3.26-3.10 (m, 3H, NCH₂ and CH), 2.82 (t, J=6.4 Hz, 2H, SO₂CH₂CH₂CH₂), 2.31-2.25 (m, 2H, SO₂CH₂CH₂), 2.04-1.99 (m, 2H, CHCH₂), 1.70 (br, 2H, CHCH₂).

### Step 7: Synthesis of 2-(2-chloro-4-((2-(4-(5-chloropyrimidin-2-yl)piperidin-1-yl)-5,5-dioxo-7,8-dihydro- 6H -thiapyrano[3,2-d]pyrimidin-4-yl)amino)phenyl)acetic acid (Example 21)

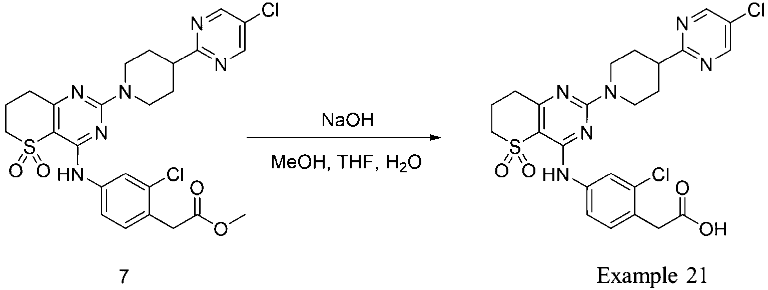

**Intermediate** 7 (270 mg, 0.48 mmol), methanol (3 mL), THF (3 mL), water (2.2 mL), and sodium hydroxide (89 mg, 2.23 mmol) were added in sequence to a 50 mL single-necked flask. The reaction was carried out at room temperature for 2 h under nitrogen protection. TLC (*V*_{dichloromethane} : *V*ₘₑₜₕₐₙₒₗ = 20:1) monitored that the starting materials have completely reacted. The pH was adjusted to 4 with diluted hydrochloric acid (1 M) in an ice bath, and a solid precipitated. The solid was filtered, the filter cake was rinsed with water (20 mL), collected and dried by forced air for 16 h to obtain 220 mg of white solid **(Example 21,** with a yield of 93.7%).

¹H NMR (400 MHz, DMSO-*d*₆) *δ* (ppm): 12.53 (s, 1H, COOH), 8.87 (s, 2H, ArH), 8.65 (s, 1H, ArH), 7.91 (s, 1H, NH), 7.34 (s, 2H, ArH), 4.70-4.55 (m, 2H, SO₂CH₂), 3.66 (s, 2H, COCH₂), 3.61-3.55 (m, 2H, NCH₂), 3.25-3.09 (m, 3H, NCH₂ and CH), 2.82 (t, J=6.4 Hz, 2H, SO₂CH₂CH₂CH₂), 2.31-2.25 (m, 2H, SO₂CH₂CH₂), 2.07-1.97 (m, 2H, CHCH₂), 1.70 (s, 2H, CHCH₂).

¹³C NMR (101 MHz, DMSO-*d*₆) *δ* (ppm): 172.18, 170.52, 167.34, 159.49, 156.18, 156.14, 138.49, 133.90, 132.48, 129.01, 128.86, 121.97, 120.23, 106.05, 51.36, 44.09, 43.92, 38.64, 31.92, 18.76.

HRMS (ESI): m/z [M+H]⁺ Calcd for C₂₄H₂₄Cl₂N₆O₄S: 563.1035; Found: 563.1025.

### Example 22

### Step 1 Synthesis of 3-ethyl 1-benzyl 2-(2,6-difluoro-4-nitrophenyl)malonate (3)

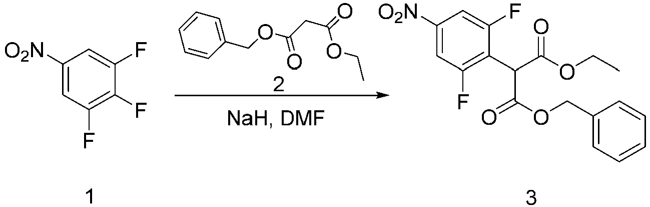

Sodium hydride (60%, 497 mg, 12.43 mmol) and DMF (10 mL) were added in sequence to a 50 mL reaction flask. Under nitrogen protection, a DMF (2 mL) solution of **intermediate 2** (2.51 g, 11.31 mmol) was added dropwise to the system in an ice bath. After the addition was complete, the reaction was kept at the temperature for 0.5 h. Then, a DMF (3 mL) solution of **compound 1** (1.00 g, 5.65 mmol) was added dropwise, and the reaction was carried out at 70°C for 1 h. TLC (*V*_{petroleum ether} : *V*_{ethyl acetate} = 5:1) monitored that the reaction was completed. The reaction solution was cooled to an ice bath, quenched with water (30 mL), and extracted with ethyl acetate (50 mL × 3), the organic phases were combined, washed with saturated brine (50 mL × 4). The organic phase was collected, dried over anhydrous sodium sulfate, filtered, concentrated and purified by flash preparative chromatography (40 g, *V*_{petroleum ether} : *V*_{ethyl acetate} = 5:1) to obtain 2.00 g of yellow oily **intermediate 3,** with a yield of 95.2%.

¹H NMR (400 MHz, CDCl₃) *δ* (ppm): 7.84 (s, 1H, ArH), 7.82 (s, 1H, ArH), 7.38-7.35 (m, 5H, ArH), 5.19 (s, 2H, ArCH₂), 5.07 (s, 1H, CH), 4.28-4.21 (q, J=7.2 Hz, 1H, CH₃CH₂), 1.25 (t, J=6.4 Hz, 3H, CH₃).

### Step 2 Synthesis of ethyl 2-(4-amino-2,6-difluorophenyl)acetate (4)

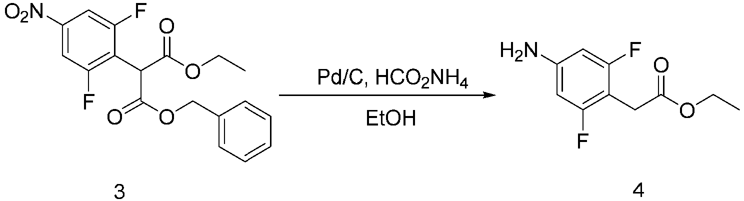

**Intermediate 3** (1.80 g, 4.75 mmol), ethanol (18 mL), ammonium formate (1.51 mg, 23.7 mmol), and Pd/C (10% wet, 270 mg, 0.15 w/w) were added in sequence to a 50 mL single-necked flask, and the reaction was carried out at 60°C for 3 h under nitrogen protection. TLC (*V*_{petroleum ether} : *V*_{ethyl acetate} = 3:1) monitored that the starting materials have completely reacted. The reaction solution was filtered through diatomaceous earth, the filter cake was rinsed with ethyl acetate (50 mL), the filtrate was collected, concentrated and purified by flash preparative chromatography (40 g, *V*_{petroleum ether} : *V*_{ethyl acetate} = 5:1) to obtain 530 mg of white solid **intermediate 4,** with a yield of 52.0%.

### Step 3 Synthesis of ethyl 2-(4-((2-chloro-7,8-dihydro- 6H -thiapyrano[3,2-d]pyrimidin-4-yl)amino)-2,6-difluorophenyl) acetate (5)

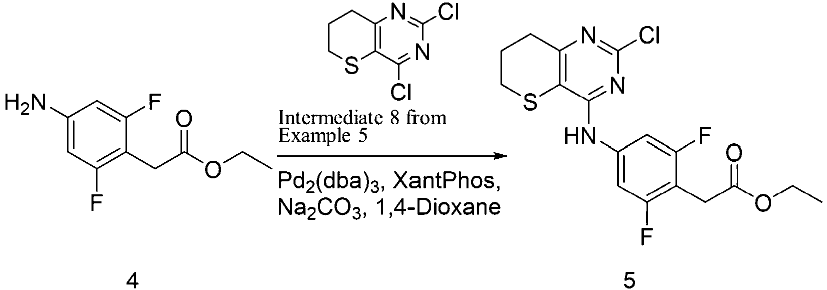

**Intermediate** 4 (530 mg, 2.63 mmol), 1,4-dioxane (10 mL), **Intermediate** 8 (699 mg, 3.16 mmol) from **Example 5,** sodium carbonate (558 mg, 5.26 mmol), Xantphos (228 mg, 0.40 mmol), and Pd₂(dba)₃ (180 mg, 0.20 mmol) were added in sequence to a 50 mL single-necked flask. The reaction was carried out at 70°C for 16 h under nitrogen protection. TLC (*V*_{ethyl acetate} : *V*_{petroleum ether} = 3 : 1) monitored that the starting materials have completely reacted. The reaction solution was filtered through diatomaceous earth, the filter cake was rinsed with ethyl acetate (100 mL), the filtrate was collected, concentrated and purified by flash preparative chromatography (40 g, *V*_{petroleum ether} : *V*_{ethyl acetate} = 3:1) to obtain 690 mg of white solid **intermediate 5,** with a yield of 54.2%.

¹H NMR (400 MHz, DMSO-*d*₆) *δ* (ppm): 8.84 (s, 1H, NH), 7.43 (d, *J*=9.7 Hz, 2H, ArH), 4.11 (q, *J*=7.2 Hz, 2H, CH₂CH₃), 3.68 (s, 2H, ArCH₂CO), 3.19-3.12 (m, 2H, SCH₂), 2.81 (t, *J*=6.3 Hz, 2H, SCH₂CH₂CH₂), 2.16-2.10 (m, 2H, SCH₂CH₂), 1.19 (t, *J*=7.0 Hz, 3H, CH₃).

### Step 4 Synthesis of ethyl 2-(4-((2-chloro-5,5-dioxo-7,8-dihydro- 6H - thiapyrano[3,2-d]pyrimidin-4-yl)amino)-2,6-difluorophenyl) acetate (6)

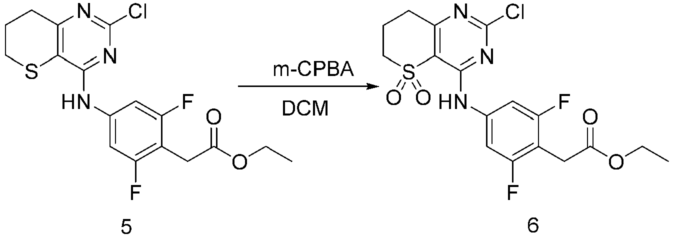

**Intermediate** 5 (690 mg, 1.73 mmol), DCM (20 mL), and m-chloroperoxybenzoic acid (85%, 869 mg, 4.32 mmol) were added in sequence to a 100 mL single-necked flask and were reacted at room temperature for 2 h under nitrogen protection. TLC (*V*_{ethyl acetate} : *V*_{petroleum ether} = 2 : 1) monitored that the starting materials have completely reacted. The reaction was quenched with saturated sodium thiosulfate (20 mL), f extracted with dichloromethane (50 mL × 3). The organic phases were combined, washed with saturated brine (50 mL × 3), collected, dried, filtered, concentrated, and purified by flash preparative chromatography (20 g, *V*_{dichloromethane} : *V*ₘₑₜₕₐₙₒₗ = 100: 1) to obtain 570 mg of white solid **intermediate 6,** with a yield of 76.5%.

### Step 5 Synthesis of ethyl 2-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2,6-difluorophenyl) acetate (7)

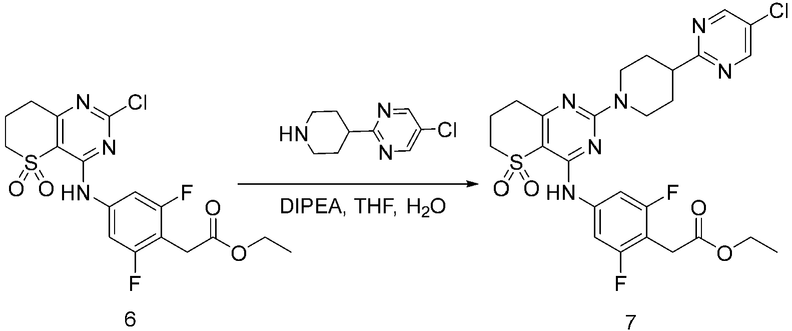

**Intermediate 6** (570 mg, 1.32 mmol), tetrahydrofuran (6 mL), water (1 mL), 5-chloro-2-(piperidin-4-yl)pyrimidine (287 mg, 1.45 mmol) and DIPEA (512 mg, 3.96 mmol) were added in sequence to a 25 mL single-necked flask, and the reaction was carried out at 65°C for 16 h under a nitrogen atmosphere. TLC (*V*_{dichloromethane} : *V*ₘₑₜₕₐₙₒₗ = 20:1) monitored that the starting materials have completely reacted. The reaction solution was cooled to room temperature, directly mixed, concentrated, and purified by flash preparative chromatography (20 g, *V*_{dichloromethane} : *V*ₘₑₜₕₐₙₒₗ = 100:1) to obtain 720 mg of white solid **intermediate 7,** with a yield of 92.1%.

¹H NMR (400 MHz, DMSO-*d*₆) *δ* (ppm): 8.87 (s, 2H, ArH), 8.75 (s, 1H, NH), 7.42 (d, J=9.6 Hz, 2H, ArH), 4.61 (br, 2H, SO₂CH₂), 4.10 (m, 2H, CH₂CH₃), 3.67 (s, 2H, COCH₂), 3.59-3.54 (m, 2H, NCH₂), 3.23-3.13 (m, 3H, NCH₂ and CH), 2.83 (t, J=6.4 Hz, 2H, SCH₂CH₂CH₂), 2.31-2.24 (m, 2H, SCH₂CH₂), 2.07-1.97 (m, 2H, CHCH₂), 1.73-1.63 (m, 2H, CHCH₂), 1.18 (t, J=7.2 Hz, 3H, CH₃).

### Step 6 Synthesis of 2-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2,6-difluorophenyl)acetic acid (Example 22)

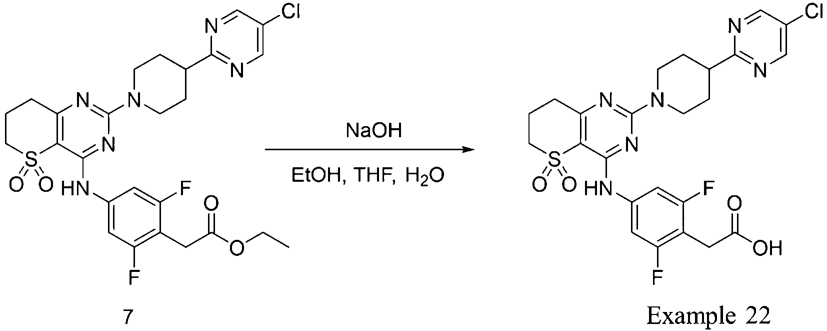

**Intermediate** 7 (300 mg, 0.51 mmol), ethanol (4 mL), THF (4 mL), water (2.2 mL), and sodium hydroxide (89 mg, 2.23 mmol) were added in sequence to a 50 mL single-necked flask. The reaction was carried out at room temperature for 2 h under nitrogen protection. TLC (*V*_{dichloromethane} : *V*ₘₑₜₕₐₙₒₗ = 20:1) monitored that the starting materials have completely reacted. The pH was adjusted to 4 with diluted hydrochloric acid (1 N) in an ice bath, and a solid precipitated. The solid was filtered, the filter cake was rinsed with water (20 mL), collected and dried by forced air for 16 h to obtain 250 mg of white solid Example 22, with a yield of 87.7%.

¹H NMR (400 MHz, DMSO-*d*₆) *δ* (ppm): 12.64 (s, 1H, COOH), 8.86 (d, J=2.4 Hz, 2H, ArH), 8.74 (s, 1H, NH), 7.40 (d, J=9.4 Hz, 2H, ArH), 4.61 (br, 2H, SO₂CH₂), 3.57 (s, 4H, NCH₂ and COCH₂), 3.25-3.14 (m, 3H, NCH₂ and CH), 2.83 (br, 2H, S CH₂CH₂CH₂), 2.31-2.26 (m, 2H, SCH₂CH₂), 2.05-2.01 (m, 2H, CHCH₂), 1.70 (s, 2H, CHCH₂).

¹³C NMR (101 MHz, DMSO-*d*₆) *δ* (ppm): 171.34, 170.50, 167.54, 161.03 (dd, *J*₁=244.4 Hz, *J*₂=11.0 Hz), 159.41, 156.13, 139.26 (t, J=14.2 Hz), 129.01, 106.34, 104.56 (d, J=29.8 Hz), 51.36, 44.06, 43.92, 31.93, 27.95, 18.71.

HRMS (ESI): m/z [M+H]⁺ Calcd for C₂₄H₂₃ClF₂N₆O₄S: 565.1236; Found: 565.1227.

### Example 23

### Step 1: Synthesis of 1-(2-fluorophenyl)cyclopropane-1-carboxynitrile (2)

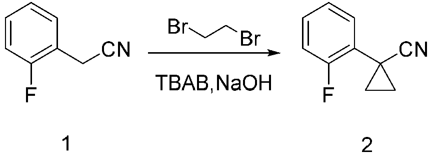

**Compound 1** (10.00 g, 74 mmol), 1,2-dibromoethane (27.82 g, 148 mmol), tetrabutylammonium bromide (2.38 g, 7.4 mmol), 50% NaOH (60 mL) and toluene (60 mL) were added to a 250 mL three-necked flask. After stirring well, the mixture was reacted overnight at room temperature under nitrogen protection. TLC (*V*_{petroleum ether} : *V*_{ethyl acetate} = 5:1) monitored that the starting materials have completely reacted. Water (50 mL) was added to the reaction solution, and the mixture was extracted with ethyl acetate (100 mL × 2). The organic phases were combined, washed with saturated brine (30 mL × 2), dried over anhydrous sodium sulfate, filtered, concentrated, and purified by flash preparative chromatography (120 g, *V*_{petroleum ether} : *V*_{ethyl acetate} = 10:1) to obtain 5.26 g of colorless oily **intermediate 2,** with a yield of 44.1%.

¹H NMR (400 MHz, CDCl₃) *δ* (ppm): 7.37-7.29 (m, 2H, ArH), 7.16-7.02 (m, 2H, ArH), 1.70-1.67 (m, 2H, cyclopropyl-H), 1.41-1.38 (m, 2H, cyclopropyl-H).

### Step 2 Synthesis of 1-(2-fluoro-4-nitrophenyl)cyclopropane-1-carboxynitrile (3)

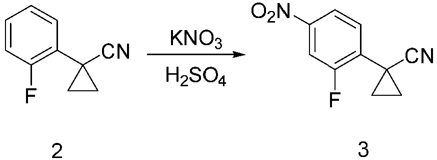

**Intermediate** 2 (4.50 g, 28.04 mmol) and sulfuric acid (25 mL) were added to a 250 mL single-necked flask, and then potassium nitrate (3.11 g, 30.84 mmol) was added in an ice-water bath under nitrogen protection and the reaction was carried out overnight at room temperature. TLC (*V*_{petroleum ether} : *V*_{ethyl acetate} = 2:1) monitored that the starting materials have completely reacted. Water (50 mL) was added to the reaction solution, and the mixture was extracted with ethyl acetate (100 mL × 2). The organic phases were combined, dried over anhydrous sodium sulfate, filtered, concentrated, and purified by flash preparative chromatography (40 g, *V*_{dichloromethane} : *V*ₘₑₜₕₐₙₒₗ = 50:1) to obtain 6.60 g of white solid **intermediate 3,** with a yield of 79.4%.

¹H NMR (400 MHz, DMSO-*d*₆) *δ* (ppm): 8.30-8.22 (m, 1H, ArH), 8.19 (dd, *J₁*=6.4 Hz, *J₂*=2.9 Hz, 1H, ArH), 7.48 (t, J=9.1 Hz, 1H, ArH), 1.48-1.41 (m, 2H, cyclopropyl-H), 1.10-1.07 (m, 2H, cyclopropyl-H).

### Step 3 Synthesis of ethyl 1-(2-fluoro-4-nitrophenyl)cyclopropane-1-carboxylate (4)

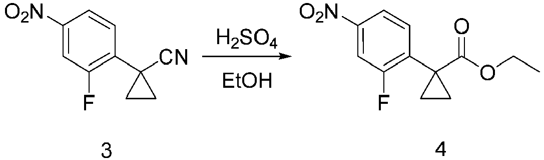

**Intermediate 3** (2.60 g, 12.61 mmol) and ethanol (26 mL) were added to a 250 mL single-necked flask, and then sulfuric acid (5.20 mL) was added under nitrogen protection. The reaction was refluxed overnight. TLC (*V*_{petroleum ether} : *V*_{ethyl acetate} = 5:1) monitored that the starting materials have completely reacted. Water (50 mL) was added to the reaction solution, and the mixture was extracted with ethyl acetate (150 mL × 2). The organic phases were combined, dried over anhydrous sodium sulfate, filtered, concentrated, and purified by flash preparative chromatography (40 g, *V*_{petroleum ether} : *V*_{ethyl acetate} = 10:1) to obtain 2.50 g of colorless oily **intermediate 4,** with a yield of 78.3%.

¹H NMR (400 MHz, CDCl₃) *δ* (ppm): 8.26-8.12 (m, 2H, ArH) 7.19 (t, *J*=8.9 Hz, 1H, ArH), 4.12 (q, *J*=7.1 Hz, 2H, C**H₂**), 1.76-1.73 (m, 2H, cyclopropyl-H), 1.25-1.22 (m, 2H, cyclopropyl-H), 1.17 (t, *J*=7.1 Hz, 3H, C**H₃**).

### Step 4 Synthesis of ethyl 1-(4-amino-2-fluorophenyl)cyclopropane-1-carboxylate (5)

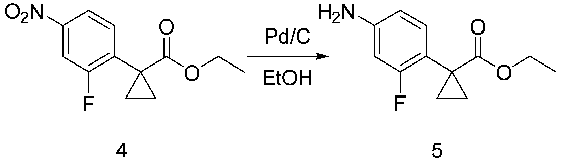

**Intermediate 4** (2.00 g, 7.89 mmol), anhydrous ethanol (40 mL), and 10% wet palladium on carbon (400 mg, 20% wt) were added to a 100 mL single-necked flask. The reaction was carried out overnight at 60°C under a hydrogen pressure of 15 psi. TLC (*V*_{petroleum ether} : *V*_{ethyl acetate} = 3:1) monitored that the starting materials have completely reacted. The reaction solution was diluted with ethyl acetate (50 mL), filtered through diatomaceous earth, concentrated and purified by flash preparative chromatography (20 g, *V*_{petroleum ether} : *V*_{ethyl acetate} = 5:1) to obtain 1.90 g of colorless oily **intermediate 5,** with a yield of 86.3%.

¹H NMR (400 MHz, CDCl₃) *δ* (ppm): 6.83 (t, *J*=8.9 Hz, 1H, Ar**H**), 6.60-6.51 (m, 2H, Ar**H**), 4.10 (q, *J*=7.1 Hz, 2H, C**H₂**), 1.62-1.59 (m, 2H, cyclopropyl-H), 1.20-1.13 (m, 5H, cyclopropyl-H and C**H₃**).

### Step 5 Synthesis of ethyl 1-(4-((2-chloro-7,8-dihydro-6H-thiapyrano[3,2-d]pyrimidin-4-yl)amino)-2-fluorophenyl)cyclopropane-1-carboxylate (6)

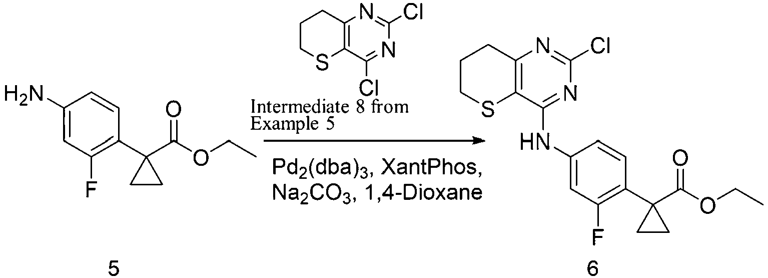

**Intermediate 5** (1.00 g, 4.47 mmol), **Intermediate 8 from Example 5** (1.18 g, 5.37 mmol), XantPhos (388 mg, 0.67 mmol), sodium carbonate (949 mg, 8.96 mmol) and 1,4-dioxane (12 mL) were added in sequence to a 50 mL single-necked flask. Under nitrogen protection, tris(dibenzylideneacetone)dipalladium (307 mg, 0.34 mmol) was added, and the reaction was carried out overnight at 75°C. TLC (*V*_{dichloromethane} : *V*ₘₑₜₕₐₙₒₗ = 20:1) monitored that the starting materials have completely reacted. Water (100 mL) was added to the reaction solution, and the mixture was extracted with ethyl acetate (200 mL × 2). The organic phases were combined, washed with saturated brine (30 mL), dried over anhydrous sodium sulfate, filtered, concentrated, and purified by flash preparative chromatography (20 g, *V*_{petroleum ether} : *V*_{ethyl acetate} = 5:1) to obtain 850 mg of white solid **intermediate 6,** with a yield of 46.6%.

¹H NMR (400 MHz, CDCl₃) *δ* (ppm): 7.53-7.46 (m, 1H, Ar**H**), 7.41 (dd, *J*₁=6.4 Hz, *J*₂=2.8 Hz, 1H, Ar**H**), 7.03 (t, *J*=9.1 Hz, 1H, Ar**H**), 6.56 (s, 1H, N**H**), 4.11 (q, *J*=7.1 Hz, 2H, C**H₂**CH₃), 3.21-3.05 (m, 2H, SC**H₂**), 2.88 (t, *J*=6.4 Hz, 2H, NCC**H₂**), 2.31-2.21 (m, 2H, SCH₂C**H₂**), 1.67-1.65 (m, 2H, cyclopropyl-H), 1.23-1.19 (m, 2H, cyclopropyl-H), 1.17 (t, *J*=7.1 Hz, 3H, C**H₃**).

### Step 6 Synthesis of ethyl 1-(4-((2-chloro-5,5-dioxo-7,8-dihydro- 6H - thiapyrano[3,2-d]pyrimidin-4-yl)amino)-2-fluorophenyl)cyclopropane-1-carboxylate (7)

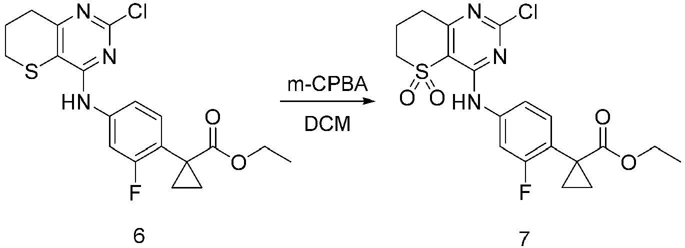

**Intermediate** 6 (850 mg, 2.08 mmol) and dichloromethane (12 mL) were added to a 50 mL single-necked flask. Under nitrogen protection, m-chloroperoxybenzoic acid (1.06 g, 5.2 mmol) was added in two batches and the reaction was allowed to proceed overnight at room temperature. TLC (*V*_{petroleum ether} : *V*_{ethyl acetate} = 1:1) monitored that the starting materials have completely reacted. 20 mL of saturated sodium thiosulfate solution was added to the reaction solution and stirred for 10 min. After extracting with dichloromethane (100 mL × 2), the organic phases were combined, washed with 30 mL of saturated brine, dried with anhydrous sodium sulfate, filtered, concentrated and purified by flash preparative chromatography (12 g, *V*_{petroleum ether} : *V*_{ethyl} acetate = 2:1) to obtain 800 mg of white solid **intermediate** 7, with a yield of 87.4%.

### Step 7 Synthesis of ethyl 1-(4-((2-(4-(5-chloropyrimidin-2-yl)piperidin-1-yl)-5,5-dioxo-7,8-dihydro- 6H-thiapyrano[3,2-d]pyrimidin-4-yl)amino)-2-fluorophenyl)cyclopropane-1-carboxylate (8)

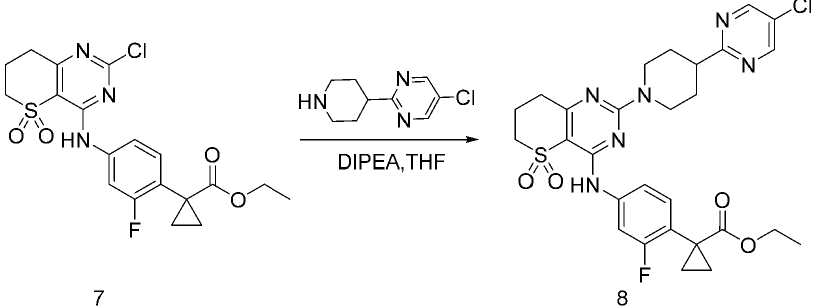

**Intermediate** 7 (300 mg, 0.68 mmol), tetrahydrofuran (5 mL), water (1 mL), 5-chloro-2-(piperidin-1-yl)pyrimidine (148 mg, 0.75 mmol) and *N,N -* diisopropylethylamine (264 mg, 2.05 mmol) were added in sequence to a 50 mL single-necked flask, and the reaction was carried out overnight at 65°C under nitrogen protection. TLC (*V*_{dichloromethane} : *V*ₘₑₜₕₐₙₒₗ = 40:1) monitored that the starting materials have completely reacted. Water (50 mL) was added to the reaction solution, and the mixture was extracted with ethyl acetate (100 mL × 2). The organic phases were combined, washed with saturated brine (30 mL), dried over anhydrous sodium sulfate, filtered, concentrated, and purified by flash preparative chromatography (12 g, *V*_{petroleum ether} : *V*_{ethyl acetate} = 1:2) to obtain 400 mg of white solid **intermediate 8,** with a yield of 97.7%.

¹H NMR (400 MHz, CDCl₃) *δ* (ppm):8.62 (s, 2H, ArH), 8.43 (s, 1H, ArH), 7.51 (dd, *J₁*=6.5 Hz, *J₂*=2.7 Hz, 1H, ArH), 7.36-7.32 (m, 1H, ArH), 6.98 (t, J=9.1 Hz, 1H, NH), 4.11 (q, J=7.1 Hz, 2H, CH₂CH₃), 3.40-3.32 (m, 2H, SCH₂), 3.19-3.12 (m, 1H, CH), 3.02 (t, *J=12.8* Hz, 2H, NCH₂), 2.84 (t, *J=6.5* Hz, 2H, NCH₂), 2.46-2.43 (m, 2H, NCCH₂), 2.06-2.03 (m, 2H, SCH₂CH₂) 1.90-1.75 (m, 2H, CHCH₂), 1.63-1.61 (m, 2H, CHCH₂), 1.50-1.43 (m, 2H, cyclopropyl-H) 1.18-1.12 (m, 5H, CH₃ and cyclopropyl-H).

### Step 8 Synthesis of 1-(4-((2-(4-(5-chloropyrimidin-2-yl)piperidin-1-yl)-5,5-dioxo-7,8-dihydro- 6H-thiapyrano[3,2-d]pyrimidin-4-yl)amino)-2-fluorophenyl)cyclopropane-1-carboxylic acid (Example 23)

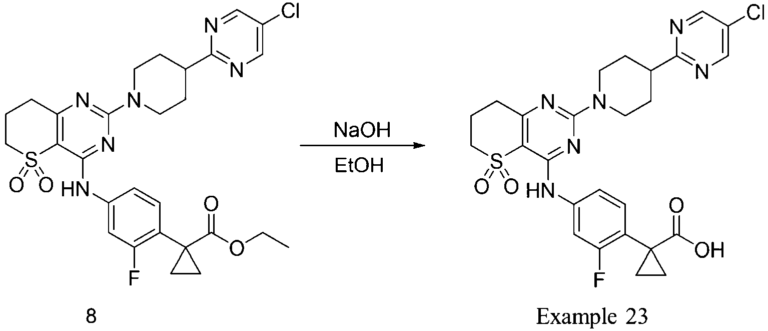

**Intermediate** 8 (300 mg, 0.50 mmol), sodium hydroxide (120 mg, 3 mmol), water (3 mL), and ethanol (3 mL) were added to a 50 mL single-necked flask in sequence, and the reaction was carried out overnight at 35°C under nitrogen protection. TLC (*V*_{dichloromethane} : *V*ₘₑₜₕₐₙₒₗ = 20:1) monitored that t the starting materials have completely reacted. Water (5 mL) was added to the reaction solution, and the pH was adjusted to 5 with 1 M hydrochloric acid. The solution was extracted with dichloromethane (50 mL × 2), the organic phases were combined, washed with saturated brine (30 mL), dried over anhydrous sodium sulfate, filtered, concentrated, and purified by flash preparative chromatography (20 g, *V*_{petroleum ether} : *V*_{ethyl acetate} = 1:2) to obtain 38 mg of pale yellow solid **Example 23,** with a yield of 13.3%.

¹H NMR (400 MHz, CDCl₃) *δ* (ppm): 8.63 (s, 2H, ArH), 8.41 (s, 1H, ArH), 7.54 (dd, *J₁*=6.4 Hz, *J₂*=2.7 Hz, 1H, ArH), 7.35-7.31 (m, 1H, ArH), 6.98 (t, *J*=9.1 Hz, 1H, NH), 4.78 (br, 2H, SCH₂), 3.39-3.32 (m, 2H, NCCH₂), 3.19-3.12 (m, 1H, CH), 3.01 (t, *J=11.7* Hz, 2H, NCH₂), 2.83 (t, *J=6.5* Hz, 2H, NCH₂), 2.48-2.40 (m, 2H SCH₂CH₂), 2.05-2.02 (m, 2H, CHCH₂), 1.82-1.78 (m, 2H, CHCH₂), 1.70-1.67 (m, 2H, cyclopropyl-H), 1.26-1.24 (m, 2H, cyclopropyl-H).

¹³C NMR (101 MHz, DMSO-*d*₆) *δ* (ppm) 174.82, 170.58, 167.02, 159.49, 158.67 (d, *J*=244.42 Hz), 156.35, 156.12, 134.09 (d, *J*=2.0 Hz), 128.99, 127.69 (d, *J*=15.2 Hz), 126 (d, *J*=4.0 Hz), 122.65 (d, *J*=8.08 Hz), 115.51 (d, *J*=23.23 Hz), 105.76, 51.46, 44.20, 43.74, 31.92, 30.66, 24.03, 18.80, 15.78.

HRMS (ESI): m/z [M+H]⁺ Calcd for C₂₆H₂₆ClFN₆O₄S: 573.1487; Found: 573.1454

### Example 24

### Step 1: Synthesis of 2-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2-fluorophenyl) -N-neopentylacetamide (Example 24)

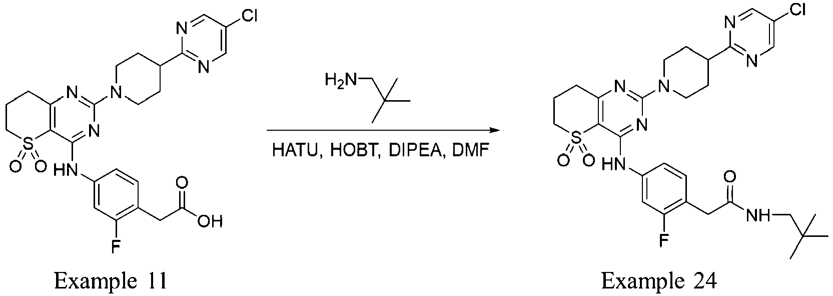

**Example 11** (300 mg, 0.55 mmol), anhydrous DMF (12 mL), HATU (316 mg, 0.83 mmol), HOBT (112 mg, 0.83 mmol), DIPEA (213 mg, 1.65 mmol), and neopentylamine (72 mg, 0.83 mmol) were added to a 100 mL single-necked flask in sequence. The reaction was carried out at room temperature for 15 h under nitrogen protection. TLC (*V*_{dichloromethane} : *V*ₘₑₜₕₐₙₒₗ = 30: 1) monitored that the reaction was completed. The reaction system was quenched with water (50 mL), extracted with ethyl acetate (50 mL × 3), and the organic phase was collected. The organic phase was washed with saturated sodium chloride aqueous solution (50 mL × 3), dried over anhydrous sodium sulfate, filtered, concentrated and purified by flash preparative chromatography (12 g, *V*_{petroleum ether} : *V*_{ethyl} acetate = 1: 1) to obtain 208 mg of white solid **Example 24,** with a yield of 61.4%.

¹H NMR (400MHz, DMSO-*d*₆) *δ* (ppm): 8.87 (br, 2H, 2NH), 8.64 (s, 1H, ArH), 7.95 (t, J=6.3 Hz, 1H, ArH), 7.60-7.50 (m, 1H, ArH), 7.28 (t, *J*=8.4 Hz, 1H, ArH), 7.24-7.16 (m, 1H, ArH), 4.87-4.37 (m, 2H, NCH₂), 3.61-3.52 (m, 2H, NCH₂), 3.47 (s, 2H, H of ArCH₂CO), 3.25-3.05 (m, 3H, CH and SO₂CH₂), 2.89 (d, *J*=6.2 Hz, 2H, NHCH₂), 2.81 (t, *J*=6.4 Hz, 2H, SO₂CH₂CH₂CH₂), 2.34-2.22 (m, 2H, SO₂CH₂CH₂CH₂), 2.07-1.96 (m, 2H, CHCH₂), 1.79-1.57 (m, 2H, CHCH₂), 0.81 (s, 9H, 3CH₃).

¹³C NMR (101 MHz, DMSO-*d*₆) *δ* (ppm): 170.09, 169.24, 166.80, 160.13 (d, *J*_{C-F}=243.4 Hz), 159.09, 155.78, 155.68, 138.03 (d, *J*_{C-F}=11.1 Hz), 131.55 (d, *J*_{C-F}=6.1 Hz), 128.55, 118.66 (d, *J*_{C-F}=16.2 Hz), 116.76 (d, *J*_{C-F}=2.0 Hz), 108.14 (d, *J*_{C-F}=27.3 Hz), 105.54, 50.90, 49.74, 43.65, 43.42, 34.99, 32.03, 31.44, 30.16, 27.21, 18.31.

HRMS (ESI): m/z [M+H]⁺ Calcd for C₂₉H₃₅ClFN₇O₃S: 616.2273; Found: 616.2241.

### Example 25

### Step 1 Synthesis of dimethyl 2-(2-fluoro-4-nitrophenyl)malonate (2)

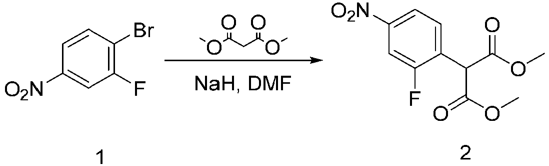

Sodium hydride (60%, 10.00 g, 250.00 mmol) and anhydrous DMF (250 mL) were added to a 1 L single-necked flask, and cooled to 0°C, and dimethyl malonate (30.03 g, 227.28 mmol) was added dropwise. Under nitrogen protection, the reaction was carried out at 0°C for 30 min. Then, an anhydrous DMF (120 mL) solution of **compound 1** (25.00 g, 113.64 mmol) was added dropwise, and the reaction was carried out at 70°C for 17 h. TLC (*V*_{petroleum ether} : *V*_{ethyl acetate} = 4 : 1) monitored that the reaction was completed. The reaction system was quenched with saturated ammonium chloride aqueous solution (1 L), extracted with ethyl acetate (1 L × 3). The organic phase was collected, washed with saturated sodium chloride aqueous solution (1 L × 3), dried over anhydrous sodium sulfate, filtered, concentrated, and purified by column chromatography (*V*_{petroleum ether} : *V*_{ethyl acetate} = 4: 1) to obtain 30.60 g of yellow solid **intermediate 2,** with a yield of 99.3%.

### Step 2 Synthesis of methyl 2-(2-fluoro-4-nitrophenyl)acetate (3)

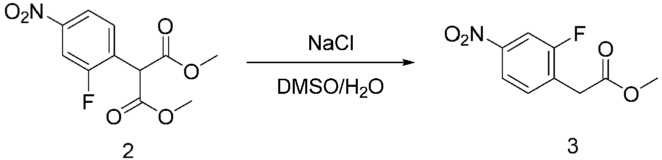

**Intermediate** 2 (30.59 g, 112.79 mmol), DMSO (210 mL), sodium chloride (6.59 g, 112.79 mmol) and water (4.2 mL) were added in sequence to a 500 mL single-necked flask. The reaction was carried out at 120°C for 4 h under nitrogen protection. TLC (*V*_{petroleum ether} : *V*_{ethyl acetate} = 10: 1) monitored that the reaction was completed. The reaction system was quenched with water (500 mL), extracted with ethyl acetate (500 mL × 3), and the organic phases were collected. The organic phase was washed with saturated sodium chloride aqueous solution (500 mL × 3), dried over anhydrous sodium sulfate, filtered, concentrated and purified by column chromatography (*V*_{petroleum ether} : *V*_{ethyl acetate} = 10: 1) to obtain 8.13 g of yellow oily **intermediate** 3, with a yield of 33.8%.

### Step 3 Synthesis of methyl 1-(2-fluoro-4-nitrophenyl)cyclobutane-1-carboxylate (4)

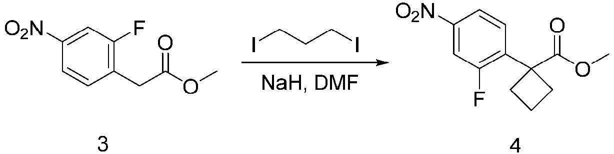

Sodium hydride (60%, 4.95 g, 123.86 mmol) and anhydrous DMF (230 mL) were added to a 500 mL single-necked flask. **Intermediate** 3 (12.00 g, 56.30 mmol) was added in an ice bath. The reaction was carried out at 0°C for 0.5 h. Then 1,3-diiodopropane (19.99 g, 67.56 mmol) was added. The reaction was carried out at 0°C for 4 h under nitrogen protection. TLC (*V*_{petroleum ether} : *V*_{ethyl acetate} = 10 : 1) monitored that the reaction was completed. The reaction system was quenched by adding saturated ammonium chloride aqueous solution (500 mL), and extracted with ethyl acetate (500 mL × 3), and the organic phase was collected. The organic phase was washed with saturated sodium chloride aqueous solution (500 mL × 3), dried over anhydrous sodium sulfate, filtered, concentrated and purified by flash preparative chromatography (120 g × 2, *V*_{petroleum ether} : *V*_{ethyl acetate} = 9: 1) to obtain 8.53 g of yellow oily **intermediate 4,** with a yield of 59.8%.

¹H NMR (400MHz, CDCl₃) *δ* (ppm): 8.07-8.02 (m, 1H, ArH), 7.92-7.85 (m, 1H, ArH), 7.49-7.38 (m, 1H, ArH), 3.69 (s, 3H, CH₃), 2.91-2.83 (m, 2H, CCH₂), 2.59-2.50 (m, 2H, CCH₂), 2.39-2.23 (m, 1H, H of CCH₂CH₂), 2.02-1.87 (m, 1H, H of CCH₂CH₂).

### Step 4 Synthesis of methyl 1-(4-amino-2-fluorophenyl)cyclobutane-1-carboxylate (5)

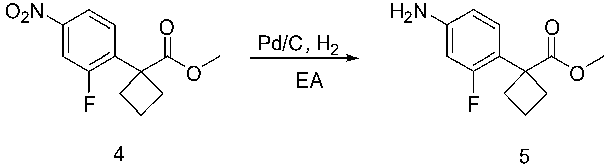

**Intermediate 4** (2.53 g, 9.99 mmol), ethyl acetate (85 mL), and 10% Pd/C (13 wt%, 0.33 g) were added in sequence to a 250 mL single-necked flask, and the reaction was carried out at room temperature for 15 h under a hydrogen atmosphere. TLC (*V*_{petroleum ether} : *V*_{ethyl acetate} = 3 : 1) monitored that the reaction was completed. The mixture was filtered, concentrated, and purified by flash preparative chromatography (40 g, *V*_{petroleum ether} : *V*_{ethyl acetate} = 4:1) to obtain 1.40 g of yellow oily **intermediate 5,** with a yield of 62.6%.

¹H NMR (400MHz, DMSO-*d*₆) *δ* (ppm): 6.95 (t, J=8.8 Hz, 1H, ArH), 6.34 (d, J=8.3 Hz, 1H, ArH), 6.25 (d, J=13.4 Hz, 1H, ArH), 5.29 (s, 2H, NH₂), 3.56 (s, 3H, CH₃), 2.66-2.55 (m, 2H, CCH₂), 2.43-2.29 (m, 2H, CCH₂), 2.07-1.92 (m, 1H, H of CCH₂CH₂), 1.90-1.74 (m, 1H, H of CCH₂CH₂).

HRMS (ESI): m/z [M+H]⁺ Calcd for C₁₂H₁₄FNO₂: 224.1087; Found: 224.1051.

### Step 5 Synthesis of methyl 1-(4-((2-chloro-7,8-dihydro- 6H -thiapyrano[3,2-d]pyrimidin-4-yl)amino)-2-fluorophenyl)cyclobutane-1-carboxylate (6)

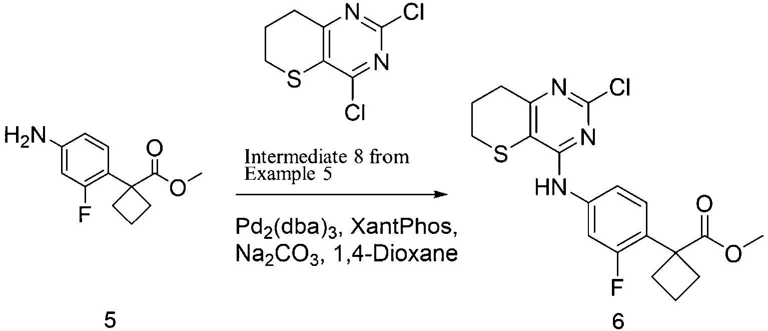

**Intermediate** 5 (1.89 g, 8.47 mmol), anhydrous 1,4-dioxane (28 mL), **intermediate 8** (2.25 g, 10.16 mmol) from **Example 5,** Pd₂(dba)₃ (590 mg, 0.64 mmol), XantPhos (730 mg, 1.27 mmol), and sodium carbonate (1.80 g, 16.94 mmol) were added in sequence to a 100 mL single-necked flask. The reaction was carried out at 75°C for 16 h under nitrogen protection. TLC (*V*_{petroleum ether} : *V*_{ethyl acetate} = 2: 1) monitored that the reaction was completed, and the reaction solution was cooled to room temperature. The mixture was filtered, concentrated, and purified by flash preparative chromatography (80 g, *V*_{petroleum ether} : *V*_{ethyl acetate} = 3:1) to obtain 5.81 g of yellow solid **intermediate 6,** with a yield of 67.5%.

¹H NMR (400MHz, DMSO-*d*₆) *δ* (ppm): 8.73 (s, 1H, NH), 7.48-7.27 (m, 3H, ArH), 3.60 (s, 3H, CH₃), 3.20-3.08 (m, 2H, SCH₂), 2.84-2.76 (m, 2H, SCH₂CH₂CH₂), 2.75-2.61 (m, 2H, SCH₂CH₂CH₂), 2.49-2.41 (m, 2H, CCH₂), 2.20-2.01 (m, 3H, CCH₂, H of CCH₂CH₂), 1.95-1.81 (m, 1H, H of CCH₂CH₂).

HRMS (ESI): m/z [M+H]⁺ Calcd for C₁₉H₁₉ClFN₃O₂S: 408.0949; Found: 408.0912.

### Step 6 Synthesis of methyl 1-(4-(((2-chloro-5,5-dioxo-7,8-dihydro- 6H - thiapyrano[3,2-d]pyrimidin-4-yl)amino)-2-fluorophenyl)cyclobutane-1-carboxylate (7)

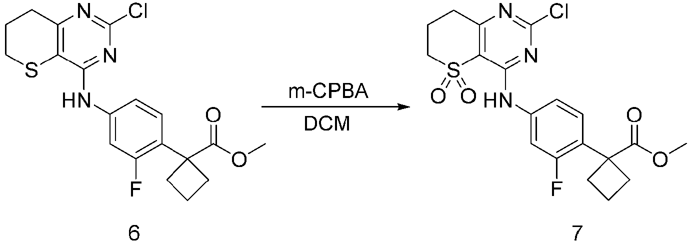

**Intermediate 6** (2.90 g, 7.11 mmol), m-CPBA (85%, 3.61 g, 17.78 mmol), and dichloromethane (87 mL) were added in sequence to a 250 mL single-necked flask. The reaction was carried out at room temperature for 2 h under N₂ protection. TLC (*V*_{petroleum ether} : *V*_{ethyl acetate} = 1 : 1) monitored that the reaction was completed. The reaction system was quenched by adding saturated sodium thiosulfate aqueous solution (200 mL), and extracted with dichloromethane (200 mL × 3) to collect the organic phase. The organic phase was washed with saturated sodium bicarbonate aqueous solution (200 mL × 3), dried over anhydrous sodium sulfate, filtered, concentrated and purified by flash preparative chromatography (40 g, *V*_{petroleum ether} : *V*_{ethyl acetate} = 1: 1) to obtain 2.86 g of yellow solid **intermediate 7,** with a yield of 91.4%.

¹H NMR (400MHz, DMSO-*d*₆) *δ* (ppm): 9.10 (s, 1H, NH), 7.50-7.36 (m, 2H, ArH), 7.35-7.29 (m, 1H, ArH), 3.75-3.67 (m, 2H, SCH₂), 3.61 (s, 3H, CH₃), 2.98 (t, *J*=6.3 Hz, 2H, SO₂CH₂CH₂), 2.75-2.64 (m, 2H, SO₂CH₂CH₂), 2.52 (d, *J*=8.2 Hz, 1H, H of CCH₂), 2.47 (d, *J*=9.4 Hz, 1H, H of CCH₂), 2.39-2.28 (m, 2H, CCH₂), 2.16-2.02 (m, 1H, H of CCH₂CH₂), 1.95-1.81 (m, 1H, H of CCH₂CH₂).

### Step 7 Synthesis of methyl 1-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2-fluorophenyl)cyclobutane-1-carboxylate (8)

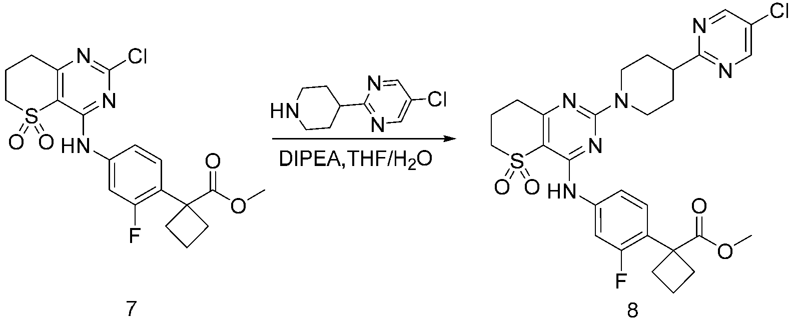

**Intermediate 7** (500 mg, 0.29 mmol), 5-chloro-2-(piperidin-4-yl)pyrimidine (247 mg, 1.25 mmol), tetrahydrofuran (8.8 mL), water (1.8 mL), and DIPEA (442 mg, 3.42 mmol) were added in sequence to a 25 mL single-necked flask. The reaction was carried out at 65°C for 16 h under nitrogen protection. TLC (*V*_{dichloromethane} : *V*ₘₑₜₕₐₙₒₗ = 50: 1) monitored that the reaction was completed. The mixture was concentrated and purified by flash preparative chromatography (12 g, *V*_{dichloromethane} : *V*ₘₑₜₕₐₙₒₗ = 50: 1) to obtain 644 mg of white solid **intermediate 8,** with a yield of 94.0%.

¹H NMR (400MHz, DMSO-*d*₆) *δ* (ppm): 8.87 (s, 2H, ArH), 8.68 (s, 1H, NH), 7.57 (d, *J*=13.2 Hz, 1H, ArH), 7.42-7.07 (m, 2H, ArH), 4.95-4.28 (m, 2H, SO₂CH₂), 3.67-3.48 (m, 5H, NCH₂ and CH₃), 3.24-3.10 (m, 3H, NCH₂ and CH), 2.92-2.77 (m, 2H, SO₂CH₂CH₂CH₂), 2.49-2.38 (m, 2H, SO₂CH₂CH₂CH₂), 2.36-2.22 (m, 2H, CCH₂), 2.09-2.02 (m, 3H, CCH₂, H of CHCH₂), 1.93-1.81 (m, 1H, H of CHCH₂), 1.80-1.58 (m, 2H, CHCH₂), 1.33-1.22 (m, 2H, CCH₂CH₂).

### Step 8 Synthesis of 1-(4-((2-(4-(5-chloropyrimidin-2-yl)piperidin-1-yl)-5,5-dioxo-7,8-dihydro- 6H -thiapyrano[3,2-d]pyrimidin-4-yl)amino)-2-fluorophenyl)cyclobutane-1-carboxylic acid (Example 25)

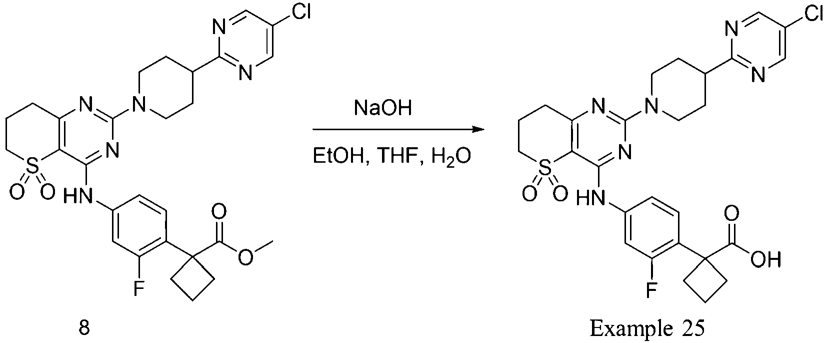

**Intermediate 8** (344 mg, 0.57 mmol), ethanol (6 mL), tetrahydrofuran (12 mL), water (6 mL), and sodium hydroxide (114 mg, 2.85 mmol) were added in sequence to a 50 mL single-necked flask. The reaction was carried out at 35°C for 1.5 h under nitrogen protection. TLC (*V*_{dichloromethane} : *V*ₘₑₜₕₐₙₒₗ = 50: 1) monitored that approximately 10% of the starting material was remained. Under ice bath conditions, the pH was adjusted to 7 with 1 N diluted hydrochloric acid, and the organic solvent was removed by concentration. The pH was adjusted to 3 with 1 N diluted hydrochloric acid, the filter cake was collected after filtration, and was then purified by a thick preparative plate (*V*_{dichloromethane} : *V*ₘₑₜₕₐₙₒₗ = 19: 1) to obtain 71 mg of white solid **Example 25,** with a yield of 21.3%.

¹H NMR (400MHz, DMSO-*d*₆) *δ* (ppm): 12.37 (s, 1H, COOH), 8.87 (s, 2H, ArH), 8.67 (s, 1H, NH), 7.60-7.51 (m, 1H, ArH), 7.35-7.22 (m, 2H, ArH), 4.63 (br, 2H, SO₂CH₂), 3.59-3.52 (m, 2H, NCH₂), 3.27-3.09 (m, 3H, CH and NCH₂), 2.82 (t, *J*=6.4 Hz, 2H, SO₂CH₂CH₂CH₂), 2.71-2.61 (m, 2H, SO₂CH₂CH₂CH₂), 2.47-2.38 (m, 2H, CCH₂), 2.33-2.23 (m, 2H, CCH₂), 2.15-1.99 (m, 3H, H of CHCH₂ and CHCH₂), 1.88-1.80 (m, 1H, H of CHCH₂), 1.77-1.66 (m, 2H, CCH₂CH₂).

¹³C NMR (101 MHz, DMSO-*d*₆) *δ* (ppm): 175.87, 170.10, 166.83, 159.82 (d, *J*_{C-F}=244.4 Hz), 159.10, 155.72, 155.67, 138.22 (d, *J*_{C-F}=11.1 Hz), 128.56, 128.20 (d, *J*_{C-F}=6.1 Hz), 126.07 (d, *J*_{C-F}=15.2 Hz), 116.22 (d, *J*_{C-F}=3.0 Hz), 108.15 (d, *J*_{C-F}=27.3 Hz), 105.60, 50.90, 48.37, 43.65, 43.49, 31.46, 31.06, 30.18, 18.31, 16.83.

HRMS (ESI): m/z [M+H]⁺ Calcd for C₂₇H₂₈ClFN₆O₄S: 587.1643; Found: 587.1639.

### Example 26

### Step 1 Synthesis of methyl 1-(4-((2-(4-(5-chloropyrimidin-2-yl)-3,6-dihydropyridin-1(2H)-yl)-5,5-dioxo-7,8-dihydro- 6H -thiapyrano[3,2-d]pyrimidin-4-yl)amino)-2-fluorophenyl)cyclobutane-1-carboxylate (2)

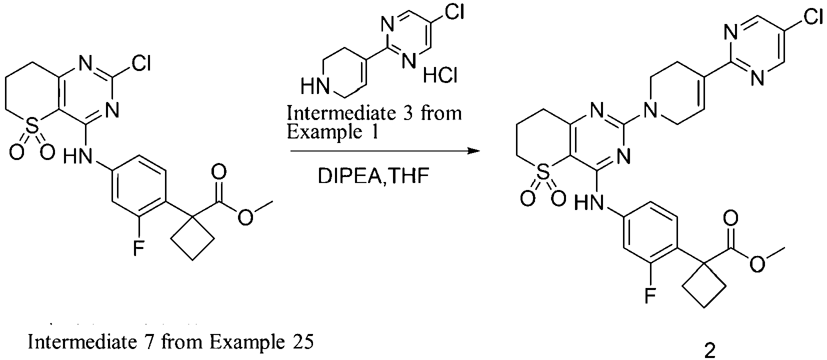

**Intermediate 7** (210 mg, 0.53 mmol) from **Example 25, Intermediate 3** (114 mg, 0.58 mmol)from **Example 1,** DIPEA (205 g, 1.59 mmol), THF (4 mL), and H₂O (1 mL) were added in sequence to a 25 mL single-necked flask, and the mixture was reacted overnight at 65°C under nitrogen protection. TLC (*V*_{dichloromethane} : *V*ₘₑₜₕₐₙₒₗ = 30:1) monitored that the reaction was completed. The mixture was concentrated and slurried with tetrahydrofuran (1 mL) to obtain 130 mg of white solid **intermediate 2,** with a yield of 87.6%.

¹H NMR (400 MHz, DMSO-*d*₆) *δ* (ppm): 8.91 (s, 2H, ArH), 8.72 (s, 1H, NH), 7.60 (d, *J*=13.0 Hz, 1H, ArH), 7.39 (d, *J*=5.2 Hz, 2H, ArH), 7.30 (s, 1H, C=CH), 4.48 (br, 2H, SO₂CH₂), 3.99 (br, 2H, NCH₂), 3.64-3.57 (m, 3H, NCH₂, H of NCH₂CH₂), 3.62 (s, 3H, CH₃), 3.16-3.11 (m, 1H, H of NCH₂CH₂), 2.86 (t, *J*=6.5 Hz, 2H, SO₂CH₂CH₂CH₂), 2.74-2.67 (m, 4H, CH₂CH₂CH₂), 2.33-2.27 (m, 2H, SO₂CH₂CH₂CH₂), 2.14-2.07 (m, 1H, H of CH₂CH₂CH₂), 1.94-1.86 (m, 1H, H of CH₂CH₂CH₂).

### Step 2 Synthesis of 1-(4-((2-(4-(5-chloropyrimidin-2-yl)-3,6-dihydropyridin-1(2H)-yl)-5,5-dioxo-7,8-dihydro- 6H -thiapyrano[3,2-d]pyrimidin-4-yl)amino)-2-fluorophenyl)cyclobutane-1-carboxylic acid (Example 26)

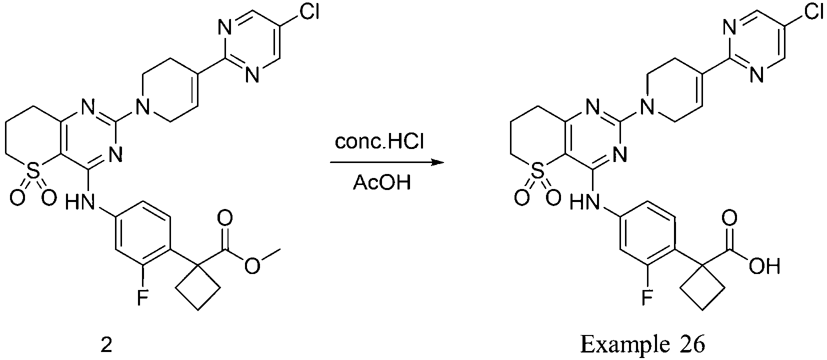

**Intermediate** 2 (280 mg, 0.47 mmol), acetic acid (5 mL), and concentrated hydrochloric acid (2.5 mL) were added in sequence to a 25 mL single-necked flask. The mixture was then reacted at 90°C for 40 min under nitrogen protection. TLC (*V*_{dichloromethane} :*V*ₘₑₜₕₐₙₒₗ = 15:1) monitored that the reaction was completed. The mixture was concentrated to 4 mL, and ice water (8 mL) was added to the residue. The mixture was stirred at room temperature for 1 h. After filtration, the filter cake was rinsed with water (2 mL × 3) , collected, and purified by preparative plate (*V*_{dichloromethane} : *V*ₘₑₜₕₐₙₒₗ = 20:1) for three times to obtain 49 mg of white solid **Example 26,** with a yield of 17.8%.

¹H NMR (400 MHz, DMSO-*d*₆) *δ* (ppm): 12.39 (s, 1H, COOH), 8.90 (s, 2H, ArH), 8.70 (s, 1H, NH), 7.58 (d, *J*=13.0 Hz, 1H, ArH), 7.34-7.33 (m, 2H, ArH), 7.31-7.28 (m, 1H, C=CH), 4.47 (br, 2H, SO₂CH₂CH₂CH₂), 3.98 (br, 2H, NCH₂), 3.59-3.56 (m, 2H, NCH₂CH₂), 2.85 (t, *J*=6.4 Hz, 2H, SO₂CH₂CH₂CH₂), 2.72-2.65 (m, 4H, CH₂CH₂CH₂), 2.47-2.42 (m, 2H, NCH₂CH₂), 2.32-2.26 (m, 2H, SO₂CH₂CH₂CH₂), 2.14-2.07 (m, 1H, H of CH₂CH₂CH₂), 1.89-1.85 (m, 1H, H of CH₂CH₂CH₂).

¹³C NMR (101 MHz, DMSO-*d*₆) *δ* (ppm): 176.35, 167.12, 161.90, 160.11 (d, *J*_{C-F}=245.4 Hz), 159.55, 156.06, 155.88, 138.23 (d, *J*_{C-F}=11.1 Hz), 134.03, 128.85, 128.41 (d, *J*_{C-F}=6.0 Hz), 126.58 (d, *J*_{C-F}=15.1 Hz), 116.74, 108.64 (d, *J*_{C-F}=27.2 Hz), 106.44, 67.47, 55.36, 51.33, 48.84, 44.66, 31.89, 31.52, 25.21, 18.74, 17.30.
HRMS (ESI): m/z [M+H]⁺ Calcd for C₂₇H₂₆ClFN₆O₄S: 585.1487; Found: 585.1507

### Example 27

### Step 1 Synthesis of methyl 2-(4-((2-(4-(5-chloropyrimidin-2-yl)piperazin-1-yl)-5,5-dioxo-7,8-dihydro- 6H -thiapyrano[3,2-d]pyrimidin-4-yl)amino)-2-fluorophenyl)acetate (1)

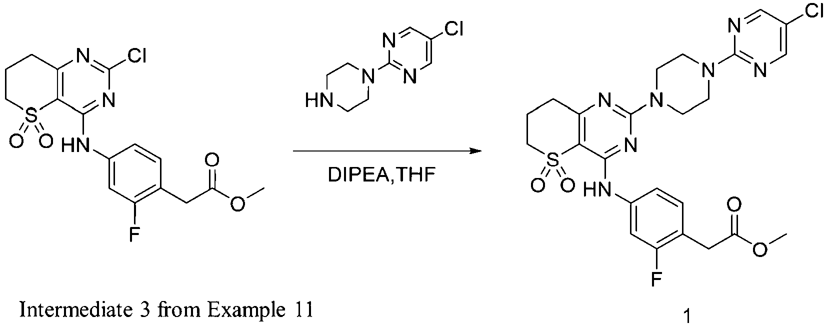

**Intermediate 3** (200 mg, 0.50 mmol) from **Example 11,** 5-chloro-2-(piperazin-1-yl)pyrimidine (109 mg, 0.55 mmol), DIPEA (194 mg, 1.50 mmol), THF (5 mL) and H₂O (1 mL) were added in sequence to a 25 mL single-necked flask. The reaction was carried out overnight at 65°C under nitrogen protection. TLC (*V*_{dichloromethane} : *V*ₘₑₜₕₐₙₒₗ = 50:1) monitored that the reaction was completed. The mixture was concentrated and slurried with tetrahydrofuran (2 mL) to obtain 142 mg of white solid **intermediate 1,** with a yield of 87.6%.

### Step 2 Synthesis of 2-(4-((2-(4-(5-chloropyrimidin-2-yl)piperazin-1-yl)-5,5-dioxo-7,8-dihydro- 6H -thiapyrano[3,2-d]pyrimidin-4-yl)amino)-2-fluorophenyl)acetic acid (Example 27)

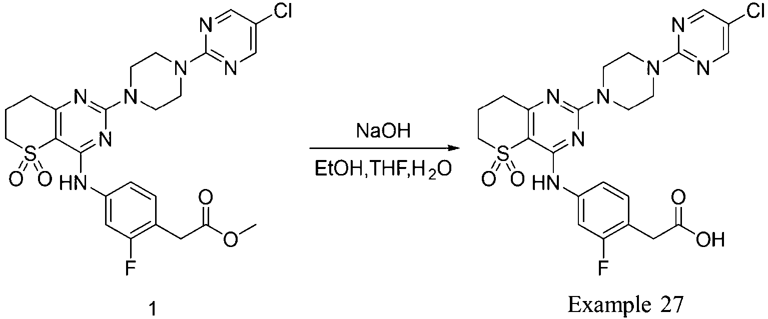

**Intermediate 1** (142 mg, 0.25 mmol), ethanol (2 mL), tetrahydrofuran (2 mL), water (2 mL), and sodium hydroxide (15 mg, 0.38 mmol) were added in sequence to a 25 mL single-necked flask. The reaction was carried out at 30°C for 4 h under N₂ protection. TLC (*V*_{dichloromethane} : *V*ₘₑₜₕₐₙₒₗ = 50:1) monitored that the reaction was completed. The pH of the solution was adjusted to 7 with 3M hydrochloric acid, concentrated, and the pH of the solution was adjusted to 4 with 3M hydrochloric acid. The solution was filtered, the filter cake was washed with water (10 mL), and the filter cake was dried to obtain 120 mg of white solid **Example 27,** with a yield of 87.6%.

¹H NMR (400 MHz, DMSO-*d*₆) *δ* (ppm): 12.62 (s, 1H, COOH), 8.70 (s, 1H, NH), 8.46 (s, 2H, ArH), 7.56 (dd, *J*₁=12.1 Hz, *J*₂=2.1 Hz, 1H, ArH), 7.36-7.24 (m, 2H, ArH), 3.82-3.80 (m, 8H, piperazine-H), 3.63-3.56 (m, 4H, CH₂COOH, SO₂CH₂), 2.84 (t, *J*=6.4 Hz, 2H, SO₂CH₂CH₂CH₂), 2.35-2.25 (m, 2H, SO₂CH₂CH₂).

¹³C NMR (101 MHz, DMSO) *δ* (ppm): 172.35, 167.22, 160.74 (d, *J*_{C-F}=244.4 Hz), 159.87, 159.63, 156.49, 156.19, 138.70 (d, *J*_{C-F}=11.1 Hz), 132.31 (d, *J*_{C-F}=6.1 Hz), 118.20, 118.08 (d, *J*_{C-F}=17.2 Hz), 117.37 (d, *J*_{C-F}=3.0 Hz), 108.66 (d, *J*_{C-F}=27.3 Hz), 106.42, 51.35, 43.72, 43.50, 34.44, 31.89, 18.75.

HRMS (ESI): m/z [M+H]⁺ Calcd for C₂₃H₂₃ClFN₇O₄S: 548.1283; Found: 548.1215.

### Bioactivity Research

### (I) Inhibitory activity of the compounds of the present invention against PDE4B enzyme

### 1. Materials and Instruments

| **Materials and Reagents** | **Suppliers** |
|---|---|
| PDE4B2 | BPS bioscience |
| Cyclic-3',5'-AMP | Sigma |
| Rolipram | MCE |
| AMP-Glo^{™} Detection Kit | Promega |
| Tris | amresco |
| BSA | Perkin Elmer |
| Magnesium chloride | Sigma |
| Tween-20 | Solarbio |
| DTT | Invitrogen |
| DMSO | Sigma |

| **Instruments and Consumables** | **Suppliers** |
|---|---|
| 96-well polypropylene transfer plate | Nunc |
| 384-well plate, white, low volumn, round bottom | Greiner |
| Plate vibrator | QILINBEIER |
| Centrifuge | Eppendorf |
| Envision Multi-tab Reader | PerkinElmer |
| Vortex Generator | IKA |
| Echo | Labcyte |

### 2. Experimental Procedures

2.1 Compound preparation and treatment: all compounds were prepared into 50 mM stock solutions using dimethyl sulfoxide.

2.2 Preparation of working stock solution: a) the reference rolipram was serially diluted 3 times with dimethyl sulfoxide starting from 2 mmol, to a total of 10 concentrations. b) 2 mmol of the compound of the present invention was serially diluted three times with dimethyl sulfoxide (DMSO) to a total of 10 concentrations. c) A 200X positive control (2 mM rolipram) and a 200X vector control (100% dimethyl sulfoxide) were prepared. d) The compound plate was centrifuged at 1000 rpm for 1 minute.

### 2.3 Complex Screening

a) An Echo 550 was used to transfer 20 nL of compound dilution to each well of the detection plate.
b) The test plate was sealed and centrifuged at 1000 rpm for 1 minute.
c) 2X PDE4B2 was prepared in frozen PDE detection buffer.
d) 2 µL of 2X PDE4B2 was added to each well of the detection plate (prepared in step b).
e) The test plate was sealed and incubated for 10 minutes under RT conditions.
f) 2X Cyclic-3',5'-AMP was prepared in PDE detection buffer.
g) 2 µl of 2X Cyclic-3',5'-AMP (prepared in step f) was added to each well of the detection plate (prepared in step e) to initiate the reaction. Incubation was performed at room temperature for 60 minutes.
h) 4 µL of AMP-Glo reagent I was added.
i) 8 µL of AMP detection solution was added. Incubation was performed at room temperature for 60 minutes.
k) The RLU signal was read on the Envision 2105 reader.

### 3. Test Results

As shown in the table below, the compounds of the present invention have extremely strong PDE4B inhibitory activity, and the activity of some compounds is significant stronger than that of compound II disclosed by WO2013026797.

| No. of Examples | PDE4B inhibitory activity | No. of Examples | PDE4B inhibitory activity |
|---|---|---|---|
| 1 | +++++ | 15 | +++++ |
| 2 | +++ | 16 | +++++ |
| 3 | +++ | 17 | +++++ |
| 4 | +++ | 18 | +++++ |
| 5 | +++ | 19 | +++++ |
| 6 | +++ | 20 | ++++ |
| 7 | ++++ | 21 | ++++ |
| 8 | +++ | 22 | ++++ |
| 9 | +++ | 23 | ++++ |
| 11 | ++++ | 24 | +++ |
| 12 | +++ | 25 | ++++ |
| 13 | +++ | 26 | |
| 14 | +++++ | 27 | ++++ |
| | | WO2013026797 (Compound II) | +++ |

| | | | |
|---|---|---|---|
| Note: "+" indicates IC₅₀ >1 µM "++" indicates IC₅₀ = 100 nM to 1 µM "++" indicates IC₅₀ = 20 nM to 100 nM "++++" indicates IC₅₀ = 1 to 20 nM "++++" indicates IC₅₀ <1 nM | | | |

### (II) Selectivity of the compounds of the present invention for PDE4 enzyme isotypes

### 1. Materials and Instruments

| **Materials and Reagents** | **Suppliers** |
|---|---|
| PDE4A1, PDE4B2A, PDE4C1, PDE4D2, PDE4D3 | BPS bioscience |
| Cyclic-3',5'-AMP | Sigma |
| Rolipram | MCE |
| AMP-Glo^{™} Detection Kit | Promega |
| Tris | amresco |
| BSA | Perkin Elmer |
| Magnesium chloride | Sigma |
| Tween-20 | Solarbio |
| DTT | Invitrogen |
| DMSO | Sigma |

| **Instrument and Consumables** | **Suppliers** |
|---|---|
| 96-well polypropylene transfer plate | Nunc |
| 384-well plate, white, low volumn, round bottom | Greiner |
| Plate vibrator | QILINBEIER |
| Centrifuge | Eppendorf |
| Envision Multi-tab Reader | PerkinElmer |
| Vortex Generator | IKA |
| Echo | Labcyte |

### 2. Methods

### Same detection method as PDE4B

### 3. Results

The compounds of the present invention exhibit better selectivity for PDE4B than compound 2 disclosed by patent WO2013026797.

| No. of Compounds | IC₅₀ (nM) | | | | |
|---|---|---|---|---|---|
| | PDE4A1A | PDE4B2A | PDE4C1 | PDE4D2 | PDE4D3 |
| WO2013026797 (Compound II) | 111.6 | 24.49 | 2191 | 37.09 | 65.84 |
| **Selectivity over PDE4B** | **4.5** | **1** | **89** | **1.5** | **2.7** |
| Example 11 | 375.1 | 4.807 | 3548 | 84.18 | 183.6 |
| **Selectivity over PDE4B** | **78** | **1** | **738** | **18** | **38** |

### (III) Toxicity of the compounds of the present invention to hERG

HEK-293 cell line stably expressing hERG potassium channels (purchased from Creacell, catalog number: A-0320) was used. HEK293 cell lines were cultured in DMEM medium containing 10% fetal bovine serum and 0.8 mg/mL G418 at a temperature of 37°C and a carbon dioxide concentration of 5%. For patch-clamp assays, cells were separated using TrypLE^{™} Express before the assay. 4 × 10³ cells were seeded onto coverslips and cultured in 24-well plates (final volume: 500 µL). The assays were performed 18 hours later.

Whole-cell patch-clamp test: after whole-cell sealing is achieved, the cell membrane voltage is clamped to -80mV. The clamping voltage is depolarized from -80 mV to -50 mV and maintained for 0.5 s (as leakage current detection), then stepped to 30 mV and maintained for 2.5 s, and then quickly restored to -50 mV and maintained for 4 s to excite the tail current of the hERG channel. Data was collected every 10 seconds to observe the effect of the drug on hERG tail current. A -50 mV stimulus of 0.5 s was used as the leakage current detection. Experimental data were collected by the EPC-10 amplifier and stored in the software. Once the hERG current recorded in whole cells stabilized, drug administration began. Each drug concentration was administered for 5 minutes (or the current stabilized) before the next concentration was measured. Data for each test compound was measured at concentrations of 0.03 µM, 0.3 µM, 1 µM, 3 µM, 10 µM, and 30 µM, and the IC₅₀ value of *hERG* was calculated according to the equation.

Compared with compound II disclosed by WO2013026797, the compounds of the present invention significantly reduce the inhibitory activity of the *hERG* potassium channel, and most of the compounds inhibit *hERG* with an IC₅₀ >30 µM, resulting in lower cardiotoxicity risk and higher safety.

| No. of Examples | *h* ERG IC₅₀ (µM) |
|---|---|
| 1 | 14.49 |
| 2 | >30 |
| 3 | >30 |
| 4 | >30 |
| 7 | >30 |
| 8 | >30 |
| 11 | >30 |
| 14 | >30 |
| 21 | >30 |
| 22 | >30 |
| 24 | >30 |
| 26 | >30 |
| WO2013026797 (Compound II) | >3.276 |

### (IV) Effects of the compounds of the present invention on chronic obstructive pulmonary disease in rats

### 1. Animals and Reagents

| **Animals and Reagents** | **Suppliers** |
|---|---|
| SPF grade SD rats (180-220g) | Henan Skebes Biotechnology Co., Ltd. |
| Lipopolysaccharide (LPS) | Sigma |
| Chloral hydrate (5%) | Shanghai Aladdin Biochemical Technology Co., Ltd. |
| Sodium carboxymethyl cellulose (CMC-Na) | Sinopharm Chemical Reagent Co., Ltd. |
| ELISA kit | Shanghai Enzyme-Linked Biotechnology Co., Ltd. |

### 2. Experimental Procedures

### 2.1 Experimental Grouping

Forty SD rats were acclimatized in an animal experiment center for 7 days, with alternating lighting for 12 hours, an ambient temperature of 20-25°C, and a humidity of 50%-65%. Subsequently, the SD rats were randomly divided into 5 groups according to their body weight, with 8 rats in each group and half male and half female. These groups were the blank control group, the model control group, and the treatment group of Example 11 (with doses of 5, 10, and 20 mg/kg, respectively).

### 2.2 Preparation of experimental reagents

Test sample solutions: weighing 5 mg, 10 mg and 20 mg of the compound of Example 11 and dissolving it in 10 ml of 0.5% CMC-Na aqueous solution to prepare solutions with concentrations of 0.5, 1 and 2 mg/ml.

### 2.3 Experimental Scheme

Except for the blank control group, SD rats were used to establish a rat model of chronic obstructive pulmonary disease (COPD) by fumigation and intratracheal instillation of LPS. On days 1, 14, 28 and 42 of modeling, the rats were anesthetized with chloral hydrate and then 125 µL of LPS (2 mg/mL) solution was instilled into the trachea. For the rest of the time, the animals were placed in a self-made fumigation box and each group of rats was continuously fumigated with 10 lit cigarettes for 1.5 hours (once in the morning and once in the afternoon) for 50 consecutive days. After the model was established, the rats in each group were given the drug twice a day for 28 consecutive days.

### 2.4 Experimental Results

### 2.4.1 Results of Forced Vital Capacity (FVC) and Maximum Expiratory Volume in One Second (FEV1) in COPD Rats

The rat lung function test instrument was used to measure FVC and FEV1 on day 14 of treatment and at the last administration, and the FEV1/FVC ratio was calculated. The results are shown in Table 1.

**Table 1. Effects of Example 11 on FEV1/FVC in rats with chronic obstructive pulmonary disease (Mean ± SD, n=8)**

| Group | Dosage (mg/kg) | 14-day FEV 1/FVC (%) | 28-day FEV 1/FVC (%) |
|---|---|---|---|
| Blank | / | 82.34±7.65 | 82.22±1.62 |
| Model Group | / | 65.45±5.48^{##} | 64.46±3.98^{##} |
| | 5 | 66.65±5.48* | 69.99±2.65* |
| Example 11 | 10 | 72.14±6.56** | 75.30±1.84** |
| | 20 | 76.78±5.41** | 77.10±1.81** |

| | | | |
|---|---|---|---|
| ^{##} P<0.01 vs. blank group; * P<0.05, ** P<0.01 vs. model group. | | | |

Compared with the blank group, the FEV1/FVC (percentage of maximum vital capacity in one second) of rats in the model group was significantly reduced (P<0.01), indicating successful modeling. Compared with the model group, the treatment group of the compound of Example 11 significantly improved the FEV1/FVC lung function of rats (P<0.05, P<0.01), indicating good therapeutic effect. The 10 mg/kg and 20 mg/kg dose treatment groups of the compound of Example 11 were significantly better than the 5 mg/kg dose treatment group (P<0.01), indicating a dose gradient relationship.

### 2.4.2 Levels of IL-1β and TNF-α in serum in COPD rats

Blood samples were collected after the last administration of the drug to measure inflammatory markers. Levels of IL-1β and TNF-α in serum were measured according to the ELISA instructions. The results are shown in Table 2.

**Table 2. Effects of Example 11 on serum IL-1β and TNF-α in COPD rats (mean ± SD, n=8)**

| Group | Dosage (mg/kg) | IL-1β (pg/ml) | TNF-α (pg/ml) |
|---|---|---|---|
| Blank | / | 31.04±13.52 | 30.93±7.94 |
| Model Group | / | 111.96±24.44^{##} | 205.00±41.68^{##} |
| | 5 | 71.13±16.14** | 118.36±27.30* |
| Example 11 | 10 | 51.92±16.06** | 90.15±15.79** |
| | 20 | 50.75±15.07** | 73.23±11.78** |

| | | | |
|---|---|---|---|
| ^{##} P<0.01 vs. blank group; * P<0.05, ** P<0.01 vs. model group. | | | |

Compared with the blank group, the levels of IL-1β and TNF-α in the serum of rats in the model group were significantly increased (P<0.01), indicating successful modeling; compared with the model group, the treatment group of the compound of Example 11 significantly improved the levels of IL-1β and TNF-α in the serum of rats (P<0.01), indicating significant therapeutic effect, and indicating a dose gradient relationship.

### 2.4.3 Pathological examination results of lung tissue from COPD rats

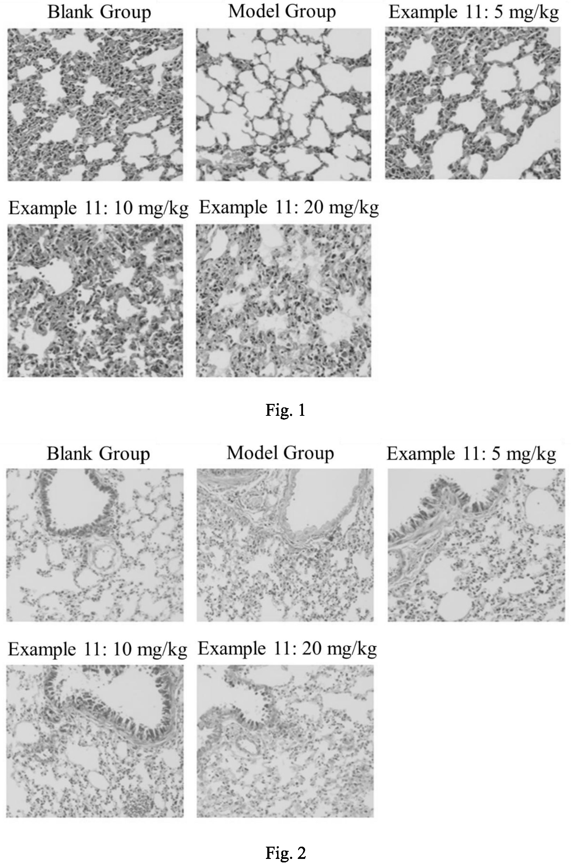

After the last treatment, all three lobes of the right lung of the rats were removed, fixed and dehydrated in 10% formaldehyde, embedded in paraffin, sectioned, and stained with hematoxylin and eosin (HE). The morphological changes of the lung tissue of the rats in each group were observed. The results are shown in Fig. 1 and Table 3.

**Table 3. Effect of Example 11 on lung tissue pathological scores in COPD rats (mean ± SD, n=8)**

| Group | Dosage (mg/kg) | Pathological score |
|---|---|---|
| Blank | / | 0.33+0.52 |
| Model Group | / | 4.83±0.75^{##} |
| | 5 | 3.83+0.75 |
| Example 11 | 10 | 2.00±0.63** |
| | 20 | 1.83±0.41** |

| | | |
|---|---|---|
| ^{##} P<0.01 vs. blank group; * P<0.05, ** P<0.01 vs. model group. | | |

Compared with the blank group, the alveolar septa in the lung tissue of rats of the model group were widened and infiltrated with inflammatory cells. The adjacent lung tissue and alveolar cavities showed compensatory expansion, indicating that the model was successfully established. Compared with the model group, the treatment group of the compound of Example 11 significantly improved the pathological score of the rat lung tissue (P<0.01), indicating that it has a significant therapeutic effect, and indicating that there is a dose gradient relationship.

### (V) Effects of the compounds of the present invention on eosinophilic asthma induced by house dust mite extract in mice

### 1. Animals and Reagents

| **Animals** | **Suppliers** |
|---|---|
| SPF grade BALB/c mice (18-22g) | Henan Skebes Biotechnology Co., Ltd. |
| Chloral hydrate (5%) | Shanghai Aladdin Biochemical Technology Co., Ltd. |
| Sodium carboxymethyl cellulose (CMC-Na) | Sinopharm Chemical Reagent Co., Ltd. |
| PBS | Invitrogen |

### 2. Experimental Procedures

### 2.1 Experimental Grouping

50 BALB/c mice were randomly divided into 5 groups according to their body weight, with 10 rats in each group and half male and half female. These groups were the blank control group, the model control group, and the treatment group of Example 11 (with doses of 5, 10, and 20 mg/kg, respectively).

### 2.2 Preparation of experimental reagents

House dust mite extract solution: weighing 25mg of house dust mite extract, adding it to 1ml of physiological saline and mixing well to prepare a solution with a concentration of 25mg/ml.

Test sample solutions: weighing 5 mg, 10 mg and 20 mg of the compound from Example 11 and dissolving it in 10 ml of 0.5% CMC-Na aqueous solution to prepare solutions with concentrations of 0.5, 1 and 2 mg/ml.

### 2.3 Experimental Scheme

Except for the blank control group, BALB/c mice were sensitized by nasal drops of house dust mite extract solution on days 1, 7, and 14 of modeling. After anesthetizing the mice with chloral hydrate, 25 mg/ml house dust mite extract solution was slowly dripped into the nose (20 µl). For 6 consecutive days from days 20 to 25, 50 mg/ml house dust mite extract solution was dripped into the nose (20 µl) to challenge the mice and establish a mouse eosinophilic asthma model. Subsequently, each treatment group received the medication twice daily for 14 consecutive days.

### 2.4 Experimental Results

### 2.4.1 Results of eosinophil count and sneeze frequency

The number of eosinophils in the lung lavage fluid of mice and the number of sneezes in mice within 20 minutes after the last administration were counted. The results are shown in Table 4.

**Table 4. Effects of Example 11 on eosinophil count and sneezing frequency in eosinophilic asthmatic mice (mean ± SD, n=10)**

| Group | Dosage (mg/kg) | Eosinophils (%) | Number of sneezes (20 minutes) |
|---|---|---|---|
| Blank | / | 4.46±1.72 | 5.4±2.8 |
| Model Group | / | 28.32±4.70^{##} | 34.2±5.9^{##} |
| | 5 | 21.97±2.58* | 17.9±3.4** |
| Example 11 | 10 | 17.93±3.30**^{△△} | 16.3±3.7** |
| | 20 | 16.04±2.97** | 13.8±1.9** |

| | | | |
|---|---|---|---|
| ^{##} P<0.01 vs. blank group; * P<0.05, ** P<0.01 vs. model group. | | | |

Compared with the blank group, the percentage of eosinophils in the bronchoalveolar lavage fluid of the model group was significantly increased (P<0.01), indicating successful modeling; compared with the model group, the treatment group of the compound of Example 11 significantly reduced eosinophils (P<0.05, P<0.01), showing significant therapeutic effect; and indicating a dose gradient relationship.

Compared with the blank group, the number of sneezes of the model group within 20 minutes was significantly increased (P<0.01), indicating successful modeling; compared with the model group, the number of sneezes of the treatment groups of the compound of Example 11 within 20 minutes was significantly reduced (P<0.01), showing significant therapeutic effect; and indicating a dose gradient relationship.

### 2.4.2 Levels of IgE and IL-4 in bronchoalveolar lavage fluid

The levels of IgE and IL-4 in the bronchoalveolar lavage fluid of mice with eosinophilic asthma were measured, and the results are shown in Table 5.

**Table 5. Effects of Example 11 on IgE and IL-4 in bronchoalveolar lavage fluid of mice with eosinophilic asthma (Mean ± SD, n = 10)**

| Group | Dosage (mg/kg) | IgE (pg/ml) | IL-4 (pg/ml) |
|---|---|---|---|
| Blank | / | 50.78±19.23 | 17.61+7.98 |
| Model Group | / | 340.86±70.67^{##} | 104.51±25.72^{##} |
| | 5 | 266.55±52.03* | 70.92±10.87** |
| Example 11 | 10 | 219.05±38.74** | 59.38±9.90** |
| | 20 | 189.36±56.69** | 51.13±16.07** |

| | | | |
|---|---|---|---|
| ^{##} P<0.01 vs. blank group; * P<0.05, ** P<0.01 vs. model group. | | | |

Compared with the blank group, the level of IgE in the bronchoalveolar lavage fluid of the model group mice was significantly increased (P<0.01), indicating that the model was successfully established; compared with the model group, the treatment groups of the compound of Example 11 could significantly reduce the IgE level in the bronchoalveolar lavage fluid (P<0.05, P<0.01), showing significant therapeutic effects, and indicating that there is a a dose gradient relationship.

Compared with the blank group, the IL-4 level in the bronchoalveolar lavage fluid of the model group mice was significantly increased (P<0.01), indicating that the model was successfully established; compared with the model group, the treatment groups of the compound of Example 11 could significantly reduce the IL-4 level in the bronchoalveolar lavage fluid (P<0.01), which showed significant therapeutic effect, and also indicated that there was a dose gradient relationship.

### (VI) Effects of the compounds of the present invention on bleomycin-induced pulmonary fibrosis in rats

### 1. Animals and Reagents

| **Animals** | **Suppliers** |
|---|---|
| SPF grade male SD rats (180-200g) | Henan Skebes Biotechnology Co., Ltd. |

| **Reagents** | **Suppliers** |
|---|---|
| Chloral hydrate (5%) | Shanghai Aladdin Biochemical Technology Co., Ltd. |
| Sodium carboxymethyl cellulose (CMC-Na) | Sinopharm Chemical Reagent Co., Ltd. |
| Bleomycin | Dalian Meilun Biotechnology Co., Ltd. |

### 2. Experimental Procedures

### 2.1 Experimental Grouping

50 SD rats were randomly divided into 5 groups according to their body weight, with 10 rats in each group and half male and half female. These groups were the blank control group, the model control group, and the treatment group of Example 11 (with doses of 5, 10, and 20 mg/kg, respectively).

### 2.2 Preparation of experimental reagents

Bleomycin solution: weighing 5 mg of bleomycin powder, adding it to 1 ml of physiological saline and mixing well to prepare a solution with a concentration of 5 mg/ml.

Test sample solutions: weighing 5 mg, 10 mg and 20 mg of the compound from Example 11 and dissolving it in 10 ml of 0.5% CMC-Na aqueous solution to prepare solutions with concentrations of 0.5, 1 and 2 mg/ml.

### 2.3 Experimental Scheme

After anesthetizing rats with chloral hydrate, bleomycin solution was injected into the trachea through the vocal cords at a dose of 5 mg/kg and a volume of 1 ml/kg; rats in the blank control group were injected with an equal volume of physiological saline into the trachea. Bleomycin was administered via intratracheal injection on day 1, and intragastric administration began on day 8 at a volume of 10 ml/kg, twice daily for 14 consecutive days. Blood and lungs were collected from each group of animals on day 22. After euthanizing the rats, the trachea was exposed, and an intravenous cannula was inserted into the rat's bronchus. The trachea was lavaged twice with PBS (5 ml), and the lavage fluid was collected. Then, centrifugation was performed at 1500 r/min for 10 min. The supernatant was collected.

### 2.4 Experimental Results

Masson's staining was used to determine the degree of fibrosis; at the same time, ELISA was used to measure the levels of IL-6, TNF-α and TGF-β1 in bronchoalveolar lavage fluid.

**Table 6. Effect of Example 11 on bleomycin-induced pulmonary fibrosis in rats (mean ± SD, n=10)**

| Group | Dosage (mg/kg) | Percentage of lung collagen fibers (%) |
|---|---|---|
| Blank | / | 3.2±1.6 |
| Model Group | / | 23.6±5.4^{##} |
| | 5 | 16.1±4.6* |
| Example 11 | 10 | 9.7±2.2** |
| | 20 | 8.5±2.5** |

| | | |
|---|---|---|
| ^{##} P<0.01 vs. blank group; * P<0.05, ** P<0.01 vs. model group. | | |

As shown in Fig. 2 and Table 6, compared with the blank control group, the collagen fiber level in the lung tissue of rats in the model group was significantly increased after bleomycin induction (P<0.01), indicating that the model was successfully established; compared with the model control group, the collagen fiber level in the lung tissue of the treatment groups of Example 11 was significantly decreased (P<0.05, P<0.01), indicating that the treatment groups had a therapeutic effect.

**Table 7. Effects of Example 11 on TGF-β and IL-6 in bleomycin-induced bronchoalveolar lavage fluid of rats (mean ± SD, n=10)**

| Group | Dosage (mg/kg) | TGF-β (pg/ml) | IL-6 (pg/ml) |
|---|---|---|---|
| Blank | / | 49.7±7.4 | 11.1±2.1 |
| Model Group | / | 84.9±8.4^{##} | 38.4±2.3^{##} |
| | 5 | 79.0±6.4 | 34.8+3.7 |
| Example 11 | 10 | 69.1±6.3** | 32.5±6.1* |
| | 20 | 56.3±5.2** | 28.3±3.9** |

| | | | |
|---|---|---|---|
| ^{#} P<0.05, ^{##} P<0.01 vs. blank group; * P<0.05, ** P<0.01 vs. model group. | | | |

As shown in Table 7, compared with the blank control group, the rats in the model group showed a significant increase in the level of TGF-β and IL-6 in bronchoalveolar lavage fluid after bleomycin induction (P<0.01), indicating successful model establishment; compared with the model control group, the treatment groups of Example 11 showed a significant decrease in the level of TGF-β and IL-6 in bronchoalveolar lavage fluid (P<0.05, P<0.01), indicating that the treatment groups had a therapeutic effect.

All references mentioned herein are incorporated by reference. It should be understood that many variations and modifications may be made to the technical solutions of the present invention without departing from the spirit and scope of the present disclosure.

## Claims

1. A compound represented by formula (I) 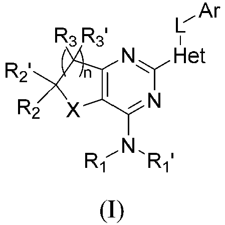
or a stereoisomer, tautomer, solvate, prodrug, isotopologue and/or pharmaceutically acceptable salt thereof, wherein
X is selected from S=O or S(=O)₂;
R₁ is selected from H or C₁ - C₆ alkyl;
R₁' is selected from C₃-C₈ carbocyclyl, C₆-C₁₀ aryl, 5-10 membered heteroaryl, or 5-10 membered heterocyclyl; wherein the carbocyclyl, aryl, heteroaryl, and heterocyclyl are substituted with R₄-(CRₐR_{B})ₘ- and optionally substituted with halogen, hydroxyl, amino, mercapto, C₁-C₆ alkyl, and/or C₁-C₆ alkoxyl;
R₂, R₂', R₃, and R₃' are each independently selected from hydrogen, C₁-C₆ alkyl, C₂-C₆ alkenyl, or C₂-C₆ alkynyl;
R₄ is selected from hydroxyl, amino, mercapto, carboxyl, -SO₃H, -PO₄H, tetrazolyl, triazolyl, -C(=O)OC₁-C₆ alkyl, or -C(=O)NR_{c}R_{d};
Rₐ and R_{b} are each independently selected from hydrogen, C₁-C₆ alkyl, halogen, hydroxyl, or Rₐ and R_{b}, together with the carbon atom to which they are attached, form a C₃-C₆ carbocyclyl; R_{c} and R_{d} are each independently selected from hydrogen, C₁-C₆ alkyl, or R_{c} and R_{d}, together with the nitrogen atom to which they are attached, form a 3- to 6-membered heterocyclyl;
Het is selected from 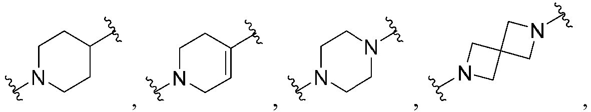 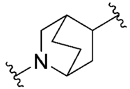 5- or 6-membered nitrogen-containing heteroaryl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from 5-10 membered heterocyclyl, 5-10 membered heteroaryl, C₆-C₁₀ aryl, or C₃-C₈ carbocyclyl; said heterocyclyl, heteroaryl, aryl, and carbocyclyl are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 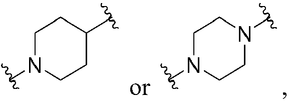 and R₁' is C₃-C₈ carbocyclyl, L is not a bond.

2. The compound according to claim 1, or a stereoisomer, tautomer, solvate, prodrug, isotopologue and/or pharmaceutically acceptable salt thereof, wherein
X is selected from S=O or S(=O)₂;
R₁ is selected from H or C₁ - C₆ alkyl;
R₁' is selected from C₃-C₈ carbocyclyl, C₆-C₁₀ aryl, or 5-10 membered heteroaryl; wherein the carbocyclyl, aryl, and heteroaryl are substituted with R₄-(CRₐR_{B})ₘ- and optionally substituted with halogen, hydroxyl, amino, mercapto, C₁-C₆ alkyl, and/or C₁-C₆ alkoxyl;
R₂, R₂', R₃, and R₃' are each independently selected from hydrogen or C₁-C₆ alkyl;
R₄ is selected from hydroxyl, amino, mercapto, carboxyl, -SO₃H, -PO₄H, tetrazolyl, triazolyl, -C(=O)OC₁-C₆ alkyl, or -C(=O)NR_{c}R_{d};
Rₐ and R_{b} are each independently selected from hydrogen, C₁-C₆ alkyl, halogen, hydroxyl, or Rₐ and R_{b}, together with the carbon atom to which they are attached, form a C₃-C₆ carbocyclyl; R_{c} and R_{d} are each independently selected from hydrogen, C₁-C₆ alkyl, or R_{c} and R_{d}, together with the nitrogen atom to which they are attached, form a 3- to 6-membered heterocyclyl;
Het is selected from 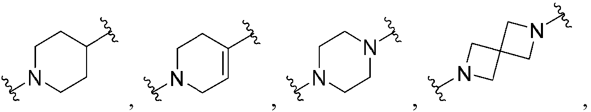 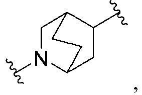 5- or 6-membered nitrogen-containing heteroaryl;
L is selected from a bond, C₁-C₆ alkylene, -C(=O)-, -NRₑC(=O)-, -C(=O)-N(Rₑ)-, - OC(=O)-, or -C(=O)O-; Rₑ is selected from hydrogen or C₁-C₆ alkyl;
Ar is selected from 5-10-membered heteroaryl or C₆-C₁₀ aryl; said heteroaryl and aryl are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 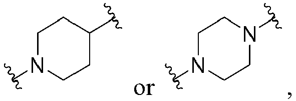 and R₁' is C₃-C₈ carbocyclyl, L is not a bond.

3. The compound according to claim 1 or 2, or a stereoisomer, tautomer, solvate, prodrug, isotopologue and/or pharmaceutically acceptable salt thereof, wherein
R₁' is selected from cyclopropyl, cyclobutyl, cyclopentyl, cyclohexyl, phenyl, pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, all of which are substituted by R₄-(CRₐR_{b})ₘ- and optionally substituted by halogen, hydroxyl, amino, mercapto, C₁-C₆ alkyl, and/or C₁-C₆ alkoxyl;
R₂, R₂', R₃, and R₃' are each independently selected from hydrogen or C₁-C₆ alkyl;
R₄ is selected from hydroxyl, amino, mercapto, carboxyl, -SO₃H, -PO₄H, tetrazolyl, triazolyl, -C(=O)OC₁-C₂₀ alkyl, or -C(=O)NR_{c}R_{d};
Rₐ and R_{b} are each independently selected from hydrogen, C₁-C₆ alkyl, halogen, hydroxyl, or Rₐ and R_{b}, together with the carbon atom to which they are attached, form a C₃-C₆ carbocyclyl; R_{c} and R_{d} are each independently selected from hydrogen, C₁-C₆ alkyl, or R_{c} and R_{d}, together with the nitrogen atom to which they are attached, form a 3- to 6-membered heterocyclyl;
Het is selected from 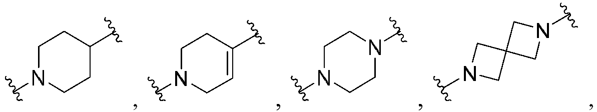 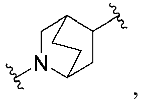 5- or 6-membered nitrogen-containing heteroaryl;
L is selected from a bond, C₁-C₆ alkylene, -C(=O)-, -NRₐC(=O)-, -C(=O)-N(Rₐ)-, - OC(=O)-, or -C(=O)O-;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl, all of which are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with the proviso that: when X is S=O, Het is 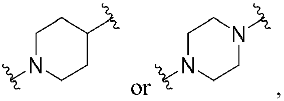 and R₁' is cyclopropyl, cyclobutyl, cyclopentyl, or cyclohexyl, L is not a bond.

4. The compound according to any one of claims 1 to 3, or the stereoisomer, tautomer, solvate, prodrug, isotopologue and/or pharmaceutically acceptable salt thereof, wherein
R₁' is selected from cyclobutyl, phenyl, or pyridinyl, all of which are substituted by R₄-(CRₐR_{B})ₘ- and optionally substituted by halogen;
R₂, R₂', R₃, and R₃' are each independently selected from hydrogen or C₁-C₆ alkyl;
R₄ is selected from hydroxyl, amino, mercapto, carboxyl, -SO₃H, -PO₄H, tetrazolyl, triazolyl, -C(=O)OC₁-C₂₀ alkyl, or -C(=O)NR_{c}R_{d};
Rₐ and R_{b} are each independently selected from hydrogen, C₁-C₃ alkyl, halogen, or Rₐ and R_{b}, together with the carbon atom to which they are attached, form cyclopropyl or cyclobutyl; R_{c} and R_{d} are each independently selected from hydrogen, C₁-C₃ alkyl, or R_{c} and R_{d}, together with the nitrogen atom to which they are attached, form a 3 to 6-membered heterocyclyl;
Het is selected from 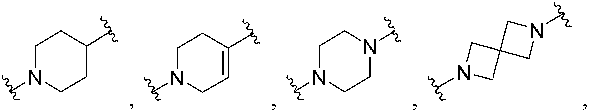 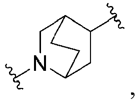 5- or 6-membered nitrogen-containing heteroaryl;
L is selected from a bond, C₁-C₆ alkylene, -C(=O)-, -NRₐC(=O)-, -C(=O)-N(Rₐ)-, - OC(=O)-, or -C(=O)O-;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl, all of which are optionally substituted by one or more groups selected from the group consisting of: halogen, hydroxyl, C₁-C₆ alkyl, and C₁-C₆ alkoxyl;
n is selected from 1 or 2; m is selected from 1, 2, 3, 4, 5 or 6;
with proviso that: when X is S=O, Het is 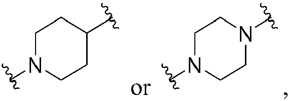 and R₁' is cyclobutyl, L is not a bond.

5. The compound according to any one of claims 1-4, wherein the compound is a compound of formula (II): 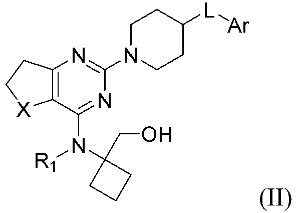
or a stereoisomer, tautomer, solvate, prodrug, isotopologue and/or pharmaceutically acceptable salt thereof, wherein,
X is selected from S=O or S(=O)₂;
R₁ is selected from H or C₁ - C₆ alkyl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl, all of which are optionally substituted by one or more groups selected from the group consisting of: halogen, hydroxyl, C₁-C₆ alkyl, and C₁-C₆ alkoxyl;
with the proviso that: when X is S=O, L is not a bond.

6. The compound according to any one of claims 1-4, wherein the compound is a compound of formula (III): 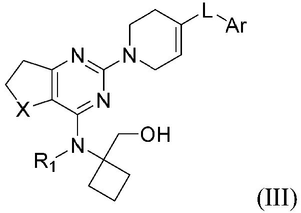
or a stereoisomer, tautomer, solvate, prodrug, isotopologue and/or pharmaceutically acceptable salt thereof, wherein,
X is selected from S=O or S(=O)₂;
R₁ is selected from H or C₁ - C₆ alkyl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, phenyl, all of which are optionally substituted by one or more groups selected from the group consisting of: halogen, hydroxyl, C₁-C₆ alkyl, and C₁-C₆ alkoxyl.

7. The compound according to any one of claims 1-4, wherein the compound is a compound of formula (IV): 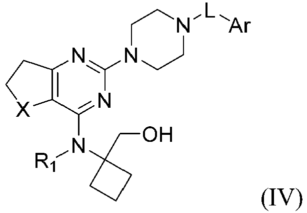
or a stereoisomer, tautomer, solvate, prodrug, isotopologue and/or pharmaceutically acceptable salt thereof, wherein,
X is selected from S=O or S(=O)₂;
R₁ is selected from H or C₁ - C₆ alkyl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, or phenyl, all of which are optionally substituted by one or more groups selected from the group consisting of: halogen, hydroxyl, C₁-C₆ alkyl, and C₁-C₆ alkoxyl;
with the proviso that: when X is S=O, L is not a bond.

8. The compound according to any one of claims 1-4, wherein the compound is a compound of formula (V): 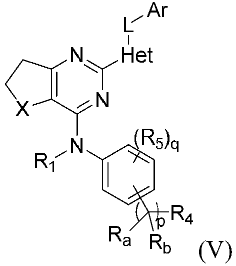
or a stereoisomer, tautomer, solvate, prodrug, isotopologue and/or pharmaceutically acceptable salt thereof, wherein,
X is selected from S=O or S(=O)₂;
R₁ is selected from H or C₁ - C₆ alkyl;
R₄ is selected from carboxyl, -SO₃H, -PO₄H, tetrazolyl, triazolyl, -C(=O)OC₁-C₆ alkyl, or -C(=O)NR_{c}R_{d};
Rₐ and R_{b} are each independently selected from hydrogen, C₁-C₃ alkyl, halogen, or Rₐ and R_{b}, together with the carbon atom to which they are attached, form cyclopropyl or cyclobutyl; R_{c} and R_{d} are each independently selected from hydrogen, C₁-C₃ alkyl, or R_{c} and R_{d}, together with the nitrogen atom to which they are attached, form a 3 to 6-membered heterocyclyl;
R₅ is selected from halogen, hydroxyl, amino, mercapto, C₁-C₆ alkyl, or C₁-C₆ alkoxyl;
Het is selected from 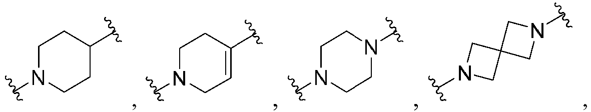 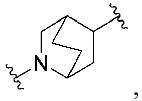 5- or 6-membered nitrogen-containing heteroaryl;
L is selected from a bond, C₁-C₆ alkylene, -C(=O)-, -NRₑC(=O)-, -C(=O)-N(Rₑ)-, -OC(=O)-, or -C(=O)O-; Rₑ is selected from hydrogen or C₁-C₆ alkyl;
Ar is selected from pyrrolyl, furanyl, thiophenyl, imidazolyl, pyrazolyl, oxazolyl, isoxazolyl, thiazolyl, isothiazolyl, pyridinyl, pyrimidinyl, pyrazinyl, pyridazinyl, phenyl, all of which are optionally substituted by one or more groups selected from the group consisting of: halogen, hydroxyl, C₁-C₆ alkyl, and C₁-C₆ alkoxyl;
p is selected from 1, 2, or 3; q is selected from 1, 2, or 3.

9. The compound according to claim 8, or a stereoisomer, tautomer, solvate, prodrug, isotopologue and/or pharmaceutically acceptable salt thereof, wherein,
X is selected from S=O or S(=O)₂;
R₁ is selected from H or C₁ - C₆ alkyl;
R₄ is selected from carboxyl, -SO₃H, -PO₄H, tetrazolyl, triazolyl, or -C(=O)OC₁-C₆ alkyl;
Rₐ and R_{b} are each independently selected from hydrogen or halogen, or Rₐ and R_{b}, together with the carbon atom to which they are attached, form cyclopropyl or cyclobutyl;
R₅ is selected from halogen, hydroxyl, amino, mercapto, C₁-C₆ alkyl, or C₁-C₆ alkoxyl;
Het is selected from 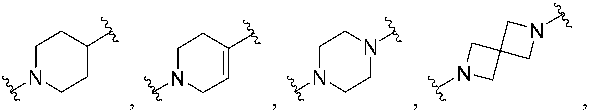 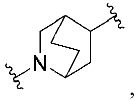 or triazolyl;
L is selected from a bond, C₁-C₆ alkylene, -C(=O)-, -NRₑC(=O)-, -C(=O)-N(Rₑ)-, - OC(=O)-, or -C(=O)O-; Rₑ is selected from hydrogen or C₁-C₆ alkyl;
Ar is selected from pyridinyl, pyrimidinyl, or phenyl, all of which are optionally substituted by one or more groups selected from the group consisting of: halogen, hydroxyl, C₁-C₆ alkyl, and C₁-C₆ alkoxyl;
p is selected from 1; q is selected from 1.

10. The compound according to claim 1, selected from: 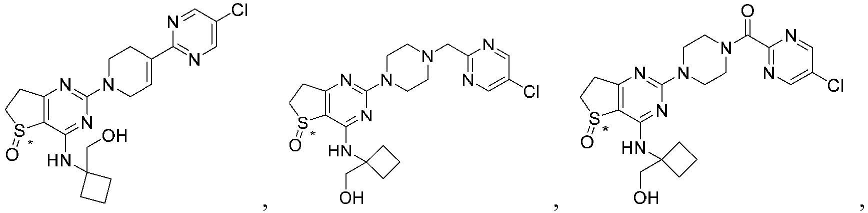 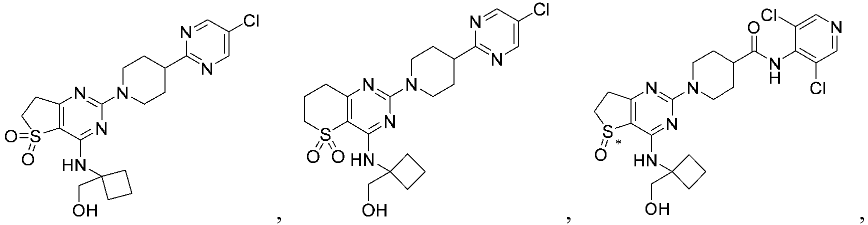 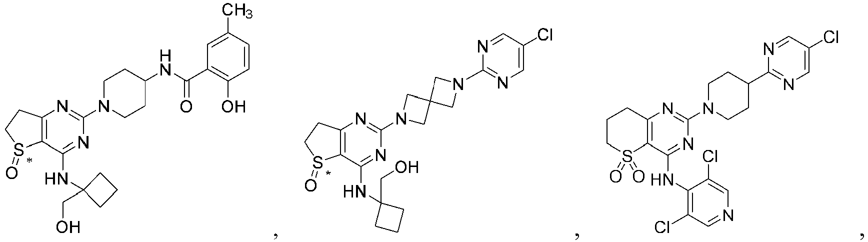 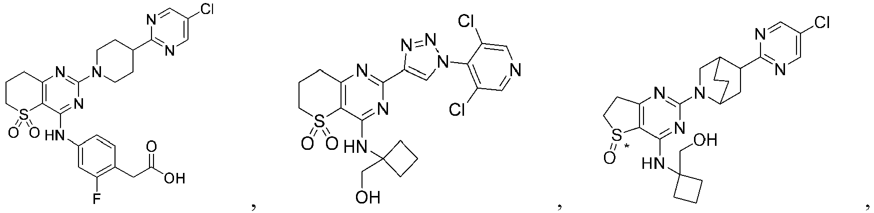 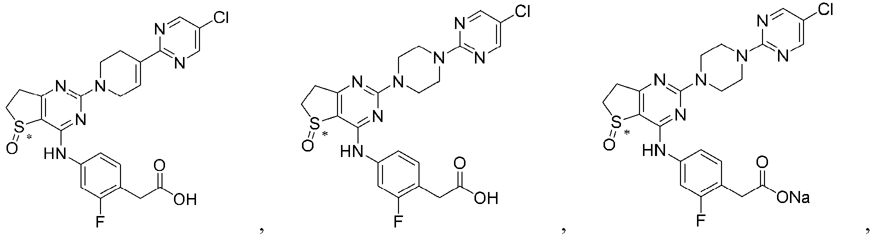 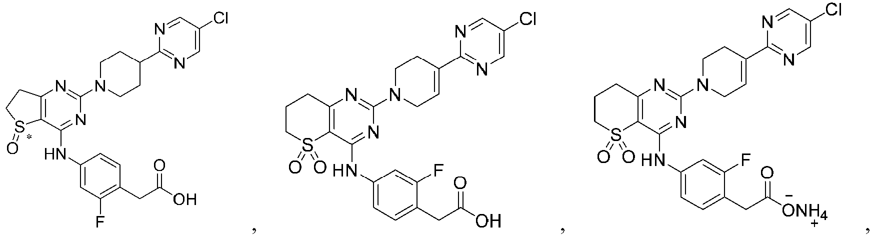 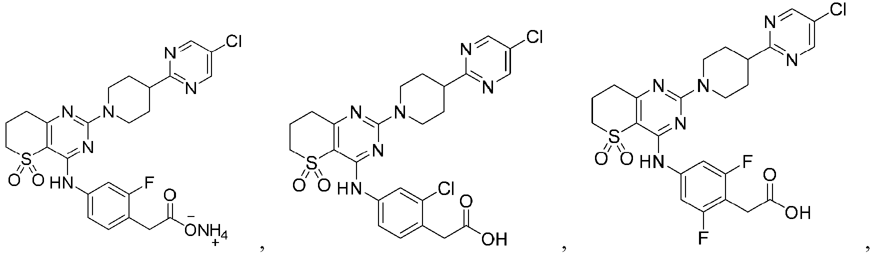 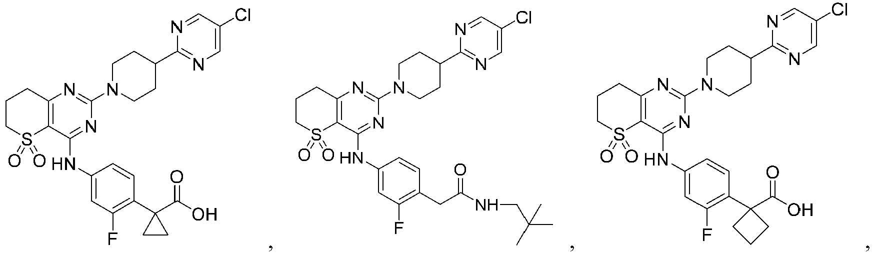 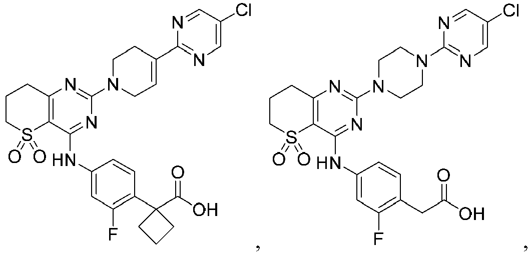 or a stereoisomer, tautomer, solvate, prodrug, isotopologue and/or pharmaceutically acceptable salt thereof.

11. A pharmaceutical composition comprising the compound according to any one of claims 1-10, or a stereoisomer, tautomer, solvate, prodrug, isotopologue and/or pharmaceutically acceptable salt thereof, and a pharmaceutically acceptable carrier.

12. Use of the compound according to any one of claims 1-10, or a stereoisomer, tautomer, solvate, prodrug, isotopologue and/or pharmaceutically acceptable salt thereof, as well as the pharmaceutical composition according to claim 11, in the manufacture of a medicament for the prevention and/or treatment of a disease mediated by phosphodiesterase 4B.

13. The use according to claim 12, wherein the disease is an inflammatory disease.

14. The use according to claim 12, wherein the inflammatory disease mediated by phosphodiesterase 4B comprises, but are not limited to, atopic dermatitis, rheumatoid arthritis, asthma, chronic obstructive pulmonary disease (COPD), idiopathic pulmonary fibrosis, interstitial lung disease, chronic sinusitis, allergic rhinitis, allergic dermatitis, contact dermatitis, psoriasis, systemic lupus erythematosus, ulcerative colitis, segmental ileitis, depression, bipolar disorder, mania, anxiety, schizophrenia, Alzheimer's disease, stroke, chronic pain, liver fibrosis, kidney fibrosis, and nephritis.
